(19) Europäisches Patentamt
European Patent Office
Office européen des brevets

(11) EP 2 277 881 A1

(12) # EUROPEAN PATENT APPLICATION
published in accordance with Art. 153(4) EPC

(43) Date of publication:
**26.01.2011 Bulletin 2011/04**

(21) Application number: **09732609.4**

(22) Date of filing: **16.04.2009**

(51) Int Cl.:
***C07D 487/04*** *(2006.01)*
***A61K 31/5025*** *(2006.01)*
***A61K 31/506*** *(2006.01)*
***A61K 31/5377*** *(2006.01)*
***A61K 31/55*** *(2006.01)*
***A61P 9/00*** *(2006.01)*
***A61P 9/10*** *(2006.01)*
***A61P 11/02*** *(2006.01)*
***A61P 17/04*** *(2006.01)*
***A61P 19/02*** *(2006.01)*
***A61P 29/00*** *(2006.01)*
***A61P 31/00*** *(2006.01)*
***A61P 35/00*** *(2006.01)*
***A61P 37/00*** *(2006.01)*
***A61P 43/00*** *(2006.01)*

(86) International application number:
**PCT/JP2009/057706**

(87) International publication number:
**WO 2009/128520 (22.10.2009 Gazette 2009/43)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **18.04.2008 JP 2008109548**

(71) Applicant: **Shionogi&Co., Ltd.**
**Osaka-shi**
**Osaka 541-0045 (JP)**

(72) Inventors:
- **TANIYAMA Daisuke**
  **Osaka-shi**
  **Osaka 553-0002 (JP)**
- **KANO Kazuya**
  **Osaka-shi**
  **Osaka 553-0002 (JP)**
- **OKAMOTO Kazuya**
  **Osaka-shi**
  **Osaka 553-0002 (JP)**
- **FUJIOKA Masahiko**
  **Osaka-shi**
  **Osaka 553-0002 (JP)**
- **MITSUOKA Yasunori**
  **Osaka-shi**
  **Osaka 553-0002 (JP)**

(74) Representative: **UEXKÜLL & STOLBERG**
**Patentanwälte**
**Beselerstrasse 4**
**22607 Hamburg (DE)**

(54) **HETEROCYCLIC COMPOUND HAVING INHIBITORY ACTIVITY ON P13K**

(57) The purpose of the present invention is to provide a compound or a pharmaceutically acceptable salt thereof which inhibits the activity of PI3K to regulate many biological processes including the growth, differentiation, survival, proliferation, migration, metabolism, and the like of cells and is therefore useful for the prevention/treatment of diseases including inflammatory diseases, arteriosclerosis, vascular/circulatory diseases, cancer/tumors, immune system diseases, cell proliferative diseases, infectious diseases, and the like. This was achieved by providing a substituted 2-amino-5,6-nitrogenated fused ring compound shown in the present specification, or a pharmaceutically acceptable salt thereof.

EP 2 277 881 A1

## Description

TECHNICAL FIELD

The present invention is related to: a compound that has inhibitory activity of phosphatidylinositol-3-kinase (hereinafter also referred to as "PI3K") and is useful for the treatment/prevention of a variety of phosphatidylinositol-3-kinase dependent diseases including cancers, inflammatory diseases, circulatory diseases, and the like; a salt thereof; or the like.

BACKGROUND ART

Phosphatidylinositol-3-kinase is an enzyme that catalyzes not only the production of a specific phospholipase, but also an intracellular mediator from phosphatidylinositol (hereinafter also referred to as "PI") of a membrane lipid. The 3'-OH group of phosphatidylinositol is phosphorylated, and thus, when phosphatidylinositol, phosphatidylinositol 4-phosphate, and phosphatidylinositol 4,5-bisphosphate are used as substrates, phosphatidylinositol 3-phosphate, phosphatidylinositol 3,4-bisphosphate, and phosphatidylinositol 3,4,5-triphosphate (PIP3) are produced respectively.
A phospholipid (PIP3) in which the hydroxyl group at 3-position of the inositol ring is phosphorylated by this PI3K works as a second messenger that activates a serine/threonine kinase such as PDK1, Akt/PKB, and the like in a signal transduction route mediated by receptor stimulation. This second messenger is said to regulate many biological processes including growth, differentiation, survival, proliferation, migration and metabolism, and the like of cells.
PI3Ks are classified into three groups, *i.e.*, Classes I to III, by a primary structure, a regulatory mechanism of activity, and specificity to a substrate. Among them, Class I is important in signaling.
Class I is, depending on the differences in the heterodimer, classified into IA ($\alpha$, $\beta$, and $\delta$) containing a subunit of 85 kDa, and IB ($\gamma$) containing a subunit of 101 kDa.
Class IA is associated with a variety of cell surface receptors such as hormones/growth factors and the like. For a signal transduction route, it is said to be a protein/kinase receptor type. Class IB is associated with a G protein receptor (GCPR), which is a receptor of a chemokine and the like. Furthermore, it is said that when a specific tyrosine residue of a receptor is phosphorylated by stimulation of an activator such as a chemokine and the like, a regulatory subunit is bound to a catalytic subunit via the SH2 domain, and thereby the inhibition activity of the regulatory subunit is reduced to exhibit enzyme activity.
PIP3 works as a messenger for intracellular signaling. In the immediate downstream of PIP3, AKT (also known as protein kinase B (PKB)) and the like are known. In these downstream routes, a signal is said to be transmitted by activating a functional protein having the PH domain.
PI3K$\alpha$ and PI3K$\beta$ are widely distributed in a variety of cells, and related to cell growth/glycometabolism. Based on these actions, inhibitors of PI3K$\alpha$ and PI3K$\beta$ are utilized as anticancer agents and the like. PI3K$\delta$ and PI3K$\gamma$ exist mainly in blood and cells of the immune (lymphatic) system. PI3K$\gamma$ is also known to be widely distributed in inflammatory cells.
Regarding PI3K$\gamma$, on the basis of studies of knock-out mice thereof and the like, it was found that respiratory burst of a neutrophil by a chemotactic factor and the migration of a macrophage/neutrophil to an infection focus were blocked, functions of T cells/dendritic cells were thereby decreased, the degranulation of mast cells was thereby blocked, and anaphylaxis was thereby decreased. Accordingly, an inhibitor of PI3K$\gamma$ is considered useful as a therapeutic agent for these diseases. Furthermore, on the basis of studies of arthritis, it is considered useful as an inhibitor of the inflammatory-cell infiltration in a part of a joint (Non-Patent Literature 1 and Non-Patent Literature 2). Furthermore, studies using a PI3K$\gamma$ inhibitor report the inhibition of the activation of a mast cell (Non-Patent Literature 3), the inhibition of the activation/migration of a leukocyte (Non-Patent Literature 4 and Non-Patent Literature 5), the inhibition of lymphocyte activation (Non-Patent Literature 6), and the like.
On the basis of these studies, a PI3K$\gamma$ inhibitor is believed to be useful for the treatment of the following diseases/disorders: thrombus; allergy/anaphylaxis (allergic diseases include, for example, asthma, atopic dermatitis, allergic rhinitis, and the like); inflammation such as pancreatitis (Non-Patent Literature 7), pneumonia, airway inflammation, chronic obstructive pulmonary disease (COPD) (Non-Patent Literature 8 and Non-Patent Literature 9), arthritis (e.g., articular rheumatism (Non-Patent Literature 8 and Non-Patent Literature 9), glomerulonephritis, and the like; systemic lupus erythematosus (SLE) (Non-Patent Literature 8 and Non-Patent Literature 9); autoimmune diseases; pulmonary disorder; circulatory diseases such as heart failure (systolic), cardiac ischemia (systolic), high blood pressure, and the like (Non-Patent Literature 10); wound healing; infectious diseases (Non-Patent Literature 11); cancer/tumors such as neoplasm (Patent Literature 1); suppression of the immune reaction in organ transplantation and autoimmune diseases (Patent Literature 2); and the like.
Regarding PI3K$\delta$, on the basis of studies of knock-out mice thereof and the like, the B cell differentiation disorder of bone marrow is induced, and in immunomodulation, the role thereof is expected.

PI3K is reported to be deeply involved with various stages of diseases in articular rheumatism, such as: the T cell/B cell activation by presenting an antigen; and the inflammatory cell infiltration such as neutrophil, macrophage, or the like, the synovial cell proliferation, the mast cell activation, and the like (Non-Patent Literature 12).

As examples of compounds having PI3 kinase inhibition activity, classically, wortmannin (Non-Patent Literature 13), 2-(4-morpholinyl)-8-phenyl-4H-1-benzopyran-4-one (Patent Literature 2), 17β-hydroxywortmannin and a derivative thereof (Patent Literature 1), and the like are known.

As substituted 2-amino-5,6-nitrogenated fused ring compounds being useful as a medicament, compounds disclosed in Patent Literature 3, Patent Literature 4, Patent Literature 5, Non-Patent Literature 14, Non-Patent Literature 15, Non-Patent Literature 16, Patent Literature 6, Patent Literature 7, Patent Literature 8, Patent Literature 9, Patent Literature 10, Non-Patent Literature 17, Patent Literature 11, Non-Patent Literature 18, Non-Patent Literature 19, Patent Literature 12, Patent Literature 13, Patent Literature 14, or the like are known.

PRIOR ART REFERENCES

[Patent Literature]

Patent Literature 1: Japanese Laid-Open Publication No. 7-145051
Patent Literature 2: Pamphlet of International Publication No. WO 95/29673
Patent Literature 3: Pamphlet of International Publication No. WO 2007/095588
Patent Literature 4: Japanese Laid-Open Publication No. 2005/008581
Patent Literature 5: Pamphlet of International Publication No. WO 2005/054246
Patent Literature 6: Specification of United States Patent No. 4092321
Patent Literature 7: Specification of United States Patent No. 4096264
Patent Literature 8: Specification of United States Patent No. 4105767
Patent Literature 9: European Patent Publication No. 0018837
Patent Literature 10: Japanese Laid-Open Publication No. 52-73896
Patent Literature 11: Pamphlet of International Publication No. WO 2002/044156
Patent Literature 12: Pamphlet of International Publication No. WO 2008/016131
Patent Literature 13: Pamphlet of International Publication No. WO 2008/016192
Patent Literature 14: Pamphlet of International Publication No. WO 2009/010530


[Non-Patent Literature]

Non-Patent Literature 1: M.P. Wymann, et al., Biochemical Society Transactions 2003, 31, pp. 275-280
Non-Patent Literature 2: Rueckle T. et al., NATURE REVIEWS DRUG DISCOVERY 2006, 5 pp. 903-918
Non-Patent Literature 3: Laffargue M. et al., Immunity 2002 16: pp. 441-451
Non-Patent Literature 4: Hirsch E. et al., Science 2000 287: pp. 1049-1053
Non-Patent Literature 5: Li Z. et al., Science 2000 287; pp. 982-983
Non-Patent Literature 6: Sasaki T. et al., Science 2000 287; pp. 1040-1046
Non-Patent Literature 7: Lupia E. et al., Am J Pathol. 2004; 165, pp. 2003-2011
Non-Patent Literature 8: Barber D F et al., Nat Med 2005 11: pp. 933-935
Non-Patent Literature 9: Camps, Nat Med 2005 11: pp. 936-943
Non-Patent Literature 10: Campbell et al., Circ Res. 2005, 96, pp. 197-206
Non-Patent Literature 11: Yadav M. et al., J Immunol. 2006, 176, pp. 5494-503
Non-Patent Literature 12: Japanese Journal of Clinical Immunology, Vol. 30, 2007, 5, pp. 369-374
Non-Patent Literature 13: Ui M T et al., Trends Biochem. Sci., 1995, 20, pp. 303-307
Non-Patent Literature 14: Bochis R J et al., J. Med. Chem. 1981, 24, pp. 1518-1521
Non-Patent Literature 15 : Peterson L H et al., J. Heterocyclic Chem. 1981, 18, pp. 659-662
Non-Patent Literature 16: Bochis R J et al., J. Med. Chem. 1978, 21(2), pp. 235-236
Non-Patent Literature 17: Hasegawa M. et al., J. Med. Chem. 2007, 50, pp. 4453-4470
Non-Patent Literature 18: Jaramillo C. et al., Tetrahedron Letters, 2002, 43, pp. 9051-9054
Non-Patent Literature 19: Mourad A E et al., J. Heterocyclic Chem., 1993, 30, pp. 1365-1372

SUMMARY OF THE INVENTION

PROBLEMS TO BE SOLVED BY THE INVENTION

The purpose of the present invention is to provide a substituted 2-amino-5, 6- nitrogenated fused ring compound or a pharmaceutically acceptable salt thereof, wherein the compound inhibits the activity of PI3K to regulate many biological processes including growth, differentiation, survival, proliferation, migration, and metabolism, and the like of cells, and is therefore useful for the prevention/treatment of diseases including inflammatory diseases (allergic diseases (allergic dermatitis/allergic rhinitis, and the like), articular rheumatism, anaphylaxis, and the like), arteriosclerosis, vascular/circulatory diseases, cancer/tumors, immune system diseases, cell-proliferative diseases, infectious diseases, and the like.

MEANS FOR SOLVING THE PROBLEMS

The present invention is related to: a compound represented by the formula (I):
[Chem. 1]

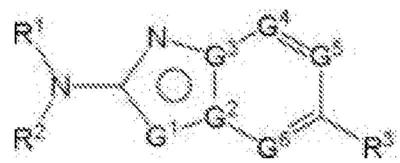
(I)

wherein $R^1$ is a hydrogen atom or alkyl;
$R^2$ is substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, or substituted or unsubstituted aminocarbonylamino;
$R^3$ is a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^8$ (wherein $R^8$ is a hydrogen atom, substituted or unsubstituted alkyl,substitutedor unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl); or a group represented by the formula: $-S(O)_mR^9$ (wherein $R^9$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted

acyl; and m is 0, 1, or 2);
a cyclic group represented by the formula:

[Chem. 2]

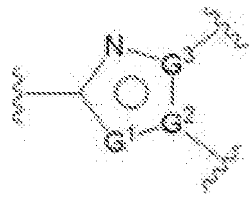

is a cyclic group represented by any of the following formulas (A)-(D):

[Chem. 3]

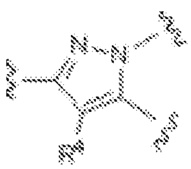, 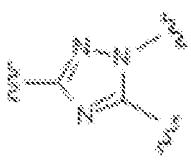, 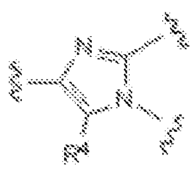, and 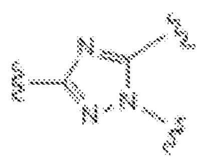

(A) (B) (C) (D) ;

$G^4$ is $C(R^5)$ or a nitrogen atom;
$G^5$ is $C(R^6)$ or a nitrogen atom;
$G^6$ is $C(R^7)$ or a nitrogen atom; and
$R^4$, $R^5$, $R^6$, and $R^7$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{10}$ (wherein $R^{10}$ is a hydrogen atom, substituted or unsubstituted alkyl,substitutedor unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{11}$ (wherein $R^{11}$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted acyl; and n is 0, 1, or 2); a pharmaceutically acceptable salt thereof, or a prodrug thereof including a solvate thereof and the like; and
a pharmaceutical composition containing this said compound, a pharmaceutically acceptable salt thereof, or a prodrug thereof including a solvate thereof and the like.

In the formula of the above compound, there is the proviso that: when the cyclic group is a cyclic group represented by (C) or (D) and $R^2$ is substituted or unsubstituted acyl, substituted or unsubstituted alkyloxycarbonyl,

substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl, $R^3$ is not 6-membered ring heteroaryl substituted with substituted or unsubstituted amino;
when the cyclic group is a cyclic group represented by (C) or (D), $G^1$ is CH, $G^4$ is CH, and $G^5$ is CH, $R^2$ is not trifluoromethylcarbonyl; and
when the cyclic group is a cyclic group represented by (C), $G^4$ is CH, $G^5$ is CH, $G^6$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the formula:
[Chem. 4]

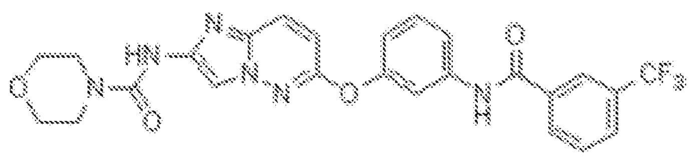

or

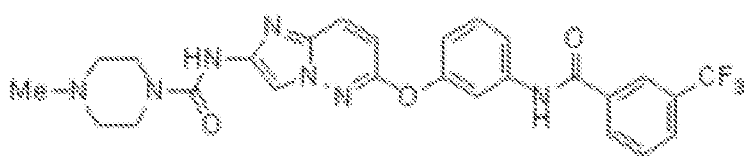

.

Preferably, the present invention is: a compound represented by the formula (II):
[Chem. 5]

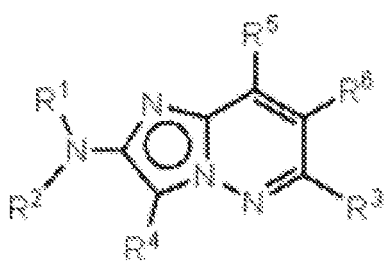

(II)

wherein the definitions of $R^1$, $R^2$, $R^3$, $R^4$, $R^5$, and $R^6$ are the same as the above,
a pharmaceutically acceptable salt thereof, or a prodrug thereof including a solvate thereof and the like; and
a pharmaceutical composition containing said compound (or salt, prodrug or solvate thereof) this as an active ingredient.
In another aspect, the present invention is related to a compound represented by the formula (IV):
[Chem. 6]

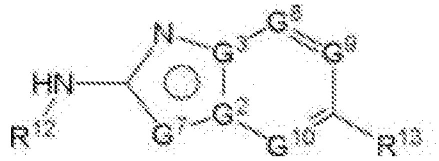 (IV)

wherein $R^{12}$ is substituted or unsubstituted aryl, substituted or unsubstituted heteroaryl, a substituted or unsubstituted non-aromatic heterocyclic group, substituted or unsubstituted alkylcarbonyl, substituted or unsubstituted alkyloxycarbonyl, or substituted or unsubstituted aminocarbonyl;

$R^{13}$ is a halogen atom, substituted or unsubstituted alkyl, substituted or unsubstituted amino, substituted or unsubstituted alkenyl, substituted or unsubstituted aryl, substituted or unsubstituted heteroaryl, a substituted or unsubstituted non-aromatic heterocyclic group, substituted or unsubstituted arylamino, substituted or unsubstituted heteroarylamino, substituted or unsubstituted alkyloxycarbonyl, substituted or unsubstituted aminocarbonyl, substituted or unsubstituted aminocarbonylamino, substituted or unsubstituted alkylamino, substituted or unsubstituted acyl, substituted or unsubstituted acylamino, a group represented by the formula: $-OR^{14}$ (wherein $R^{14}$ is substituted or unsubstituted aryl, substituted or unsubstituted heteroaryl, or a substituted or unsubstituted non-aromatic heterocyclic group); or a group represented by the formula: $-S(O)_mR^{15}$ (wherein $R^{15}$ is substituted or unsubstituted aryl, or substituted or unsubstituted heteroaryl; and m is 0, 1, or 2);

a cyclic group represented by the formula:

[Chem. 7]

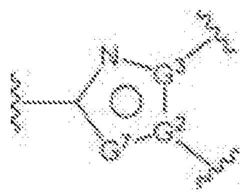

is a cyclic group represented by any of the following formulas (E) - (H) :

[Chem. 8]

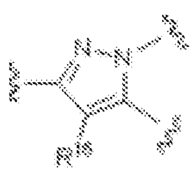 ,
(E)

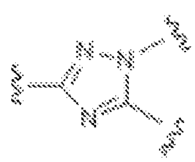 ,
(F)

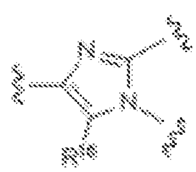 , and
(G)

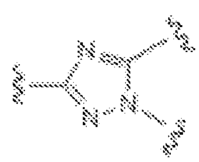
(H) ;

$G^8$ is $C(R^{17})$ or a nitrogen atom;

$G^9$ is $C(R^{18})$ or a nitrogen atom;

$G^{10}$ is $C(R^{19})$ or a nitrogen atom; and

$R^{16}$, $R^{17}$, $R^{18}$, and $R^{19}$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{20}$ (wherein $R^{20}$ is substituted or unsubstituted alkyl, substituted or unsubstituted alkenyl, substituted or unsubstituted alkynyl,substitutedor unsubstituted aryl, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{21}$ (wherein $R^{21}$ is substituted or unsubstituted alkyl, or substituted or unsubstituted aryl; and n is 0, 1, or 2), a pharmaceutically acceptable salt thereof, or a prodrug thereof including a solvate thereof and the like; and a pharmaceutical composition containing said compound (or salt, prodrug or solvate thereof) this as an active ingredient.

There is the proviso that: when the cyclic group is a cyclic group represented by (G) or (H), and $R^{12}$ is substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl, $R^{13}$ is not 6-membered ring heteroaryl substituted with substituted or unsubstituted amino;
when $R^{12}$ is substituted or unsubstituted phenyl, $G^2$ is a carbon atom, $G^3$ is a nitrogen atom, and $G^{10}$ is a nitrogen atom, $R^{16}$ is not cyano or carbamoyl;
when the cyclic group is a cyclic group represented by (G) or (H), $R^{12}$ is substituted or unsubstituted phenyl, $G^7$ is a nitrogen atom, and $G^8$ is a nitrogen atom, $R^{13}$ is not substituted or unsubstituted phenyl;
when the cyclic group is a cyclic group represented by (G) or (H) , $G^7$ is CH, $G^8$ is CH, and $G^9$ is CH, $R^{12}$ is notmethoxycarbonyl, methylcarbonyl, or trifluoromethylcarbonyl;
when the cyclic group is a cyclic group represented by (E) or (F), $R^{12}$ is methyloxycarbonyl, $G^7$ is CH, $G^8$ is CH, and $G^9$ is CH, $R^{13}$ is not phenylthio or phenylsulfinyl; and when the cyclic group is a cyclic group represented by (G), $G^8$ is CH, $G^9$ is CH, $G^{10}$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the following formula:

[Chem. 9]

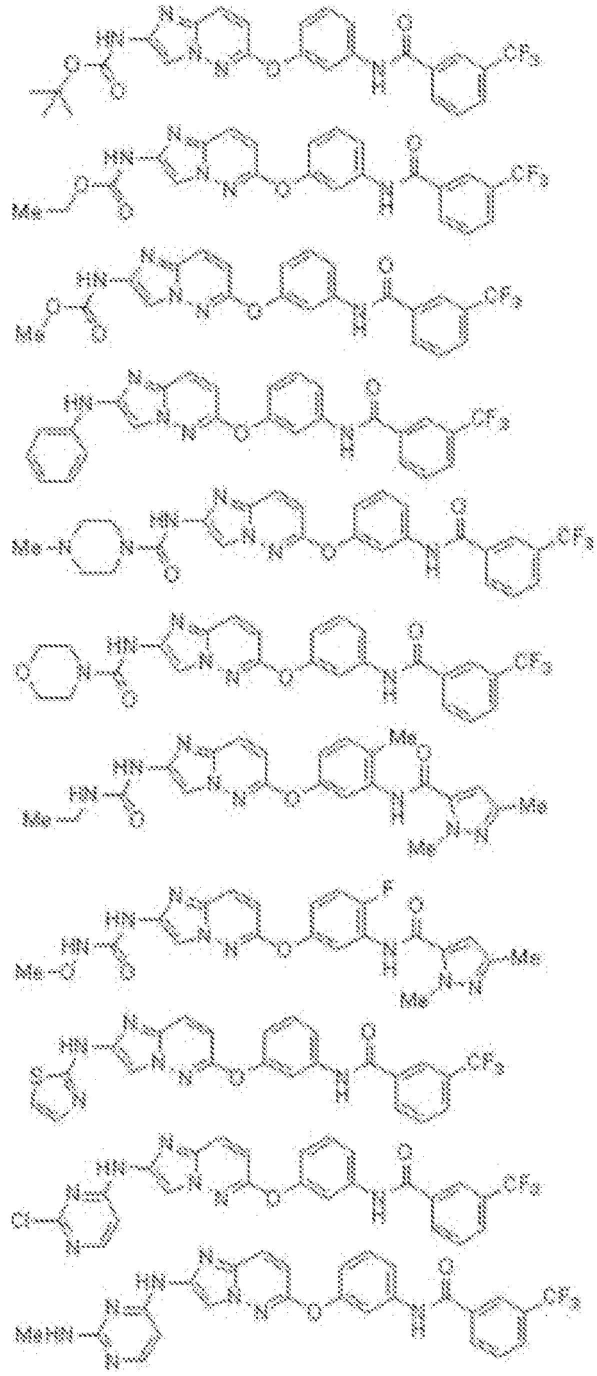

or

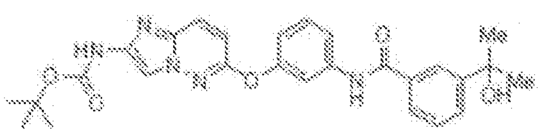

.

In a preferred embodiment, the present invention is related to a compound represented by the formula (V):

[Chem. 10]

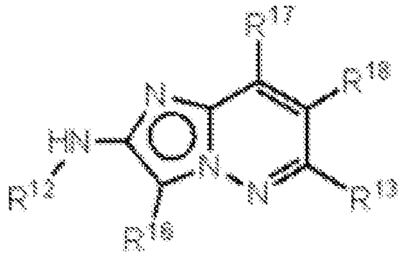

(V)

,

wherein the definitions of $R^{12}$, $R^{13}$, $R^{16}$, $R^{17}$, and $R^{18}$ are the same as above, a pharmaceutically acceptable salt thereof, or a prodrug thereof including a solvate thereof and the like; and a pharmaceutical composition containing said compound (or salt, prodrug or solvate thereof) this as an active ingredient.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{16}$, $R^{17}$, and $R^{18}$ may be hydrogen atoms.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{12}$ may be substituted or unsubstituted aryl, or substituted or unsubstituted heteroaryl.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{12}$ may be substituted or unsubstituted aminocarbonyl.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{13}$ may be substituted or unsubstituted aryl, or substituted or unsubstituted heteroaryl.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{13}$ may be substituted or unsubstituted aryloxy, substituted or unsubstituted arylthio, or substituted or unsubstituted arylamino.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{13}$ may be substituted or unsubstituted alkyloxycarbonyl, or substituted or unsubstituted aminocarbonyl.

In another preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{12}$ may be substituted or unsubstituted aryl, or substituted or unsubstituted heteroaryl, and $R^{13}$ may be substituted or unsubstituted aryl, or substituted or unsubstituted heteroaryl.

In a preferred embodiment, with regard to the compound of the present invention, a pharmaceutically acceptable salt thereof, or a prodrug thereof including a solvate thereof and the like; and a pharmaceutical composition containing said compound (or salt, prodrug or solvate thereof) this as an active ingredient, $R^{12}$ may be substituted or unsubstituted aminocarbonyl, and $R^{13}$ may be substituted or unsubstituted acylamino, substituted or unsubstituted arylamino, substituted or unsubstituted heteroarylamino, substituted or unsubstituted aryl, substituted or unsubstituted heteroaryl, substituted or unsubstituted acyl, the formula: $-OR^{14}$ (wherein the definition of $R^{14}$ is the same as above), or the formula: $-S(O)_mR^{15}$ (wherein the definitions of $R^{15}$ and m are the same as above).

In another aspect, the present invention is related to a phosphatidylinositol-3-kinase inhibitor containing the compound of the present invention, a pharmaceutically acceptable salt thereof, or a prodrug thereof including a solvate thereof and the like as an active ingredient.

In one embodiment, the inhibitor of the present invention may be specific to one or more types of α, β, γ, and δ phosphatidylinositol-3-kinase inhibitors.

From a pharmaceutical aspect, in a preferred embodiment, the pharmaceutical composition may be a composition for treating phosphatidylinositol-3-kinase dependent diseases. Such phosphatidylinositol-3-kinase dependent diseases can include: encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary disease, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, sarcoidosis, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; the prevention and/or therapeutic agents therefor; burn; traumatic inflammation; and the like.

In an embodiment, the present invention is related to a phosphatidylinositol-3-kinase inhibitor containing the compound of the present invention, a pharmaceutically acceptable salt thereof, or a prodrug thereof including a solvate thereof and the like.

In an embodiment, the present invention is related to a protein kinase B (ATK) inhibitor containing the compound of the present invention, a pharmaceutically acceptable salt thereof, or a prodrug thereof including a solvate thereof and the like.

In an embodiment, the present invention is related to an anticancer agent containing the compound of the present invention, a pharmaceutically acceptable salt thereof, or a prodrug thereof including a solvate thereof and the like.

In an embodiment, the present invention is related to an anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like), wherein the anti-inflammatory or the therapeutic agent includes the compound of the present invention, a pharmaceutically acceptable salt thereof, or a prodrug thereof including a solvate thereof and the like.

In an embodiment, the present invention is related to an antiallergic agent (asthma, atopic dermatitis, allergic rhinitis, and the like) containing the compound of the present invention, a pharmaceutically acceptable salt thereof, or a prodrug thereof including a solvate thereof and the like.

In an embodiment, the present invention is related to a therapeutic agent for immune system diseases wherein the therapeutic agent includes the compound of the present invention, a pharmaceutically acceptable salt thereof, or a prodrug thereof including a solvate thereof and the like.

In an embodiment, the present invention is related to an immunosuppressant containing the compound of the present invention, a pharmaceutically acceptable salt thereof, or a prodrug thereof including a solvate thereof and the like.

In an embodiment, the present invention is related to a therapeutic agent for autoimmune diseases wherein the therapeutic agent includes the compound of the present invention, a pharmaceutically acceptable salt thereof, or a prodrug thereof including a solvate thereof and the like.

In an embodiment, the present invention is related to an anti-circulatory-disease agent, such as antihypertensive agent and the like, wherein the agent includes the compound of the present invention, a pharmaceutically acceptable salt thereof, or a prodrug thereof including a solvate thereof and the like.

In an embodiment, the present invention is related to an antiinfectant containing the compound of the present invention, a pharmaceutically acceptable salt thereof, or a prodrug thereof including a solvate thereof and the like.

In an embodiment, the present invention is related to a wound-healing agent containing the compound of the

present invention, a pharmaceutically acceptable salt thereof, or a prodrug thereof including a solvate thereof and the like.

The present invention is also related to a method, a system, an apparatus, a kit, and the like for producing the compound of the present invention, a pharmaceutically acceptable salt thereof, or a prodrug thereof including a solvate thereof and the like.

The present invention is also related to a method, a system, an apparatus, a kit, and the like for preparing a pharmaceutical composition containing the compound of the present invention, a pharmaceutically acceptable salt thereof, or a prodrug thereof including a solvate thereof and the like.

The present invention is also related to a method, a system, an apparatus, a kit, and the like using the compound of the present invention, a pharmaceutically acceptable salt thereof, or a prodrug thereof including a solvate thereof and the like.

For example, the present invention provides the following items.

(1) A compound represented by the formula (I):

[Chem. 11]

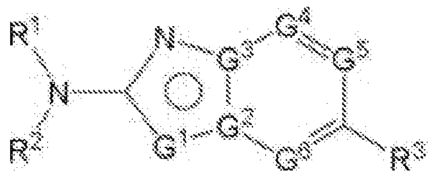

(I)

wherein $R^1$ is a hydrogen atom or alkyl;

$R^2$ is substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, or substituted or unsubstituted aminocarbonylamino;

$R^3$ is a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^8$ (wherein $R^8$ is a hydrogen atom, substituted or unsubstituted alkyl,substitutedor unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl); or a group represented by the formula: $-S(O)_mR^9$ (wherein $R^9$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted acyl; and m is 0, 1, or 2);

a cyclic group represented by the formula:

[Chem. 12]

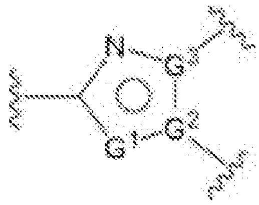

is a cyclic group represented by any of the following formulas (A)-(D):

[Chem. 13]

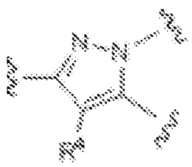
,
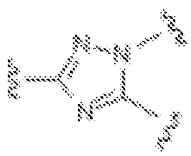
,
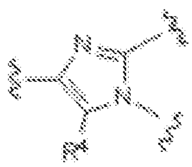
, and
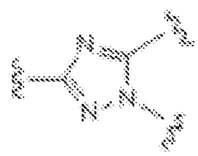

(A) (B) (C) (D) ;

$G^4$ is $C(R^5)$ or a nitrogen atom;
$G^5$ is $C(R^6)$ or a nitrogen atom;
$G^6$ is $C(R^7)$ or a nitrogen atom; and
$R^4$, $R^5$, $R^6$, and $R^7$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{10}$ (wherein $R^{10}$ is a hydrogen atom, substituted or unsubstituted alkyl,substitutedor unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{11}$ (wherein $R^{11}$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted acyl; and n is 0, 1, or 2);
with the proviso that, in the formula of the above compound: when the cyclic group is a cyclic group represented by (C) or (D) and $R^2$ is substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, a substituted or unsubstituted non-aromatic heterocyclic group-oxycarbonyl, or substituted or unsubstituted aminocarbonyl, $R^3$ is not 6-membered ring heteroaryl substituted with substituted or unsubstituted amino;
when the cyclic group is a cyclic group represented by (C) or (D), $G^1$ is CH, $G^4$ is CH, and $G^5$ is CH, $R^2$ is not trifluoromethylcarbonyl; and

when the cyclic group is a cyclic group represented by (C) , $G^4$ is CH, $G^5$ is CH, $G^6$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the following formula:

[Chem. 14]

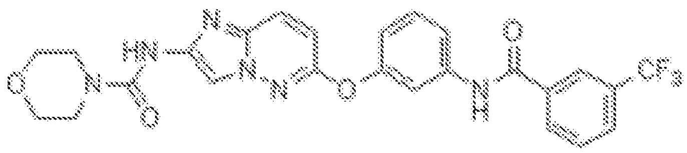

or

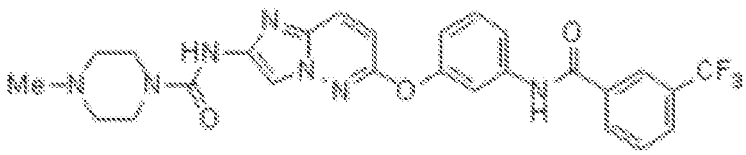

,

a pharmaceutically acceptable salt thereof, or a prodrug thereof including a solvate thereof and the like; and

a pharmaceutical composition containing thiscontaining said compound (or salt, prodrug or solvate thereof) compound, a pharmaceutically acceptable salt thereof, or a prodrug thereof including a solvate thereof and the like.

(2) The pharmaceutical composition according to the preceding item (1), containing a compound represented by the formula (II) :

[Chem. 15]

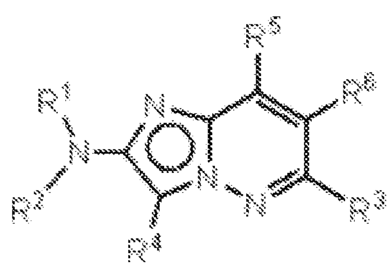

(II)

wherein the definitions of $R^1$, $R^2$, $R^3$, $R^4$, $R^5$, and $R^6$ are the same as the preceding item (1), a pharmaceutically acceptable salt thereof, or a prodrug thereof including a solvate thereof and the like.

(2A) The pharmaceutical composition according to the preceding item (2), containing the compound according to the preceding item (2)

wherein $R^1$ is a hydrogen atom,

$R^2$ is substituted or unsubstituted aryl, substituted or unsubstituted heteroaryl, substituted or unsubstituted acyl or substituted or unsubstituted aminocarbonyl,

$R^3$ is substituted or unsubstituted aryl, substituted or unsubstituted heteroaryl, substituted or unsubstituted acylamino, substituted or unsubstituted arylamino, substituted or unsubstituted heteroarylamino, substituted or unsubstituted acyl, the formula: $-OR^8$ (wherein the definition of $R^8$ is the same as the preceding item (1)) or the formula: $-S(O)_mR^9$ (wherein the definitions of $R^9$ and $_m$ are the same as the preceding item (1)),

$R^4$ is a hydrogen atom,

$R^5$ is a hydrogen atom, and

$R^6$ is a hydrogen atom, a pharmaceutically acceptable salt thereof, or a prodrug thereof including a solvate thereof and

the like.

(2B) The pharmaceutical composition according to the preceding item (2) or (2B), containing the compound according to the preceding item (2) or (2B) wherein $R^2$ is substituted or unsubstituted aminocarbonyl, a pharmaceutically acceptable salt thereof, or a prodrug thereof including a solvate thereof and the like.

(3) The compound represented by the formula (IV):

[Chem. 16]

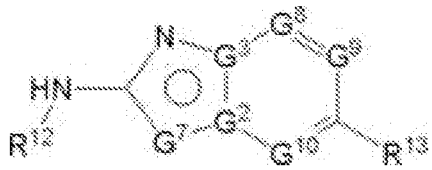

(IV)

wherein $R^{12}$ is substituted or unsubstituted aryl, substituted or unsubstituted heteroaryl, a substituted or unsubstituted non-aromatic heterocyclic group, substituted or unsubstituted alkylcarbonyl, substituted or unsubstituted alkyloxycarbonyl, or substituted or unsubstituted aminocarbonyl;
$R^{13}$ is a halogen atom, substituted or unsubstituted alkyl, substituted or unsubstituted amino, substituted or unsubstituted alkenyl, substituted or unsubstituted aryl, substituted or unsubstituted heteroaryl, a substituted or unsubstituted non-aromatic heterocyclic group, substituted or unsubstituted arylamino, substituted or unsubstituted heteroarylamino, substituted or unsubstituted alkyloxycarbonyl, substituted or unsubstituted aminocarbonyl, substituted or unsubstituted aminocarbonylamino, substituted or unsubstituted alkylamino, substituted or unsubstituted acyl, substituted or unsubstituted acylamino, a group represented by the formula: $-OR^{14}$ (wherein $R^{14}$ is substituted or unsubstituted aryl, substituted or unsubstituted heteroaryl, or a substituted or unsubstituted non-aromatic heterocyclic group); or a group represented by the formula: $-S(O)_mR^{15}$ (wherein $R^{15}$ is substituted or unsubstituted aryl, or substituted or unsubstituted heteroaryl; and m is 0, 1, or 2);
a cyclic group represented by the formula:

[Chem. 17]

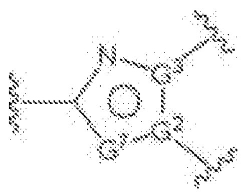

is a cyclic group represented by any of the following formulas (E)-(H):
[Chem. 18]

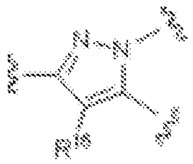 , 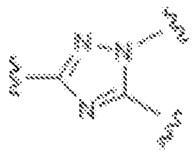 , 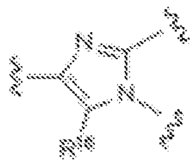 , and 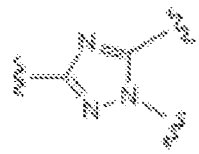

(E) (F) (G) (H) ;

$G^8$ is $C(R^{17})$ or a nitrogen atom;

$G^9$ is $C(R^{18})$ or a nitrogen atom;

$G^{10}$ is $C(R^{19})$ or a nitrogen atom; and

$R^{16}$, $R^{17}$, $R^{18}$, and $R^{19}$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{20}$ (wherein $R^{20}$ is substituted or unsubstituted alkyl, substituted or unsubstituted alkenyl, substituted or unsubstituted alkynyl, substituted or unsubstituted aryl, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{21}$ (wherein $R^{21}$ is substituted or unsubstituted alkyl, or substituted or unsubstituted aryl; and n is 0, 1, or 2);

with the proviso that: when the cyclic group is a cyclic group represented by (G) or (H) and $R^{12}$ is substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, a substituted or unsubstituted non-aromatic heterocyclic group-oxycarbonyl, or substituted or unsubstituted aminocarbonyl, $R^{13}$ is not 6-membered ring heteroaryl substituted with substituted or unsubstituted amino;

when $R^{12}$ is substituted or unsubstituted phenyl, $G^2$ is a carbon atom, $G^3$ is a nitrogen atom, and $G^{10}$ is a nitrogen atom, $R^{16}$ is not cyano or carbamoyl;

when the cyclic group is a cyclic group represented by (G) or (H), $R^{12}$ is substituted or unsubstituted phenyl, $G^7$ is a nitrogen atom, and $G^8$ is a nitrogen atom, $R^{13}$ is not substituted or unsubstituted phenyl;

when the cyclic group is a cyclic group represented by (G) or (H), $G^7$ is CH, $G^8$ is CH, and $G^9$ is CH, $R^{12}$ is notmethoxycarbonyl, methylcarbonyl, or trifluoromethylcarbonyl;

when the cyclic group is a cyclic group represented by (E) or (F), $R^{12}$ is methyloxycarbonyl, $G^7$ is CH, $G^8$ is CH, and $G^9$ is CH, $R^{13}$ is not phenylthio or phenylsulfinyl; and

when the cyclic group is a cyclic group represented by (G), $G^8$ is CH, $G^9$ is CH, $G^{10}$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the following formula:

[Chem. 19]

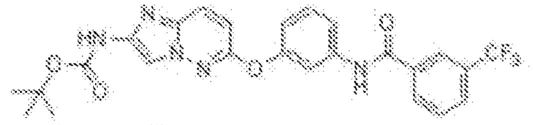

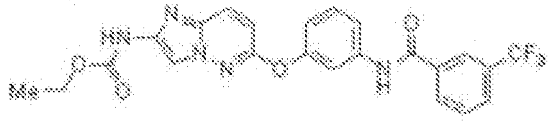

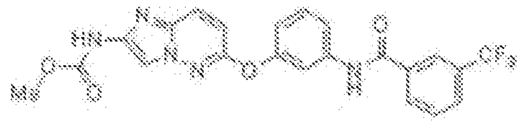

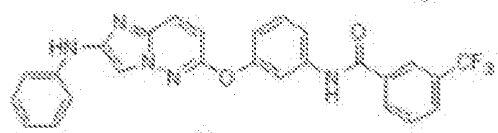

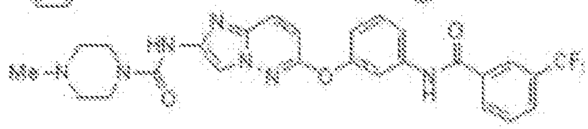

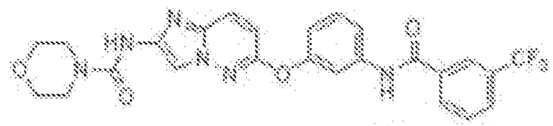

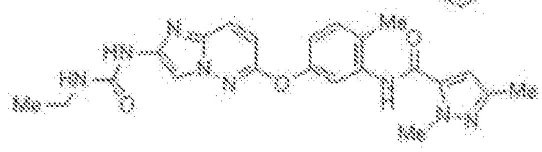

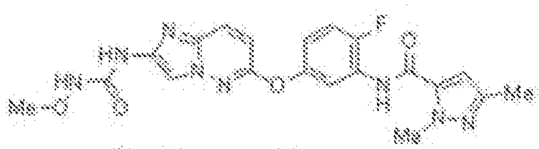

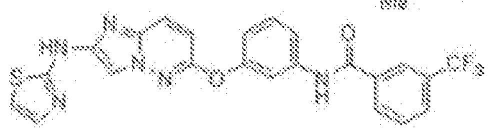

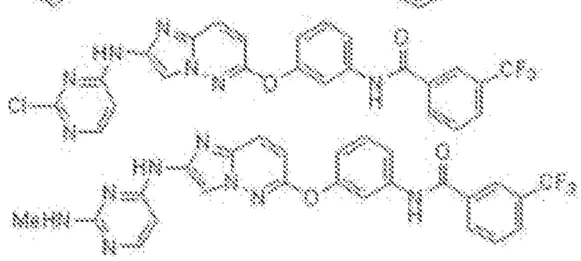

or

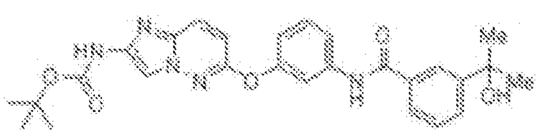

,

a pharmaceutically acceptable salt thereof, or a prodrug thereof including a solvate thereof and the like; and

a pharmaceutical composition containing thiscontaining said compound (or salt, prodrug or solvate thereof) as

an active ingredient.

(4) The compound according to the preceding item (3), represented by the formula (V):

[Chem. 20]

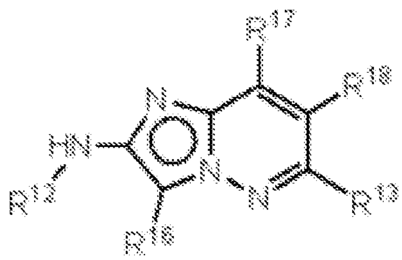

(V)

wherein the definitions of $R^{12}$, $R^{13}$, $R^{16}$, $R^{17}$, and $R^{18}$ are the same as the preceding item (3), a pharmaceutically acceptable salt thereof, or a prodrug thereof including a solvate thereof and the like; and a pharmaceutical composition containing thiscontaining said compound (or salt, prodrug or solvate thereof) as an active ingredient.

(5) The compound according to the preceding item (3) or (4) wherein $R^{16}$, $R^{17}$, and $R^{18}$ are hydrogen atoms, a pharmaceutically acceptable salt thereof, or a prodrug thereof including a solvate thereof and the like; and a pharmaceutical composition containing this containing said compound (or salt, prodrug or solvate thereof) as an active ingredient.

(6) The compound according to any of the preceding items (3)-(5) wherein $R^{12}$ is substituted or unsubstituted aryl, or substituted or unsubstituted heteroaryl, a pharmaceutically acceptable salt thereof, or a prodrug thereof including a solvate thereof and the like; and a pharmaceutical composition containing thiscontaining said compound (or salt, prodrug or solvate thereof) as an active ingredient.

(7) The compound according to any of the preceding items (3)-(5) wherein $R^{12}$ is substituted or unsubstituted aminocarbonyl, a pharmaceutically acceptable salt thereof, or a prodrug thereof including a solvate thereof and the like; and a pharmaceutical composition containing this said compound (or salt, prodrug or solvate thereof) as an active ingredient.

(8) The compound according to any of the preceding items (3)-(7) wherein $R^{13}$ is substituted or unsubstituted aryl, or substituted or unsubstituted heteroaryl, a pharmaceutically acceptable salt thereof, or a prodrug thereof including a solvate thereof and the like; and a pharmaceutical composition containing said compound (or salt, prodrug or solvate thereof) this as an active ingredient.

(9) The compound according to any of the preceding items (3) - (7) wherein $R^{13}$ is substituted or unsubstituted aryloxy, substituted or unsubstituted arylthio, or substituted or unsubstituted arylamino, a pharmaceutically acceptable salt thereof, or a prodrug thereof including a solvate thereof and the like; and a pharmaceutical composition containing said compound (or salt, prodrug or solvate thereof) this as an active ingredient.

(10) The compound according to any of the preceding items (3)-(7) wherein $R^{13}$ is substituted or unsubstituted alkyloxycarbonyl, or substituted or unsubstituted aminocarbonyl, a pharmaceutically acceptable salt thereof, or a prodrug thereof including a solvate thereof and the like; and a pharmaceutical composition containing said compound (or salt, prodrug or solvate thereof) this as an active ingredient.

(11) The compound according to the preceding items (3)-(5) wherein $R^{12}$ is substituted or unsubstituted aryl, or substituted or unsubstituted heteroaryl, and $R^{13}$ is substituted or unsubstituted aryl, or substituted or unsubstituted heteroaryl, a pharmaceutically acceptable salt thereof, or a prodrug thereof including a solvate thereof and the like; and a pharmaceutical composition containing said compound (or salt, prodrug or solvate thereof) this as an active ingredient.

(12) The compound according to any of the preceding items (3)-(5) wherein $R^{12}$ is substituted or unsubstituted aminocarbonyl, and $R^{13}$ is substituted or unsubstituted acylamino, substituted or unsubstituted arylamino, substituted or unsubstituted heteroarylamino, substituted or unsubstituted aryl, substituted or unsubstituted heteroaryl, substituted or unsubstituted acyl, the formula: $-OR^{14}$ (wherein the definition of $R^{14}$ is the same as above), or the formula: $-S(O)_mR^{15}$ (wherein the definitions of $R^{15}$ and m are the same as above), a pharmaceutically acceptable salt thereof, or a prodrug thereof including a solvate thereof and the like; and a pharmaceutical composition containing said compound (or salt, prodrug or solvate thereof) this as an active ingredient.

(12A) The compound according to any of the preceding items (3)-(5) and (12) wherein $R^{12}$ is aminocarbonyl substituted with (substituted or unsubstituted alkyl, substituted or unsubstituted aryl, substituted or unsubstituted heteroaryl, substituted or unsubstituted cycloalkyl, substituted or unsubstituted heterocycle, substituted or unsubstituted alkoxy, hydroxy, or substituted or unsubstituted amino), a pharmaceutically acceptable salt thereof, or a prodrug thereof including a solvate thereof and the like; and a pharmaceutical composition containing said compound (or salt, prodrug or solvate thereof) this as an active ingredient.

(13) The compound according to any of the preceding items (3)-(12) and (12A), a pharmaceutically acceptable salt thereof, or a prodrug thereof including a solvate thereof and the like; and a pharmaceutical composition containing said compound (or salt, prodrug or solvate thereof) this as an active ingredient.

(14) The compound according to any of the preceding items (3)-(12) and (12A), a pharmaceutically acceptable salt thereof, or a prodrug thereof including a solvate thereof and the like; and a pharmaceutical composition for treating phosphatidylinositol-3-kinase dependent diseases containing said compound (or salt, prodrug or solvate thereof) this as an active ingredient.

(15) A phosphatidylinositol-3-kinase inhibitor containing the compound, a pharmaceutically acceptable salt thereof, or a prodrug thereof including a solvate thereof and the like as an active ingredient according to any of the preceding items (3)-(12) and (12A).

(16) The inhibitor according to the preceding item (15), being specific to one or more types of $\alpha$, $\beta$, $\gamma$ and $\delta$ phosphatidylinositol-3-kinase inhibitors.

(17) The pharmaceutical composition according to any of the preceding items (3)-(12) and (12A), treating that treats the following phosphatidylinositol-3-kinase dependent diseases:

encephalitis, myelitis and encephalomyelitis, meningitis, inflammatory polyneuropathy, neuritis, dacryoadenitis, orbital inflammation, conjunctivitis (allergic conjunctivitis, vernalkeratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, sarcoidosis, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis,

bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; the prevention and/or therapeutic agents therefor; burn; traumatic inflammation; and the like.

(18) A phosphatidylinositol-3-kinase inhibitor containing the compound, a pharmaceutically acceptable salt thereof, or a prodrug thereof including a solvate thereof and the like according to any of the preceding items (3)-(12) and (12A).

(19) A protein kinase B (ATK) inhibitor containing the compound, a pharmaceutically acceptable salt thereof, or a prodrug thereof including a solvate thereof and the like according to any of the preceding items (3)-(12) and (12A).

(20) An anticancer agent containing the compound, a pharmaceutically acceptable salt thereof, or a prodrug thereof including a solvate thereof and the like according to any of the preceding items (3)-(12) and (12A).

(21) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent includes the compound, a pharmaceutically acceptable salt thereof, or a prodrug thereof including a solvate thereof and the like according to any of the preceding items (3)-(12) and (12A).

(22) An antiallergic agent (asthma, atopic dermatitis, allergic rhinitis, and the like) containing the compound, a pharmaceutically acceptable salt thereof, or a prodrug thereof including a solvate thereof and the like according to any of the preceding items (3)-(12) and (12A).

(23) A therapeutic agent for immune system diseases wherein the therapeutic agent includes the compound, a pharmaceutically acceptable salt thereof, or a prodrug thereof including a solvate thereof and the like according to any of the preceding items (3)-(12) and (12A).

(24) An immunosuppressant containing the compound, a pharmaceutically acceptable salt thereof, or a prodrug thereof including a solvate thereof and the like according to any of the preceding items (3)-(12) and (12A).

(25) A therapeutic agent for autoimmune diseases wherein the therapeutic agent includes the compound, a pharmaceutically acceptable salt thereof, or a prodrug thereof including a solvate thereof and the like according to any of the preceding items (3)-(12) and (12A).

(26) An anti-circulatory-disease agent, such as antihypertensive agent and the like, wherein the agent includes the compound, a pharmaceutically acceptable salt thereof, or a prodrug thereof including a solvate thereof and the like according to any of the preceding items (3) - (12) and (12A).

(27) An antiinfectant containing the compound, a pharmaceutically acceptable salt thereof, or a prodrug thereof including a solvate thereof and the like according to any of the preceding items (3)-(12) and (12A).

(28) A wound-healing agent containing the compound, a pharmaceutically acceptable salt thereof, or a prodrug thereof including a solvate thereof and the like according to any of the preceding items (3)-(12) and (12A).

(29) A method, a system, an apparatus, a kit, and the like for producing the compound, a pharmaceutically acceptable salt thereof, or a prodrug thereof including a solvate thereof and the like according to any of the preceding items (3) - (12) and (12A).

(30) A method, a system, an apparatus, a kit, and the like for preparing a pharmaceutical composition containing the compound, a pharmaceutically acceptable salt thereof, or a prodrug thereof including a solvate thereof and the like according to any of the preceding items (3)-(12) and (12A).

(31) A method, a system, an apparatus, a kit, and the like using the compound, a pharmaceutically acceptable salt thereof, or a prodrug thereof including a solvate thereof and the like according to any of the preceding items (3) - (12) and (12A).

(32) The pharmaceutical composition according to any of the preceding items (1), (2), (2A), (2B), (13), and (14),

wherein the composition is for treating and/or preventing inflammation.

(33) A method for preventing or treating inflammation, wherein the method is characterized by administering the compound, a pharmaceutically acceptable salt thereof, or a solvate thereof according to any of the preceding items (3)-(12) and (12A).

(34) Use of the compound, a pharmaceutically acceptable salt thereof, or a solvate thereof according to any of the preceding items (3)-(12) and (12A) for producing a therapeutic agent and/or a prophylactic agent for inflammation.

(35) The compound, a pharmaceutically acceptable salt thereof, or a solvate thereof according to any of the preceding items (3)-(12) and (12A) for treating and/or preventing inflammation.

Thus, these and other advantages of the present invention are apparent when the following detailed description is read.

EFFECT OF THE INVENTION

The present invention provides a medicament for treating phosphatidylinositol-3-kinase dependent diseases, a compound used therefor, a pharmaceutically acceptable salt thereof, or a prodrug thereof including a solvate thereof and the like. The compound of the present invention exhibits excellent PI3-kinase $\gamma$ inhibition activity as described in Examples below. Accordingly, the pharmaceutical composition of the present invention may be used for the prevention and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernalkeratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, sarcoidosis, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The compound of the present invention is a compound havingutilityas amedicament. Here, utilityas amedicament includes the following points: the compound has good metabolic stability; the induction of a drug-metabolizing enzyme is low; the inhibition of a drug-metabolizing enzyme which metabolizes another drug is also low; the compound has high oral absorbency; the clearance is low; the half-life is sufficiently long to express the efficacy; or the like.

BEST MODE FOR CARRYING OUT THE INVENTION

Hereinafter, the present invention is described with showing embodiments. It should be understood that, throughout the present specification, the expression of a singular form includes the concept of its plural form unless specified otherwise. Consequently, it should be understood that the article of a singular form (for example, in English language, "a", "an", "the," and the like) includes the concept of its plural form unless specified otherwise. Furthermore,

it should be understood that the terms used herein are used in a meaning normally used in the art unless specified otherwise. Thus, unless defined otherwise, all technical and scientific terms used herein have the same meanings as those generally understood by those skilled in the art in the field to which the present invention pertains. If there is a contradiction, the present specification (including definitions) precedes.

Each meaning of terms used herein is described below. In the present specification, each term is used in a unified meaning. Both when used alone and in combination with another word, each term is used in the same meaning.

As used herein, the term "halogen atom" means fluorine atom, chlorine atom, bromine atom, and iodine atom. Fluorine atom, chlorine atom, and bromine atom are preferable.

As used herein, the term "alkyl" encompasses a straight or branched monovalent hydrocarbon group having 1 to 8 carbon atoms. Examples thereof include methyl, ethyl, *n*-propyl, isopropyl, *n*-butyl, isobutyl, *sec*-butyl, *tert*-butyl, *n*-pentyl, isopentyl, *neo*-pentyl, *n*-hexyl, isohexyl, *n*-heptyl, *n*-octyl, and the like. Preferred is C1-C6 alkyl. More preferred is C1-C4 alkyl. When the carbon number is specified in particular, "alkyl" having carbon in a range thereof is meant.

As used herein, the term "alkenyl" encompasses a straight or branched monovalent hydrocarbon group having 2 to 8 carbon atoms and one or more double bonds. Examples thereof include vinyl, allyl, 1-propenyl, 2-butenyl, 2-pentenyl, 2-hexenyl, 2-heptenyl, 2-octenyl, and the like. Preferred is C2-C6 alkenyl. More preferred is C2-C4 alkenyl.

As used herein, the term "alkynyl" encompasses a straight or branched monovalent hydrocarbon group having 2 to 8 carbon atoms and one or more triple bonds. Examples thereof include ethynyl, 1-propynyl, 2-propynyl, 2-butynyl, 2-pentynyl, 2-hexynyl, 2-heptynyl, 2-octynyl, and the like. Preferred is C2-C6 alkynyl. More preferred is C2-C4 alkynyl.

As used herein, the term "cycloalkyl" encompasses cycloalkyl having 3 to 8 carbon atoms. Examples thereof include cyclopropyl, cyclobutyl, cyclopentyl, cyclohexyl, cycloheptyl, and cyclooctyl. Preferred is C3-C6 cycloalkyl.

As used herein, the term "cycloalkenyl" encompasses cycloalkenyl having 3 to 8 carbon atoms. Examples thereof include cyclopropenyl, cyclobutenyl, cyclopentenyl, cyclohexenyl, and cycloheptenyl. Preferred is C3-C6 cycloalkenyl.

As used herein, the term "alkyloxy" includes methyloxy, ethyloxy, n-propyloxy, isopropyloxy, n-butyloxy, isobutyloxy, sec-butyloxy, tert-butyloxy, n-pentyloxy, isopentyloxy, 2-pentyloxy, 3-pentyloxy, n-hexyloxy, isohexyloxy, 2-hexyloxy, 3-hexyloxy, n-heptyloxy, n-octyloxy,andthelike. Preferred is C1-C6 alkyloxy. More preferred is C1-C4 alkyloxy. When the carbon number is specified in particular, "alkyloxy" having carbon in a range thereof is meant.

As used herein, the term "alkylthio" includes methylthio, ethylthio, n-propylthio, isopropylthio, n-butylthio, isobutylthio, sec-butylthio, tert-butylthio, n-pentylthio, isopentylthio, 2-pentylthio, 3-pentylthio, n-hexylthio, isohexylthio, 2-hexylthio, 3-hexylthio, n-heptylthio, n-octylthio, and the like. Preferred is C1-C6 alkylthio. More preferred is C1-C4 alkylthio. When the carbon number is specified in particular, "alkylthio" having carbon in a range thereof is meant.

As used herein, the term "alkylsulfonyl" includes methylsulfonyl, ethylsulfonyl, n-propylsulfonyl, isopropylsulfonyl, *n*-butylsulfonyl, isobutylsulfonyl, *sec*-butylsulfonyl, *tert*-butylsulfonyl, *n*-pentylsulfonyl, isopentylsulfonyl, 2-pentylsulfonyl, 3-pentylsulfonyl, *n*-hexylsulfonyl, isohexylsulfonyl, 2-hexylsulfonyl, 3-hexylsulfonyl, *n*-heptylsulfonyl, *n*-octylsulfonyl, and the like. Preferred is C1-C6 alkylsulfonyl. More preferred is C1-C4 alkylsulfonyl.

Asused herein,theterm"alkyloxycarbonyl"includes methyloxycarbonyl, ethyloxycarbonyl, *n*-propyloxycarbonyl, isopropyloxycarbonyl, *n*-butyloxycarbonyl, tert-butyloxycarbonyl, *n*-pentyloxycarbonyl, and the like. Preferred is C1-C4 alkyloxycarbonyl. More preferred is C1-C2 alkyloxycarbonyl.

As used herein, the term "acyl" means formyl, alkylcarbonyl, alkenylcarbonyl, cycloalkylcarbonyl, cycloalkenylcarbonyl, arylcarbonyl, heteroarylcarbonyl, or non-aromatic heterocyclic group-carbonyl. Examples thereof include acetyl, propionyl, butyloyl, benzoyl, and the like.

As used herein, the term "substituted or unsubstituted amino" encompasses amino that may be substituted with the aforementioned "alkyl," the below-mentioned "aryl," the below-mentioned "heteroaryl," the aforementioned "acyl," the aforementioned "alkyloxycarbonyl," and/or the aforementioned "alkylsulfonyl" at 1 or 2 positions. Examples thereof include amino, methylamino, dimethylamino, ethylamino, diethylamino, ethylmethylamino, benzylamino, acetylamino, benzoylamino, methyloxycarbonylamino, methylsulfonylamino, and the like. Preferred are amino, methylamino, dimethylamino, ethylmethylamino, diethylamino, acetylamino, methylsulfonylamino, and the like.

As used herein, the term "substituted or unsubstituted carbamoyl" encompasses substituted or unsubstituted aminocarbonyl in which the substituted or unsubstituted amino portion is the aforementioned "substituted or unsubstituted amino." Examples thereof include carbamoyl, N-methylcarbamoyl, N,N-dimethylcarbamoyl, N-ethyl-N-methylcarbamoyl, N,N-diethylcarbamoyl, N-benzylcarbamoyl, N-acetylcarbamoyl, N-methylsulfonylcarbamoyl, and the like. Preferred are carbamoyl, N-methylcarbamoyl, N,N-dimethylcarbamoyl, N-methylsulfonylcarbamoyl, and the like.

As used herein, the term "alkylene" means straight or branched alkylene having 1 to 10 carbons. Examples thereof include methylene, 1-methylmethylene, 1,1-dimethylmethylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene, 2,2-di-*n*-propyltrimethylene, and the like. In particular, preferred is straight or branched alkylene having 2 to 6 carbons.

As used herein, the term "alkenylene" means straight or branched alkenylene having 2 to 10 carbons. Examples thereof include ethenylene, 1-methylethenylene, 1-ethylethenylene, 1,2-dimethylethenylene, 1,2-diethylethenylene, 1-ethyl-2-methylethenylene, propenylene, 1-methyl-2-propenylene, 2-methyl-2-propenylene, 1,1-dimethyl-2-propenylene, 1,2-dimethyl-2-propenylene, 1-ethyl-2-propenylene, 2-ethyl-2-propenylene, 1,1-diethyl-2-propenylene, 1,2-diethyl-2-propenylene, 1-butenylene, 2-butenylene, 1-methyl-2-butenylene, 2-methyl-2-butenylene, 1,1-dimethyl-2-butenylene, 1,2-dimethyl-2-butenylene,and the like. In particular, preferred is straight or branched alkenylene having 2 to 6 carbons.

As used herein, the term "aryl" encompasses monocyclic or fused-cyclic aromatic hydrocarbon, which may be fused with the aforementioned "cycloalkyl" or the below-mentioned "non-aromatic heterocyclic group" at any possible position. Both in the cases that aryl is monocyclic and fused-cyclic, it may be bound at any possible position. Examples thereof include phenyl, 1-naphthyl, 2-naphthyl, anthryl, tetrahydronaphthyl, 1,3-benzodioxolyl, 1,4-benzodioxanyl, and the like. Preferred are phenyl, 1-naphthyl, and 2-naphthyl. More preferred is phenyl.

As used herein, the term "non-aromatic heterocyclic group" encompasses a 5 to 7-membered non-aromatic ring containing one or more optionally-selected oxygen atoms, sulfur atoms, or nitrogen atoms in the ring, or a ring in which two or more of them are fused. Examples thereof include pyrrolidinyl (e.g., 1-pyrrolidinyl, 2-pyrrolidinyl), pyrrolinyl (e.g., 3-pyrrolinyl), imidazolidinyl (e.g., 2-imidazolidinyl), imidazolinyl (e.g., imidazolinyl), pyrazolidinyl (e.g., 1-pyrazolidinyl, 2-pyrazolidinyl), pyrazolinyl (e.g., pyrazolinyl), piperidyl (e.g., piperidino, 2-piperidyl), piperazinyl (e.g., 1-piperazinyl), indolinyl (e.g., 1-indolinyl), isoindolinyl (e.g., isoindolinyl), morpholinyl (e.g., morpholino, 3-morpholinyl), and the like.

As used herein, the term "heteroaryl" encompasses a 5 to 6-membered aromatic ring containing one or more optionally-selected oxygen atoms, sulfur atoms, or nitrogen atoms in the ring. This may be fused with the aforementioned "cycloalkyl," the aforementioned "aryl," the aforementioned "non-aromatic heterocyclic group", or another heteroaryl at any possible position. Both in the cases that heteroaryl is monocyclic and fused-cyclic, it maybe bound at any possible position. Examples thereof include pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl, 4-pyrazolyl), isothiazolyl (e.g., 3-isothiazolyl), isoxazolyl (e.g., 3-isoxazolyl), oxazolyl (e.g.,2-oxazolyl, 4-oxazolyl, 5-oxazolyl), thiazolyl (e.g., 2-thiazolyl, 4-thiazolyl, 5-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolizinyl (e.g., 2-indolizinyl, 6-indolizinyl), isoindolyl (e.g., 2-isoindolyl), indolyl (e.g., 1-indolyl, 2-indolyl, 3-indolyl), indazolyl (e.g., 3-indazolyl),purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinolanyl (e.g., 2-quinolanyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 4-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), dibenzothienyl (e.g., 2-dibenzothienyl), benzodioxolyl(e.g., 1,3-benzodioxolyl), and the like.

As used herein, the alkyl portion of "alkylcarbonyl" means the aforementioned "alkyl."

As used herein, the alkenyl portion of "alkenyloxycarbonyl" and "alkenylcarbonyl" means the aforementioned "alkenyl."

As used herein, the alkynyl portion of "alkynyloxycarbonyl" and "alkynylcarbonyl" means the aforementioned "alkynyl."

As used herein, the cycloalkyl portion of "cycloalkyloxycarbonyl" and "cycloalkylcarbonyl" means the aforementioned "cycloalkyl."

As used herein, the cycloalkenyl portion of "cycloalkenyloxycarbonyl" and "cycloalkenylcarbonyl" means the aforementioned "cycloalkenyl."

As used herein, the aryl portion of "aryloxycarbonyl" and "arylcarbonyl" means the aforementioned "aryl."

As used herein, the heteroaryl portion of "heteroaryloxycarbonyl" and "heteroarylcarbonyl" means the aforementioned "heteroaryl."

As used herein, the non-aromatic heterocyclic group portion of "non-aromatic heterocyclic group-oxycarbonyl" and "non-aromatic heterocyclic group-carbonyl" means the aforementioned "non-aromatic heterocyclic group."

As used herein, the term "arylsulfonyl" includes phenylsulfonyl, naphthylsulfonyl, and the like.

As used herein, substituents in "substituted or unsubstituted alkyl," "substituted or unsubstituted alkyloxy," "substituted or unsubstituted alkylthio," "substituted or unsubstituted alkylsulfonyl," and "substituted or unsubstituted alkyloxycarbonyl" include cycloalkyl, hydroxy, alkyloxy that may be substituted at 1 to 3 positions with a substituent selected from Substituent Group A, mercapto, alkylthio, a halogen atom, nitro, cyano, carboxy, alkyloxycarbonyl, substituted or unsubstituted amino, substituted or unsubstituted carbamoyl, acyl, aryl (e.g., phenyl) that may be substituted at 1 to 3 positions with a substituent selected from Substituent Group B, heteroaryl (e.g., pyridyl, furyl, thienyl, imidazolyl, oxazolyl, thiazolyl, and pyrazolyl) that may be substituted at 1 to 3 positions with a substituent selected from Substituent Group C, non-aromatic heterocyclic group (e.g., morpholinyl, pyrrolidinyl, and piperazinyl) that may be substituted at 1 to 3 positions with a substituent selected from Substituent Group C, alkylsulfonyl, and the like. These may be substituted with 1 to 3 substituents at any possible position.

As used herein, substituents in "substituted or unsubstituted alkenyl," "substituted or unsubstituted alkynyl," and "substituted or unsubstituted cycloalkyl" include alkyl that may be substituted at 1 to 3 positions with a substituent selected from Substituent Group D, cycloalkyl, hydroxy, alkyloxy that may be substituted at 1 to 3 positions with a substituent selected from Substituent Group A, mercapto, alkylthio, a halogen atom, nitro, cyano, carboxy, alkyloxycarbonyl, substituted or unsubstituted amino, substituted or unsubstituted carbamoyl, acyl, aryl (e.g., phenyl) that may be substituted at 1 to 3 positions with a substituent selected from Substituent Group B, heteroaryl (e.g., pyridyl, furyl, thienyl, imidazolyl, oxazolyl, thiazolyl, pyrazolyl) that may be substituted at 1 to 3 positions with a substituent selected from Substituent Group C, non-aromatic heterocyclic group (e.g.,morpholinyl, pyrrolidinyl, piperazinyl) that may be substituted at 1 to 3 positions with a substituent selected from Substituent Group C, alkylsulfonyl, and the like. These may be substituted with one or more substituents at any possible position.

As used herein, substituents in "substituted or unsubstituted aryl," "substituted or unsubstituted arylsulfonyl," "substituted or unsubstituted heteroaryl," "substituted or unsubstituted 5-membered ring heteroaryl," and "substituted or unsubstituted non-aromatic heterocyclic group" include alkyl that maybe substituted at 1 to 3 positions with a substituent selected from Substituent Group D, cycloalkyl, alkenyl, alkynyl, hydroxy, alkyloxy that may be substituted at 1 to 3 positions with a substituent selected from Substituent Group A, aryloxy (e.g., phenoxy) that may be substituted at 1 to 3 positions with a substituent selected from Substituent Group B, mercapto, alkylthio, a halogen atom, nitro, cyano, carboxy, alkyloxycarbonyl, acyl, alkylsulfonyl, substituted or unsubstituted amino, substituted or unsubstituted carbamoyl, aryl (e.g., phenyl) that may be substituted at 1 to 3 positions with a substituent selected from Substituent Group B, heteroaryl (e. g. , pyridyl, furyl, thienyl, imidazolyl, oxazolyl, thiazolyl, pyrazolyl) that may be substituted at 1 to 3 positions with a substituent selected from Substituent Group C, non-aromatic heterocyclic group (e.g., morpholinyl, pyrrolidinyl, and piperazinyl) that may be substituted at 1 to 3 positions with a substituent selected from Substituent Group C, and the like. These may be substituted with one or more substituents at any possible position.

Here, Substituent Group A is a halogen atom and phenyl that may be substituted at 1 to 3 positions with a substituent selected from Substituent Group B; Substituent Group B is a halogen atom, alkyl, alkyloxy, cyano and nitro; Substituent Group C is a halogen atom and alkyl; and Substituent Group D is a halogen atom and alkyloxy.

Pharmaceutically acceptable salts of the compound of the present invention include pharmaceutically acceptable acid addition salts, metal salts, ammonium salts, organic amine addition salts, amino acid addition salts, and the like. Pharmaceutically acceptable acid addition salts of the compound (I) include, for example: inorganic acid salts such as hydrochlorides, sulfates, phosphates, and the like; and organic acid salts such as acetates, maleates, fumarates, tartrates, citrates, methanesulfonates, and the like. Pharmaceutically acceptable metal salts include, for example: alkali metal salts such as sodium and potassium salts and the like; alkaline-earth metal salts such as magnesium and calcium salts and the like; aluminum salts; zinc salts; and the like. Pharmaceutically acceptable ammonium salts include, for example, salts of ammonium, tetramethylammonium, and the like. Pharmaceutically acceptable organic amine addition salts include addition salts of morpholine, piperidine, and the like. Pharmaceutically acceptable amino acid addition salts include addition salts of lysine, glycin, phenylalanine, and the like.

Pharmaceutically acceptable hydrates of the compound of the present invention include compounds effective in a hydrate form, and compounds effective after dehydration. In the present invention, both can be used.

As a pharmaceutically acceptable prodrug of the present invention, any form publicly known in the field of the art can be adopted. A prodrug refers to a compound that, taking advantage of a metabolic machinery *in vivo,* does not exhibit a pharmaceutical effect or merely exhibits very low activity in its original form, but is modified so as to, when metabolized *in vivo,* thereby exhibit or increase pharmacological activity for the first time. Examples of prodrugs include not only salts, solvates, and the like, but also esters, amides, and the like.

The compound of the present invention may be in a solvate form. Examples thereof include solvates with alcohol (e.g.: ethanol).

(Production Method)

Hereinafter, a method for producing the compound of the present invention is described.
A compound represented by the formula:

[Chem. 21]

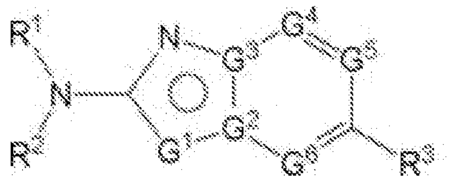

(I)

(wherein the definition of each substituent is as defined herein), can be synthesized by reference to a method publicly known in the relevant field.

Here, the cyclic group represented by the formula:

[Chem. 22]

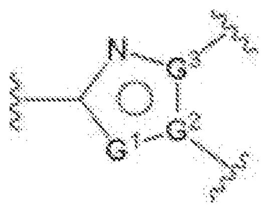

is a cyclic group represented by any of the following formulas (A) - (D):

[Chem. 23]

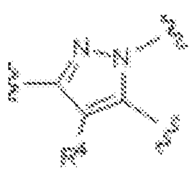 , 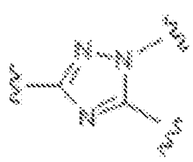 , 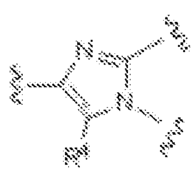 , and 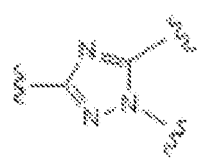

(A) (B) (C) (D)

For example, in the case of:

[Chem. 24]

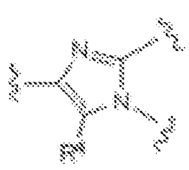

(C) ,

on the basis of Synthesis 1998, 867 as described on page 63 of Patent Literature 3 (Pamphlet of International Publication No. WO 2007/095588), the compound represented by the formula:

[Chem. 25]

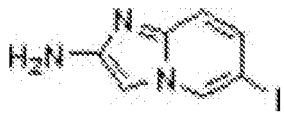

can be synthesized. The bromo-compound can be also synthesized as described on page 65 of Patent Literature 3. Furthermore, a compound in which the six-membered ring contains two nitrogen atoms can be also similarly synthesized. For example, Compounds 1 and 2 can be synthesized by a method described on pages 63 and 64 of Patent Literature 3.

[Chem. 26]

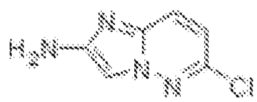

1

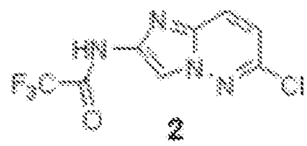

2

The trifluoroacetamide compound, which is the protected compound similar to this Compound 2, can be synthesized by reference to page 64 of Patent Literature 3.

[Chem. 27]

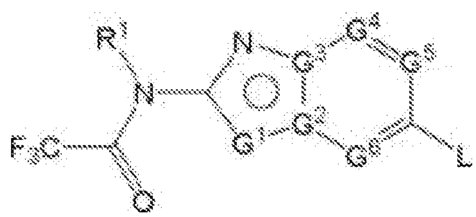

(The substituents are the same as above. L is a leaving group, typically representing halogen (I, Br, Cl, F, and the like), lower (wherein lower typically means C1-C6) alkoxy, lower alkylthio, lower alkylsulfonyloxy, arylsulfonyloxy, or the like.)

Using this trifluoroacetamide compound and a compound of which $R^3$ is boronic acid, or the like, the compound of the present invention can be synthesized. Here, to these two rawmaterials is added a coupling catalyst such as Pd $(PPh_3)_4$ and the like in an appropriate inactive solvent, for example lower alcohol such as methanol, ethanol, isopropanol, and the like, halogenated hydrocarbon such as chloroform, dichloromethane, and the like, aromatic hydrocarbon such as benzene, toluene, and the like, ether solvent such as diethyl ether, THF, 1,4-dioxane, and the like, aprotic polar solvent such as dimethylformamide, N-methylpyrrolidone, dimethylsulfoxide, and the like, or a mixed solvent thereof, a reaction in the presence of a base at a temperature between room temperature and 180 degrees Celsius for 10 minutes to 48 hours can yield it. Examples of bases include: organic bases such as triethylamine, pyridine, and the like; inorganic bases such as potassium carbonate, sodium carbonate, cesium carbonate, potassium phosphate, sodium hydroxide, and the like; solutions thereof; metal alkoxide such as sodium methoxide, potassium t-butoxide; and the like.

A similar protected compound, in the compound represented by the formula:

[Chem. 28]

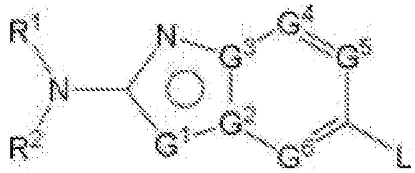

(wherein the substituents are the same as above, and L is a leaving group, and typically represents halogen (I, Br, Cl, F, and the like), lower (wherein lower typically means, but is not limited to, C1-C6) alkoxy, lower alkylthio, lower alkylsulfonyloxy, arylsulfonyloxy, or the like), can be produced by protecting an optional substituent of $R^1$ or $R^2$ by a method publicly known in the relevant field. Examples of such substituents can include protecting groups, such as ethoxycarbonyl, t-butoxycarbonyl, acetyl, benzyl, and the like, which are described in Protective Groups in Organic Synthesis, written by T. W. Green, John Wiley & Sons Inc. (1981), or the like. Methods for the introduction and removal of a protecting group are methods commonly used in organic synthetic chemistry, methods described in [see, for example, Protective Groups in Organic Synthesis, written by T. W. Greene, John Wiley & Sons Inc. (1981)] or the like, or can be obtained in accordance therewith. Furthermore, a functional group included in each substituent can be converted by a publicly known method [for example, Comprehensive Organic Transformations, written by R. C. Larock (1989), and the like] in addition to the above producing methods. Some of the compound of the present invention can be used as a synthetic intermediate to further be lead to a new derivative. Intermediates and target compounds in each of the above producing methods can be isolated and purified by a purification method commonly used in organic synthetic chemistry, for example, subjecting them to neutralization, filtration, extraction, washing, drying, concentration, recrystallization, every kind of chromatography, or the like. Furthermore, intermediates can be subjected to a next reaction without purification in particular.

As a raw material compound, a compound commercially available, one described in Patent Literature 3, Patent Literature 4, Patent Literature 5, Non-Patent Literature 14, Non-Patent Literature 15, Non-Patent Literature 16, Patent Literature 6, Patent Literature 7, Patent Literature 8, Patent Literature 9, Patent Literature 10, Non-Patent Literature 17, Patent Literature 11, Non-Patent Literature 18, Non-Patent Literature 19, Patent Literature 12, or Patent Literature 13, one described herein, one described in other cited references herein, or another one publicly known can be utilized.

Regarding some of the compound of the present invention, a tautomer thereof may exist. However, the present invention encompasses all possible isomers, including these, and mixtures thereof.

When a salt of the compound of the present invention is desired to be obtained, in the case that the compound of the present invention is obtained in salt form, it may be purified as it is. Furthermore, in the case that it is obtained in free form, after it is dissolved or suspended in an appropriate organic solvent and then acid or base is added thereto, a salt may be formed by a general method.

Furthermore, the compound of the present invention and a pharmaceutically acceptable salt thereof may exist in form of adduct with water or every kind of solvent (hydrate or solvate). These adducts are also encompassed by the present invention.

Derivatives thereof is converted in the body to be activated, and are named "prodrug" herein. It is understood that Examples of prodrugs includes not only the aforementioned salts and solvates, but also esters, amides, and the like.

Various examples of the compound of the present invention are listed in Examples. By reference to these, those skilled in the art can produce and use compounds that are not exemplified in the present invention.

The present invention is also related to a system, an apparatus, and a kit for producing the compound of the present invention. It is understood that, as elements of such a system, an apparatus, and a kit, matters publicly known in the relevant field are available, and those skilled in the art can appropriately design them.

(Medicament)

The compound of the present invention or a pharmaceutically acceptable salt can be administered alone as it is, but it is usually preferable to provide it as every kind of pharmaceutical formulation. Furthermore, those pharmaceutical formulations are used for an animal and a human.

With regard to an administration route, it is preferable to use the most effective route on treatment. It can be peroral administration, or parenteral administration, for example, intrarectal, intraoral, subcutaneous, intramuscular, intravenous, or the like.

Administration forms include capsule, tablet, granule, powder, syrup, emulsion, suppository, injection, and the

like. A liquid preparation, such as emulsion and syrup, which is suitable for oral administration, can be produced using: water; sugars such as sucrose, sorbite, fructose, and the like; glycols such as polyethylene glycol, propylene glycol, and the like; oils such as sesame oil, olive oil, soybean oil, and the like; antiseptics such as p-hydroxybenzoate esters, and the like; and flavors such as strawberry flavor, peppermint, and the like. Furthermore, a capsule, a tablet, a powder, a granule, and the like can be produced using: an excipient such as lactose, glucose, sucrose, mannite, and the like; a disintegrator such as starch, sodium alginate and the like; a lubricant such as magnesium stearate, talc, and the like; a binder such as polyvinyl alcohol, hydroxypropylcellulose, gelatin, and the like; surfactant such as fatty ester and the like; and a plasticizer such as glycerin and the like.

A formulation suitable for parenteral administration preferably consists of a sterilized-water-based formulation containing an active compound and being isotonic to blood of a recipient. For example, in the case of injection, a solution for an injection is prepared using: a carrier consisting of a salt solution, a glucose solution, or a mixture of salt water and a glucose solution; and the like.

A topical formulation is prepared by dissolving or suspending an active compound in one or more kinds of media, such as mineral oil, petroleum, polyalcohol, and the like, or other bases used for a topical pharmaceutical formulation. A formulation for enteral administration is prepared using a general carrier such as cacao butter, hydrogenated fat, hydrogenated fatty carboxylic acid, and the like, and then provided as a suppository.

In the present invention, to a parenteral agent can be added one or more kinds of auxiliary ingredients selected from glycols, oils, flavors, antiseptics (including antioxidants), excipients, disintegrators, lubricants, binders, surfactants, plasticizer, and the like exemplified in an oral agent.

An effective dose and the frequency of administration of the compound of the present invention or a pharmaceutically acceptable salt are different according to administration form, the age of a patient, weight, characteristics or the severity, and the like of a condition to be treated. Generally, a dose is 0.01 to 1000 mg/person per day, preferably 5-500 mg/person per day, and a frequency of administration is preferably once per day or divided administration.

All of the compounds of the present invention is are immediately applicable to therapeutic use as a kinase inhibitor for controlling kinase dependent diseases in mammals, particularly, a kinase inhibitor related to phosphatidylinositol-3-kinase.

The compound of the present invention is preferably a compound having an $IC_{50}$ value in a range of 0.1 nM to 10 $\mu$M. A certain compound of the present invention wherein the compound is capable of specifically inhibiting one of four types of Class I phosphatidylinositol-3-kinase (e.g., $\alpha$, $\beta$, $\gamma$, and $\delta$) can be selected. For example, by utilizing a compound selectively inhibiting only y type, merely diseases related to inflammation, such as a lymphocyte and the like can be treated. In the case that a compound is $\alpha$-type selective, the utility as a selective anticancer agent can be found.

Phosphatidylinositol-3-kinase dependent diseases include inflammatory diseases (allergic diseases (allergic dermatitis/allergic rhinitis, and the like), articular rheumatism, anaphylaxis, and the like), arteriosclerosis, vascular/circulatory diseases, cancer/tumors (hyperproliferative malfunction), immune system diseases, cell-proliferative diseases, infectious diseases, and the like initiated/maintained by unusual phosphatidylinositol-3-kinase enzyme activity. For example, psoriasis, pulmonary fibrosis, glomerulonephritis, cancers, atherosclerosis, and antiangiogenesis (e.g., tumor growth, diabetic retinopathy) are included. Specifically, for example, the pharmaceutical composition of the present invention may be used for the prevention and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastrojejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, scleroderma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, sarcoidosis, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The present invention is also related to a system, an apparatus, and a kit for producing the pharmaceutical composition of the present invention. It is understood that, as elements of such a system, an apparatus, and a kit, matters publicly known in the relevant field are available, and those skilled in the art can appropriately design them.

The present invention is also related to a system, an apparatus, and a kit using the compound of the present invention, a pharmaceutically acceptable salt thereof, or a prodrug thereof including a solvate thereof and the like. It is understood that, as elements of such a system, an apparatus, and a kit, matters publicly known in the relevant field are available, and those skilled in the art can appropriately design them.

Wortmannin, which is a classical PI3K inhibitor, has low inhibition selectivity, high toxicity, and the like, and consequently is highly cytotoxic. Thus, by using a usual test to measure cytotoxicity, a PI3K inhibitor (or another class of a kinase inhibitor) that intends to cause an unpreferable side effect due to lack of the selectivity can be identified.

The compound of the present invention is a compound havingutilityas amedicament. Here, utility as amedicament includes the following points: the compound has good metabolic stability; the induction of a drug-metabolizing enzyme is low; the inhibition of a drug-metabolizing enzyme which metabolizes another drug is also low; the compound has high oral absorbency; the clearance is low; the half-life is sufficiently long to express the efficacy; or the like.

Reference literature including scientific literature, patents, patent applications, and the like cited herein is incorporated herein by reference in its entirety at the same level as the case where each reference is specifically described.

Hereinafter, Examples describe the constitution of the present invention in more detail, but the present invention is not limited thereto. Regarding reagents and the like used below, unless specified otherwise, those commercially available are used.

EXAMPLES

Hereinafter, the present invention is described in more detail with working examples and experimental examples. However, the present invention is not limited to them.

In Examples, abbreviations described below are used.

DMA: Dimethylacetamide
DMSO: Dimethylsulfoxide
HPLC: High Performance Liquid Chromatography
Me: Methyl
Ph: Phenyl
MBI: Mechanism-Based Inhibition
FAT: Fluctuation Ames Test

The LC-MS of compounds were measured under a condition described below, and the retention times and [M+H] are shown. Column: Phenomenex Luna 5 μM C18(2) 100 Å (50 x 4.6 mm) Flow rate: 3 mL/min
UV detection wavelength: 254 nm
Mobile phase: [A] is aqueous 0.1% formic-acid-containing solution, and [B] is 0.1% formic-acid-containing solution in methanol.
A gradient of a mobile phase from a mixed solution of 90% of [A] and 10% of [B] at a time of 0 minute to a solution of 100% of [B] after 3 minutes was used.

Compounds 1 and 2 were synthesized by a method described on pages 63 and 64 of Patent Literature 3 (Pamphlet of International Publication No. WO 2007/095588).

[Chem. 29]

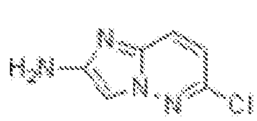

1

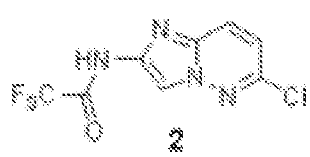

2

(Reference Example 1 Synthesis of Compound 3)

[Chem. 30]

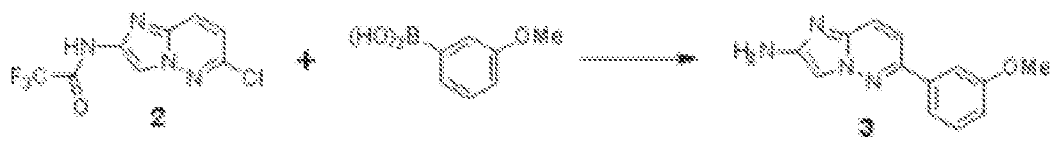

2 3

To a solution of a compound (2, 2.65 g, 10 mmol), 3-methyloxyphenylboronic acid (2.279 g, 15 mmol), and Pd $(PPh_3)_4$ (1.156 g, 1.0 mmol) in dioxane (18 mL) was added aqueous 2 mol/L sodium carbonate solution (15 mL). The reaction solution was then stirred under nitrogen atmosphere at reflux for 1 hour and 30 minutes. The reaction solution was cooled down to room temperature, filtrated through a Celite, and then washed with a mixed solution of ethyl acetate and water. The filtrate was concentrated *in vacuo,* the residue was extracted with ethyl acetate, and then the organic layer was washed with saturated brine. The solvent was concentrated *in vacuo,* and then purified by silica gel chromatography to yield a compound (3, 2.26 g, 94% yield).

LC-MS: 2.10 min, [M+H] = 243

(Example 1 Synthesis of Compound I-1)

[Chem. 31]

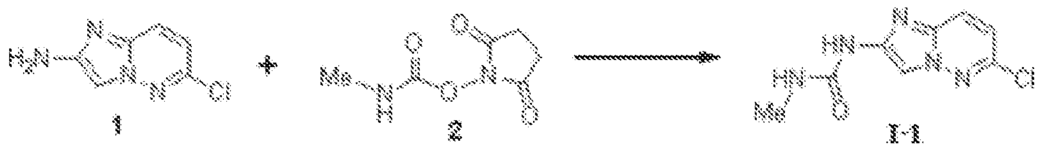

1 2 I-1

A solution of the compound (1, 7.11 g, 42.2 mmol) obtained in Reference Example 1 and a compound (2, 21.78 g, 126 mmol) in pyridine (200 mL) was stirred at 70 degrees Celsius for 9 hours. The reaction solution was then concentrated *in vacuo.* The resulting crystals were filtrated, washed with water, and then dried. The filtrate was then extracted with ethyl acetate. The organic layer was washed with saturated brine, and then dried with anhydrous magnesium sulfate. The solvent was then concentrated *in vacuo.* The residue was mixed with the previous crystals, washed with methanol, and then dried to yield a compound (I-1, 7.51 g, 33.3 mmol).
LC-MS: 1.07 min, [M+H] = 226

(Example 2 Synthesis of Compounds I-2 and I-3)

[Chem. 32]

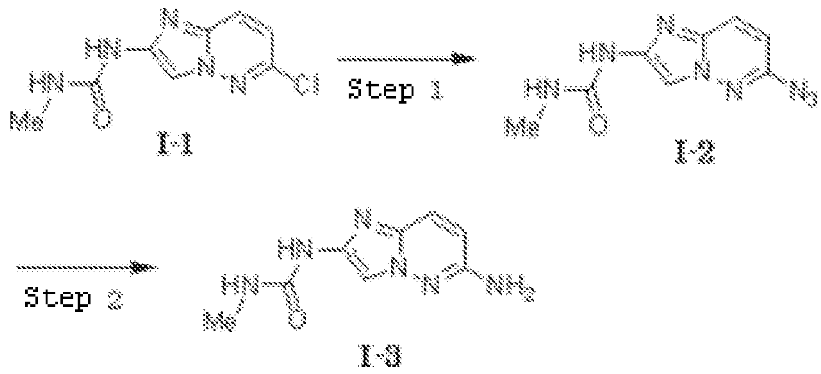


I-1 I-2 I-3

Step 1

To a solution of the compound (I-1, 500 mg, 2.22mmol) in hexamethylphosphoric triamide (4 mL) was added sodium azide (1.44 g, 22.2 mmol) and sodium methanesulfinate (226 mg, 2.22 mmol) at room temperature. The reaction solution was then stirred at 110 degrees Celsius for 30 hours. Water was then added to the reaction solution, followed by extraction with ethyl acetate. The organic layer was washed with water and saturated brine, and then dried with anhydrous magnesium sulfate. The solvent was then concentrated *in vacuo* to yield a crude product (I-2).
LC-MS: 1.02 min, [M+H] = 233

Step 2

To a solution of the crude product (I-2) obtained in Step 1 in ethanol (30 mL) was added 10% palladium on carbon (250 mg) at room temperature. The reaction solution was then stirred under hydrogen atmosphere at room temperature for 2 hours. The reaction solution was filtrated, and then the filtrate was concentrated *in vacuo.* The residue was purified by column chromatography to yield a compound (I-3, 113.4 mg, 0.55 mmol).
LC-MS: 0.27 min, [M+H] = 207

(Example 3 Synthesis of Compound I-4)

[Chem. 33]

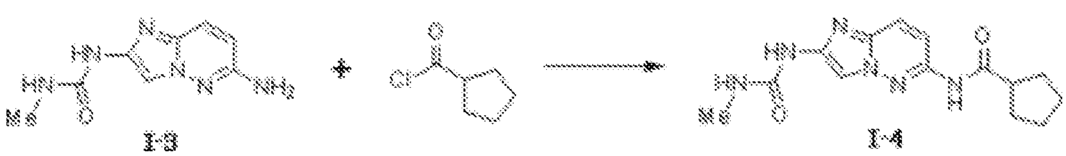

I-3 I-4

To a solution of the compound (I-3, 20 mg, 0.097 mmol) obtained in Step 2 of Example 2 in tetrahydrofuran (2 mL) was added pyridine (77 mg, 0.97 mmol) and cyclopentanecarboxylic acid chloride (38.6 mg, 0.29 mmol) at room temperature. After the reaction solution was stirred at room temperature for 5 hours, saturated brine was added thereto. The reaction solution was extracted with ethyl acetate, and then the organic layer was dried with anhydrous magnesium sulfate. The solvent was concentrated *in vacuo,* and then the residue was purified by column chromatography to yield a compound (I-4, 18.5 mg, 0.061 mmol).
LC-MS: 1.18 min, [M+H] = 303

(Example 4 Synthesis of Compound I-5)

[Chem. 34]

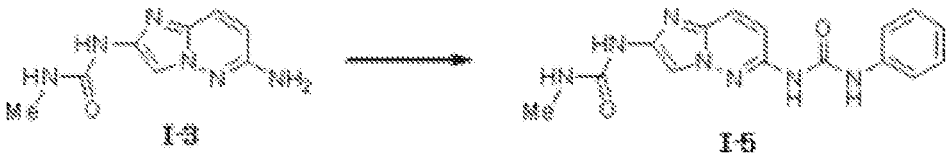

I-3 I-5

To a solution of the compound (I-3, 20 mg, 0.097 mmol) obtained in Step 2 of Example 2 in tetrahydrofuran (2 mL) was added phenyl isocyanate (34.7 mg, 0.29 mmol) at room temperature. The reaction solution was then stirred at room temperature for 10 hours and further at 50 degrees Celsius for 2 hours. The solvent was concentrated *in vacuo,* and then the residue was purified by column chromatography to yield a compound (I-5, 5.3 mg, 0.016 mmol).
LC-MS: 1.20 min, [M+H] = 326

(Example 5 Synthesis of Compound I-6)

[Chem. 35]

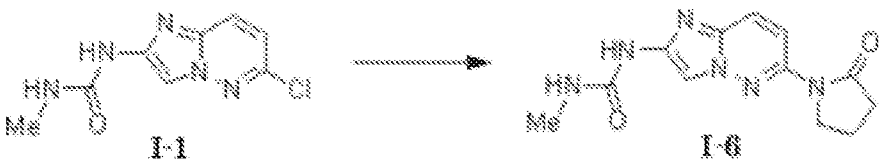

I-1 I-6

Under nitrogen atmosphere, the compound (I-1, 70 mg, 0.31 mmol) obtained in Example 1, 2-pyrrolidinone (31.7 mg, 0.372 mmol), 9,9-dimethyl 4,5-bis(diphenylphosphino)xanthene (26.9 mg, 0.047 mmol), palladium acetate (6.97mg, 0.031 mmol), and cesium carbonate (142 mg, 0.434 mmol) were suspended in 1,4-dioxane (3 mL). The reaction solution was stirred at 100 degrees Celsius for 5 hours, and then cooled down to room temperature. Ethyl acetate was then added thereto. The reaction solution was filtrated, and then the filtrate was concentrated *in vacuo.* The residue was purified by column chromatography to yield a compound (I-6, 19 mg, 0.069 mmol).
LC-MS: 0.86 min, [M+H] = 275

(Example 6 Synthesis of Compound I-7)

[Chem. 36]

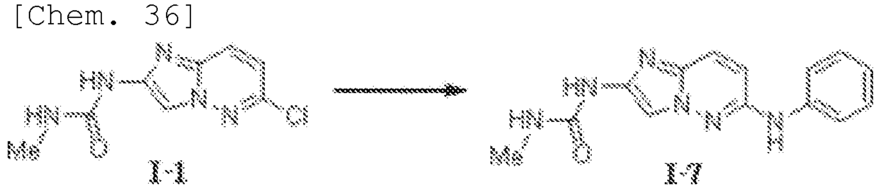

I-1 I-7

Under nitrogen atmosphere, the compound (I-1, 70 mg, 0.31 mmol) obtained in Example 1, 9,9-dimethyl 4,5-bis(diphenylphosphino)xanthene (26.9 mg, 0.047 mmol), palladium acetate (6.97 mg, 0.031 mmol), and cesium carbonate (142 mg, 0.434 mmol) were suspended in 1,4-dioxane (3 mL). To the reaction solution was added aniline (34.7 mg, 0.372 mmol), subsequently stirring at 100 degrees Celsius for 8 hours. The reaction solution was cooled down to room temperature, and then ethyl acetate was added thereto, followed by filtration. The filtrate was concentrated *in vacuo,* and then the residue was purified by column chromatography to yield a compound (I-7, 40.2mg, 0.142 mmol).
LC-MS: 1.11 min, [M+H] = 283

(Example 7 Synthesis of Compound I-8)

[Chem. 37]

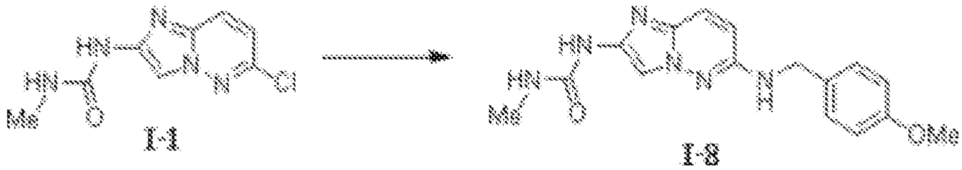

I-1 I-8

Under nitrogen atmosphere, the compound (I-1, 50 mg, 0.22 mmol) obtained in Example 1, 2,2'-bis(diphenylphosphino)-1,1'-binaphthyl (20.7 mg, 0.033 mmol), palladium acetate (4.98 mg, 0.022 mmol), and sodium tert-butoxide (29.8 mg, 0.31 mmol) were suspended in 1,4-dioxane (2 mL). To the reaction solution was added 4-methoxybenzylamine (36.5 mg, 0.266 mmol), subsequently stirring at 100 degrees Celsius for 8 hours. The reaction solution was cooled down to room temperature, and then water was added thereto, followed by extraction with ethyl acetate. The organic layer was washed with saturated brine, and then dried with anhydrous magnesium sulfate. The solvent was concentrated *in vacuo,* and then the residue was purified by column chromatography to yield a compound (I-8, 19.5 mg, 0.06 mmol).
LC-MS: 1.05 min, [M+H] = 327

(Example 8 Synthesis of Compound I-9)

[Chem. 38]

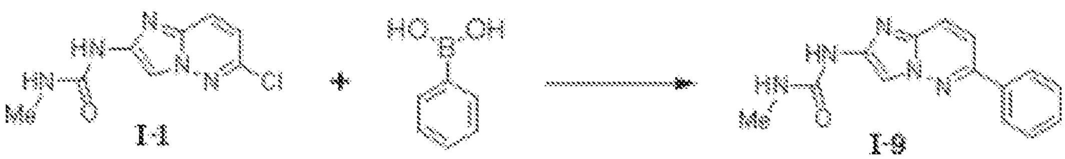

I-1 I-9

To a solution of phenylboronic acid (14.6 mg, 0.12 mmol) and the compound (I-1, 20 mg, 0.089 mmol) in dioxane (500 µL) was added a solution of [1,3-bis(2,6-diisopropylphenyl)imidazol-2-ylidene](3-ch loropyridyl)palladium (II) dichloride (3.01 mg, 4.43pmol)in dioxane (500 µL) and aqueous 2 mol/L potassium carbonate solution (177 µL, 0.355 mmol). For the reaction solution, displacement with nitrogen was carried out, subsequently stirring at 100 degrees Celsius for 5 hours. The reaction solution was filtered through a silica gel pad, and then purified by reversed phase HPLC to yield a compound (I-9, 2.84 mg, 11% yield).
LC-MS: 1.73 min, [M+H] = 268

(Example 9 Synthesis of Compound I-10)

[Chem. 39]

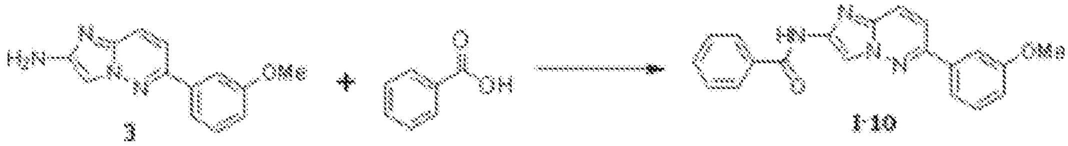

3 I-10

To a solution of benzoic acid (13.2 mg, 0.108 mmol) and O-(7-azabenzotriazol-1-yl)-N,N,N',N'-tetramethyluronium hexafluorophosphate (38mg, 0.100 mmol) in dimethylformamide (500 μL) was added the compound (3, 20 mg, 0.083 mmol) obtained in Reference Example 3 and triethylamine (23 μL, 0.166 mmol). The reaction solution was stirred at room temperature for 3 hours, and then purified by reversed phase HPLC to yield a compound (I-10, 11.5 mg, 40% yield).
LC-MS: 2.09 min, [M+H] = 345

(Example 10 Synthesis of Compounds I-11 and I-12)

[Chem. 40]

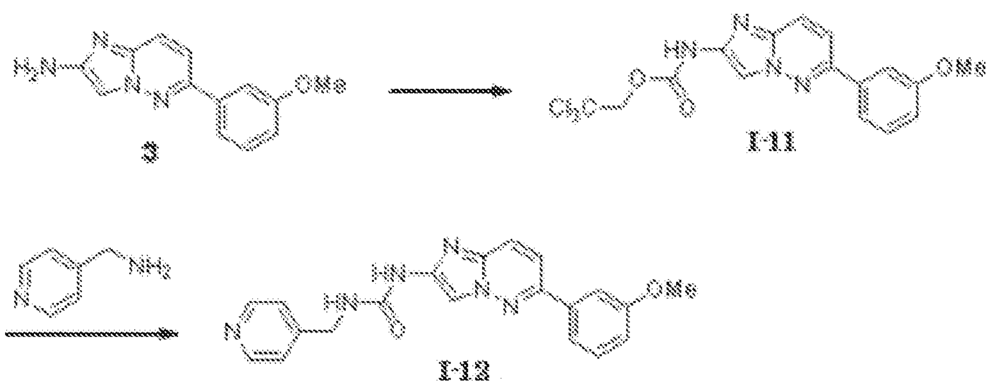

3

I-11

I-12

Step 1

To a solution of the compound (3, 3.00 g, 12.5 mmol) in DMA was added drop-wise 2,2,2-trichloroethyl chloroformate ester (3.17 g, 15.0 mmol) under ice-cooling, subsequently stirring at room temperature for 30 minutes. After 20g of ice water was added to the reaction solution, the resulting solid was filtrated, washed with water, and then dried to yield a compound (I-11, 2.43 g, 47% yield).

Step 2

A solution of the compound (I-11, 20 mg, 0.048 mmol) obtained in Step 1, 4-pyridylmethylamine (7.81 mg, 0.072 mmol) in DMSO (1.00 mL) was stirred at 100 degrees Celsius for 8 hours. The reaction solution was filtrated, the filtrate was concentrated *in vacuo,* and then the residue was purified by reversed phase HPLC to yield a compound (I-12, 5.8 mg, 31% yield).
LC-MS: 1.95 min, [M+H] = 380

(Example 11 Synthesis of Compounds I-13, I-14, and I-15)

[Chem. 41]

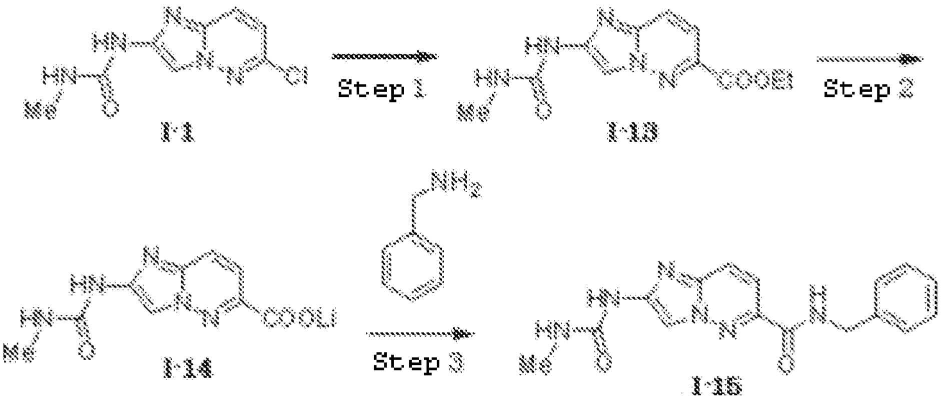


I-1 I-13 I-14 I-15

Step 1

To a suspension of the compound (I-1, 50.1 mg, 0.222 mmol) obtained in Example 1 in ethanol (1.5 mL)/dimethylformamide (0.3 mL) was added [1,1'-bis(diphenylphosphino)ferrocene]dichloropalladium (II) dichloromethane adduct (18.1 mg, 0.022 mmol) and sodium acetate (36.4 mg, 0.444 mmol), subsequently stirring under carbon monoxide atmosphere at 70 degrees Celsius for 7 hours. The reaction solution was concentrated *in vacuo,* and then extracted with ethyl acetate. The organic layer was washed with water and saturated brine, and then dried with anhydrous magnesium sulfate. The solvent was concentrated *in vacuo,* and then the residue was purified by silica gel column chromatography (chloroform:methanol = 100:0 to 95:5) to yield a compound (I-13, 51.5 mg, 88% yield).
LC-MS: 1.05 min, [M+H] = 264

Step 2

To a suspension of the compound (I-13, 126. 5 mg, 0.481 mmol) obtained in Step 1 in tetrahydrofuran (4. 0 mL) /methanol (4.0 mL) was added aqueous 1 mol/L lithium hydroxide solution (577 μL, 0.577 mmol), subsequently stirring at room temperature for 1 hour and 30 minutes. The reaction solution was concentrated *in vacuo,* and then the residue was dried under reduced pressure at 50 degrees Celsius for 5 hours to yield a crude product (I-14).
LC-MS: 0.64 min, [M+H] = 236

Step 3

To a suspension of the crude product (I-14, 25.0 mg) obtained in Step 2 in N,N-dimethylformamide (500 μL) was added benzylamine (17.8 mg, 0.166 mmol), O-(7-azabenzotriazoltriazol-1-yl)-N,N,N',N'-tetramethyl uronium hexafluorophosphate (44 mg, 0.117 mmol), and N-methylmorpholine (25.7 μL, 0.234 mmol). The reaction solution was stirred under nitrogen atmosphere at room temperature overnight, and then purified by reversed phase HPLC to yield a compound (I-15, 11.5 mg, 39% yield). LC-MS: 1.65 min, [M+H] = 325

(Example 12 Synthesis of Compounds I-16 and I-17)

[Chem. 42]

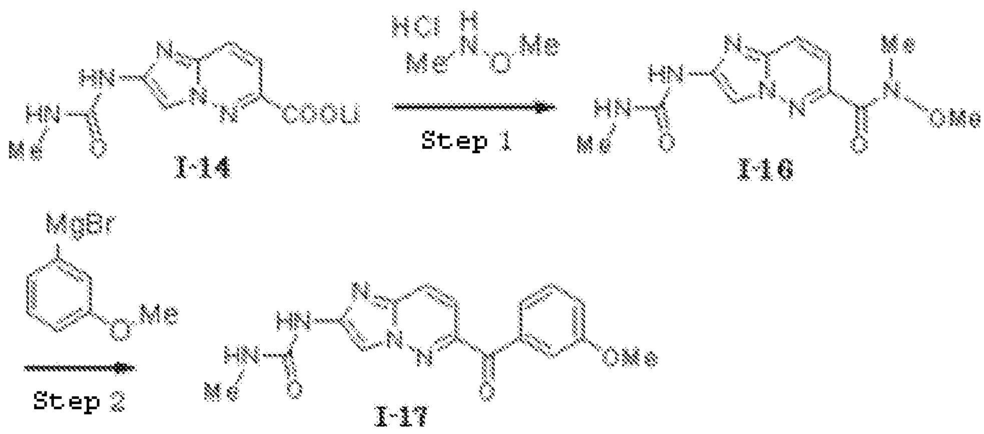


I-14 I-16 I-17

Step 1

To a suspension of the crude product (I-14, 61.3 mg) obtained in Step 2 of Example 11 in N,N-dimethylformamide (1.5 mL) was added O,N-dimethylhydroxylamine hydrochloride (28.7 mg, 0.294 mmol), O-(7-azabenzotriazol-1-yl)-N, N,N',N'-tetramethyluronium hexafluorophosphate (129 mg, 0.339 mmol), and N-methylmorpholine (99 μL, 0.904 mmol). The reaction solution was stirred under nitrogen atmosphere at room temperature for 1 hour and 30 minutes, and then purified by reversed phase HPLC to yield a compound (1-16, 60.0 mg, 95% yield).
LC-MS: 0.74 min, [M+H] = 279

Step 2

To a suspension of the compound (I-16, 28.8 mg, 0.103 mmol) obtained in Step 1 in tetrahydrofuran (1.0 mL) was added 1.0 mol/L 3-methoxyphenyl magnesium bromide solution in tetrahydrofuran (621 μL, 0.621 mmol) under nitrogen atmosphere at -78 degrees Celsius, subsequently stirring at -45 degrees Celsius for 2 hours and 30 minutes. To the reaction solution was added aqueous saturated ammonium chloride solution, and then extracted with ethyl acetate. The organic layer was washed with water and saturated brine, and then dried with anhydrous magnesium sulfate. The solvent was concentrated *in vacuo,* and then the residue was purified by silica gel column chromatography (chloroform:methanol = 100: 0 → 97:3) to yield a compound (I-17, 20.2 mg, 60% yield). LC-MS: 1.47 min, [M+H] = 326

(Example 13 Synthesis of Compound I-18)

[Chem. 43]

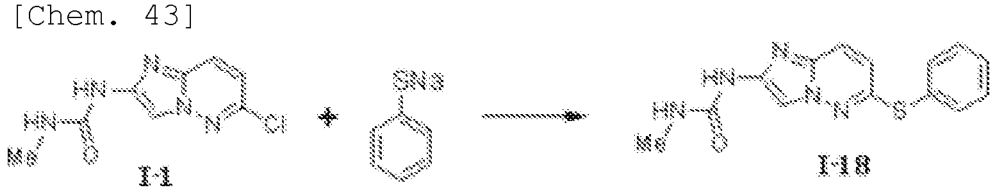

I-1 I-18

To a suspension of the compound (I-1, 57.0 mg, 0.253 mmol) obtained in Example 1 in N,N-dimethylformamide (1.0 mL) was added thiophenol sodium salt (100.2 mg, 0.758 mmol), subsequently stirringunder nitrogen atmosphere at 60 degrees Celsius for 7 hours and 30 minutes. Water was added to the reaction solution, followed by extraction with ethyl acetate. The organic layer was washed with water and saturated brine, and then dried with anhydrous magnesium sulfate. The solvent was concentrated *in vacuo,* and then the residue was purified by reversed phase HPLC to yield a compound (I-18, 21.8 mg, 29% yield).
LC-MS: 1.62 min, [M+H] = 300

(Example 14 Synthesis of Compound I-19)

[Chem. 44]

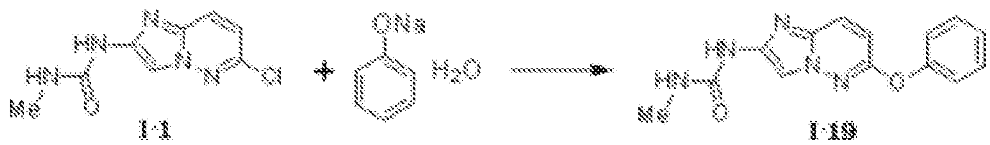


I-1 I-19

To a suspension of the compound (I-1, 90.1 mg, 0.399 mmol) obtained in Example 1 in N,N-dimethylformamide (1.0 mL) was added phenol sodium salt trihydrate (407.6 mg, 2.40 mmol) and hexamethylphosphoric triamide (417 μL, 2.40 mmol), subsequently stirring under nitrogen atmosphere at 100 degrees Celsius for 9 hours. Water was added to the reaction solution, followed by extraction with ethyl acetate. The organic layer was washed with water and saturated brine, and then dried with anhydrous magnesium sulfate. The solvent was concentrated *in vacuo,* and then the residue was purified by reversed phase HPLC to yield a compound (1-19, 12.8 mg, 11% yield).
LC-MS: 1.41 min, [M+H] = 284

(Example 15 Synthesis of Compound I-20)

[Chem. 45]

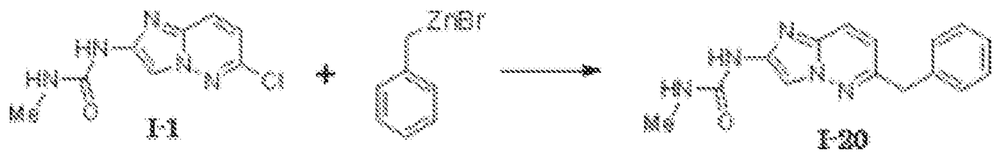

I-1 I-20

To a suspension of the compound (I-1, 57.4 mg, 0.254 mmol) obtained in Example 1 in tetrahydrofuran (1.5 mL) was added 0.5 mol/L benzylzinc bromide solution in tetrahydrofuran (2.14 mL, 1.07 mmol) and tetrakis(triphenylphosphine)palladium (14.7 mg, 0.013 mmol) under nitrogen atmosphere at room temperature, subsequently stirring at 60 degrees Celsius for 7 hours. To the reaction solution was added aqueous saturated ammonium chloride solution, followed by extraction with ethyl acetate. The extract was washed with water and saturated brine, and then dried with anhydrous magnesium sulfate. The solvent was concentrated *in vacuo,* and then purified by reversed phase HPLC to yield a compound (I-20, 14.8 mg, 21% yield). LC-MS: 1.40 min, [M+H] = 282

(Example 16 Synthesis of Compounds I-21 and I-22)

[Chem. 46]

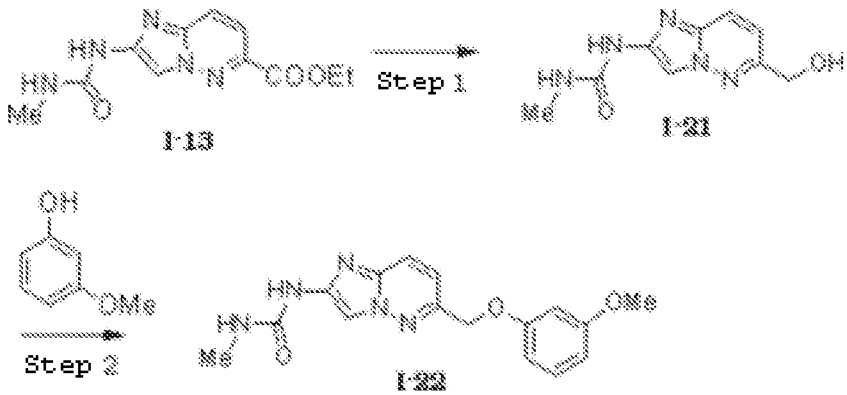


I-13 I-21 I-22

Step 1

To a suspension of the compound (I-13, 2.24 g, 8.51 mmol) obtained in Step 1 of Example 11 in tetrahydrofuran (40 mL)/methanol (20 mL) was added lithium tetrahydroborate (1.11 g, 51.1 mmol) under nitrogen atmosphere at 0 degree Celsius, subsequently stirring at room temperature for 2 hours and 30 minutes. To the reaction solution was added acetone at 0 degree Celsius, followed by stirring at room temperature for 1 hour. The reaction solution was filtrated, the filtrate was concentrated *in vacuo,* and then the residue was purified by silica gel column chromatography (chloroform:methanol = 100:0 → 85:15) to yield a compound (I-21, 1.56 g, 83% yield).
LC-MS: 0.46 min, [M+H] = 222

Step 2

To a suspension of the compound (I-21, 32.3 mg, 0.146 mmol) obtained in Step 1 in N,N-dimethylformamide (1.0 mL) was added 3-methoxyphenol (24 μL, 0.219 mmol), triphenylphosphine (54.7 mg, 0.219 mmol), and diisopropyl azodicarboxylate (43 μL, 0.219 mmol), subsequently stirring under nitrogen atmosphere at room temperature for 3 hours and 30 minutes. Purification by reversed phase HPLC yielded a compound (I-22, 23.3 mg, 49% yield).
LC-MS: 1.45 min, [M+H] = 328

(Example 17 Synthesis of Compounds I-23 and I-24)

[Chem. 47]

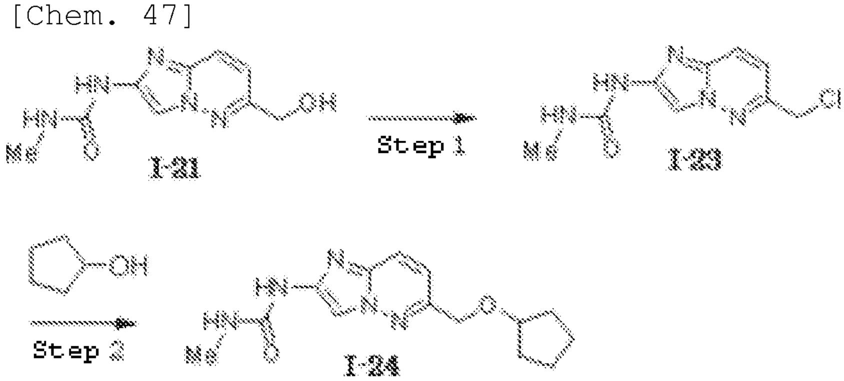


I-21 I-23 I-24

Step 1

To a suspension of the compound (I-21, 106. 3 mg, 0.481 mmol) obtained in Step 1 of Example 16 in methylene chloride (3.0 mL) was added thionyl chloride (88 μL, 1.20 mmol) under nitrogen atmosphere at 0 degree Celsius, subsequently stirring at room temperature for 1 hour. The reaction solution was concentrated *in vacuo,* and then the

residue was purified by silica gel column chromatography (chloroform:methanol = 100:0 → 95:5) to yield a compound (I-23, 103.2 mg, 90% yield).
LC-MS: 1.00 min, [M+H] = 240

Step 2

To a suspension of 60% sodium hydride (12.2 mg, 0.305 mmol) in N,N-dimethylformamide (0.3 mL) was added cyclopentanol (22 μL, 0.244 mmol) under nitrogen atmosphere at 0 degree Celsius, subsequently stirring at room temperature for 30 minutes. At 0 degree Celsius, to the reaction solution was added a solution of the compound (I-23, 29.2 mg, 0.122 mmol) obtained in Step 1 in N,N-dimethylformamide (1.0 mL), and then stirred at room temperature for 3 hours and 30 minutes. Water was then added to the reaction solution, followed by extraction with ethyl acetate. The organic layer was washed with water and saturated brine, and then dried with anhydrous magnesium sulfate. The solvent was concentrated *in vacuo,* and then the residue was purified by reversed phase HPLC to yield a compound (I-24, 5.5 mg, 16% yield).
LC-MS: 1.43 min, [M+H] = 290

(Example 18 Synthesis of Compounds I-25 and I-26)

[Chem. 48]

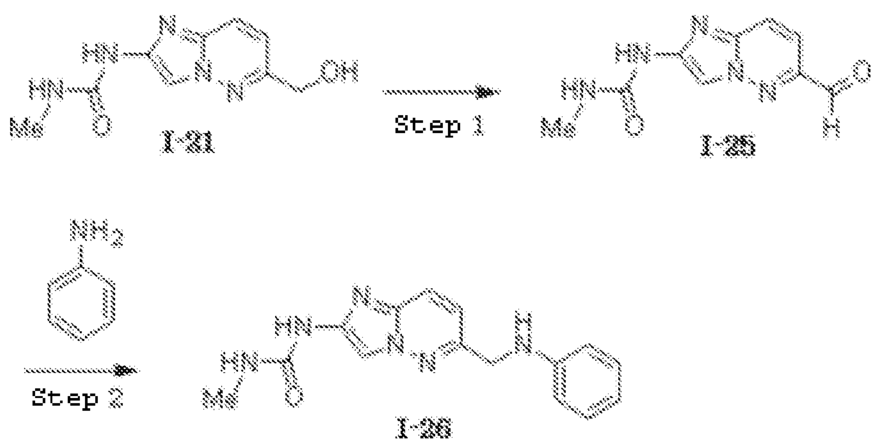


I-21 I-25 I-26

Step 1

To a solution of 2-iodoxybenzoic acid (65.8 mg, 0.235 mmol) in dimethylsulfoxide (2.0 mL) was added the compound (I-21, 40.0 mg, 0.181 mmol) obtained in Step 1 of Example 16, subsequently stirring at room temperature for 2 hours. Water was added to the reaction solution, followed by extraction with ethyl acetate. The organic layer was washed with water and saturated brine, and then dried with anhydrous magnesium sulfate. The solvent was concentrated *in vacuo* to yield a crude product (I-25, 68.3 mg).
LC-MS: 0.86 min, [M+H] = 220

Step 2

To a suspension of the crude product (I-25, 36.9 mg) obtained in Step 1 in methylene chloride (1.5 mL) was added aniline (20 μL, 0.215 mmol), sodium triacetoxyborohydride (60.7 mg, 0.286 mmol), and acetic acid (20 μL, 0.358 mmol) under nitrogen atmosphere, subsequently stirring at room temperature for 4 hours. Water was added to the reaction solution, followed by extraction with ethyl acetate. The organic layer was washed with water and saturated brine, and then dried with anhydrous magnesium sulfate. The solvent was concentrated *in vacuo,* and then the residue was purified by reversed phase HPLC to yield a compound (I-26, 12.3 mg, 23%).
LC-MS: 1.31 min, [M+H] = 297

(Example 19 Synthesis of Compound I-27)

[Chem. 49]

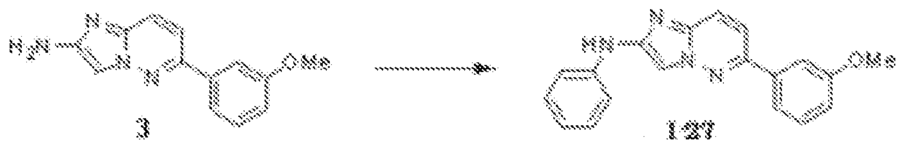

3 I-27

A suspension of the compound (3, 20 mg) obtained in Reference Example 3, xantphos (7.2 mg), palladium acetate (1.9 mg), cesium carbonate (81 mg), and benzene bromide (29 mg) in dioxane (2.5 mL)/DMA (0.25 mL) was heated under microwave irradiation at 150 degrees Celsius for 5 minutes. To the reaction solution was added aqueous ammonium chloride solution, and then extracted with chloroform, followed by concentration *in vacuo.* The residue was then purified by reversed phase HPLC to yield a compound (I-27, 16 mg) as a pale yellow solid.
LC-MS: 2.33 min, [M+H] = 317

(Example 20 Synthesis of Compounds I-28 and above)

Compounds I-28 and above were synthesized similarly to the Examples described above.
The physical properties (retention time and mass spectrum) of Compound Nos. I-1 to 213, 215 to 218, 220 to 224, and 232 to 294 are shown below. In Tables 1-1 to 1-50, the term "chiral" indicates a chiral compound.
[Table 1-1]

| Compound No. | Structural formula | Retention time (min) | Mass (M+H) |
|---|---|---|---|
| I-1 | 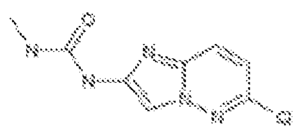 | 1.07 | 226 |
| I-2 | 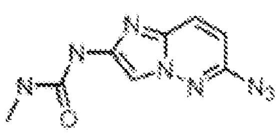 | 1.02 | 233 |
| I-3 | 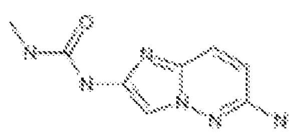 | 0.27 | 207 |
| I-4 | 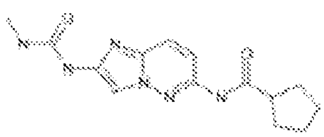 | 1.18 | 303 |
| I-5 | 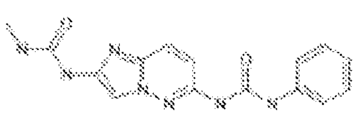 | 1.20 | 326 |

(continued)

| Compound No. | Structural formula | Retention time (min) | Mass (M+H) |
|---|---|---|---|
| I-6 | 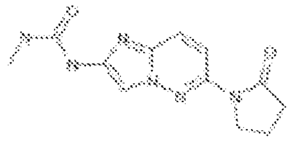 | 0.86 | 275 |
| I-7 | 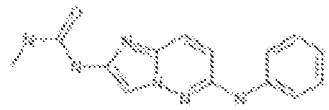 | 1.11 | 283 |

[Table 1-2]

| Compound No. | Structural formula | Retention time (min) | Mass (M+H) |
|---|---|---|---|
| I-8 | 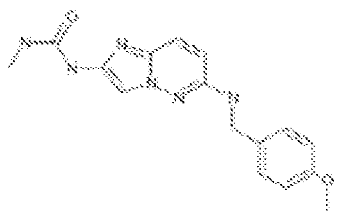 | 1.05 | 327 |
| I-9 | 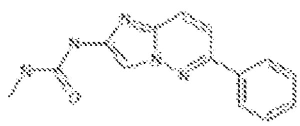 | 1.73 | 268 |
| I-10 | 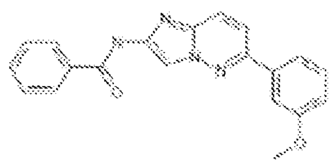 | 2.09 | 345 |
| I-12 | 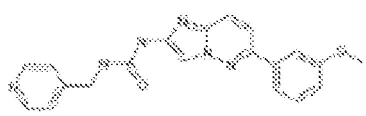 | 1.95 | 380 |
| I-13 | 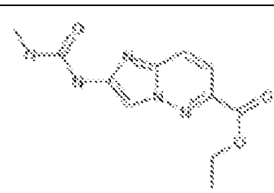 | 1.05 | 264 |
| I-14 | 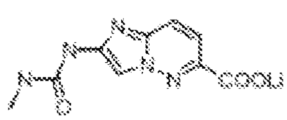 | 0.64 | 236 |

[Table 1-3]

| Compound No. | Structural formula | Retention time (min) | Mass (M+H) |
|---|---|---|---|
| I-15 | 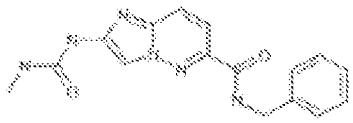 | 1.65 | 325 |
| I-16 | 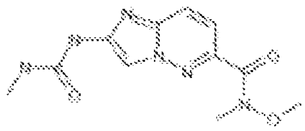 | 0.74 | 279 |
| I-17 | 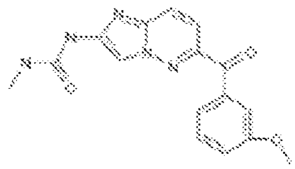 | 1.47 | 326 |
| I-18 | 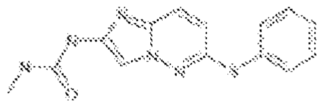 | 1.62 | 300 |
| I-19 | 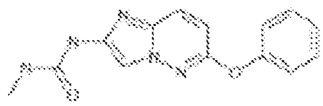 | 1.41 | 284 |
| I-20 | 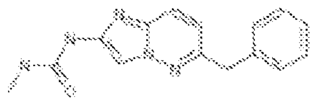 | 1.40 | 282 |

[Table 1-4]

| Compound No. | Structural formula | Retention time (min) | Mass (M-H) |
|---|---|---|---|
| I-21 | 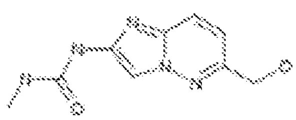 | 0.46 | 222 |
| I-22 | 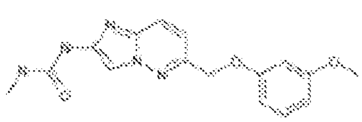 | 1.45 | 328 |
| I-23 | 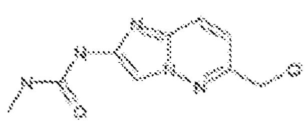 | 1.00 | 240 |

(continued)

| Compound No. | Structural formula | Retention time (min) | Mass (M-H) |
|---|---|---|---|
| I-24 | 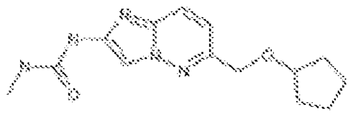 | 1.43 | 290 |
| I-25 | 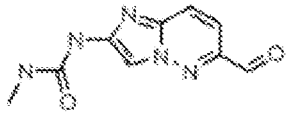 | 0.86 | 220 |
| I-26 | 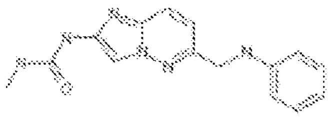 | 1.31 | 297 |
| I-27 | 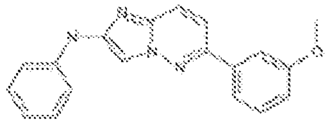 | 2.33 | 317 |

[Table 1-5]

| Compound No. | Structural formula | Retention time (min) | Mass (M+H) |
|---|---|---|---|
| I-28 | 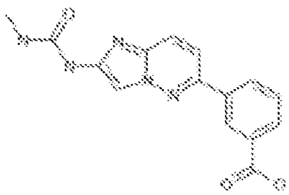 | 1.58 | 312 |
| I-29 | 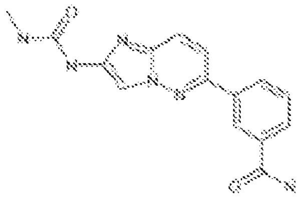 | 0.99 | 311 |
| I-30 | 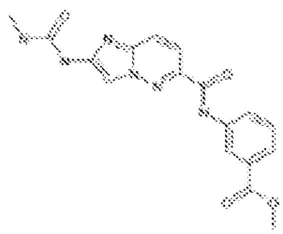 | 1.47 | 369 |

(continued)

| Compound No. | Structural formula | Retention time (min) | Mass (M+H) |
|---|---|---|---|
| I-31 | 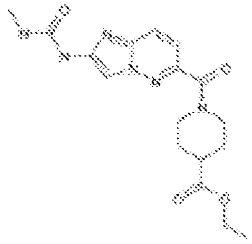 | 1.17 | 375 |

[Table 1-6]

| Compound No. | Structural formula | Retention time (min) | Mass (M+H) |
|---|---|---|---|
| I-32 | 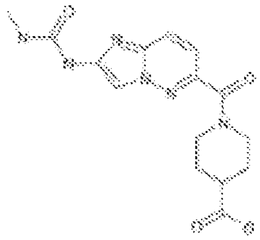 | 0.74 | 347 |
| I-33 | 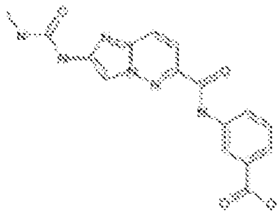 | 1.14 | 355 |
| I-34 | 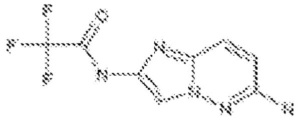 | 0.99 | 246 |
| I-35 | 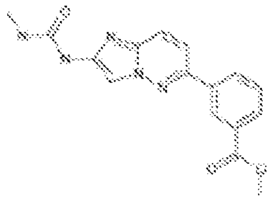 | 1.46 | 326 |
| I-36 | 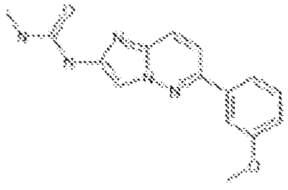 | 1.74 | 296 |

[Table 1-7]

| Compound No. | Structural formula | Retention time (min) | Mass (M+H) |
|---|---|---|---|
| I-37 | 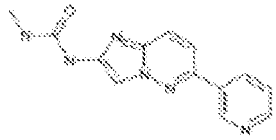 | 0.81 | 269 |
| I-38 | 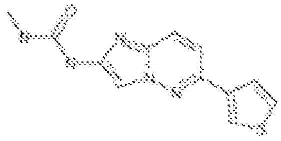 | 1.69 | 274 |
| I-39 | 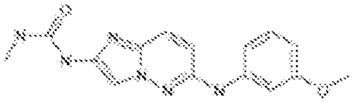 | 1.14 | 313 |
| I-40 | 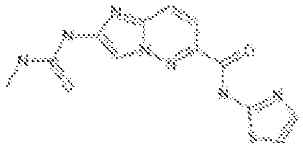 | 1.60 | 318 |
| I-41 | 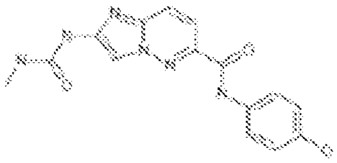 | 1.29 | 327 |
| I-42 | 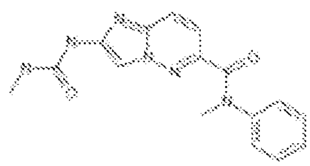 | 1.61 | 325 |

[Table 1-8]

| Compound No. | Structural formula | Retention time (min) | Mass (M+H) |
|---|---|---|---|
| I-43 | 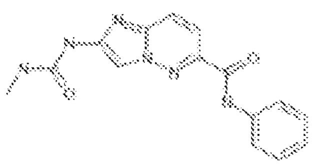 | 1.69 | 311 |
| I-44 | 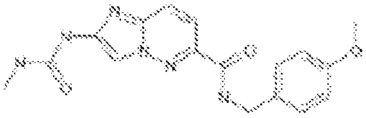 | 1.65 | 355 |

(continued)

| Compound No. | Structural formula | Retention time (min) | Mass (M+H) |
|---|---|---|---|
| I-45 | 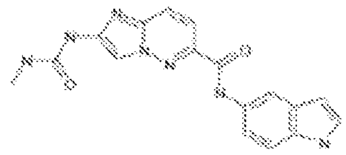 | 1.62 | 350 |
| I-46 | 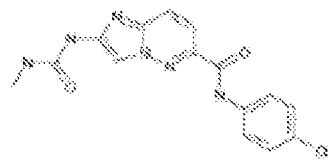 | 1.82 | 345 |
| I-47 | 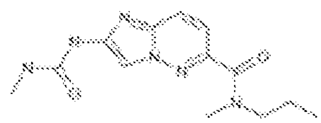 | 1.52 | 291 |
| I-48 | 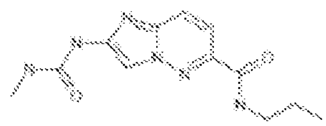 | 1.50 | 277 |

[Table 1-9]

| Compound No. | Structural formula | Retention time (min) | Mass (M+H) |
|---|---|---|---|
| I-49 | 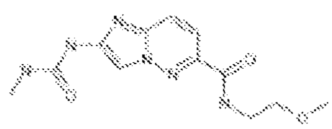 | 1.23 | 293 |
| I-50 | 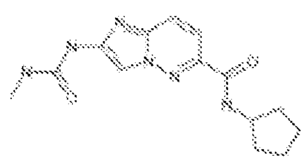 | 1.64 | 303 |
| I-51 | 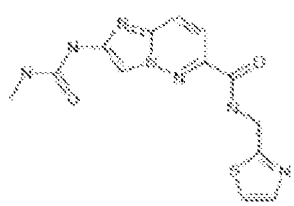 | 1.20 | 332 |
| I-52 | 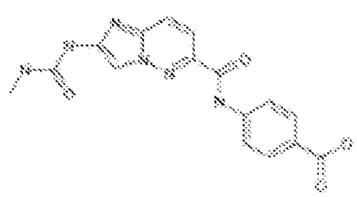 | 1.54 | 355 |

(continued)

| Compound No. | Structural formula | Retention time (min) | Mass (M+H) |
|---|---|---|---|
| I-53 | 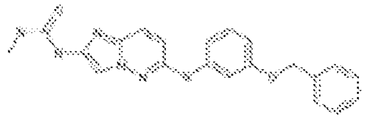 | 1.68 | 389 |
| I-54 | 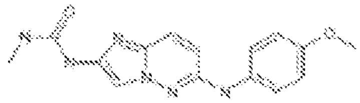 | 1.06 | 313 |

[Table 1-10]

| Compound No. | Structural formula | Retention time (min) | Mass (M+H) |
|---|---|---|---|
| I-55 | 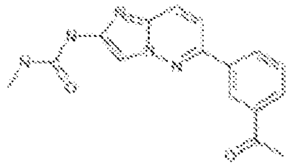 | 1.65 | 310 |
| I-56 | 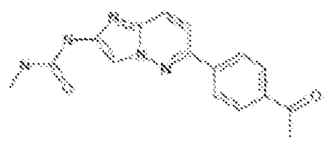 | 1.88 | 310 |
| I-57 | 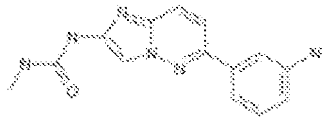 | 1.22 | 283 |
| I-58 | 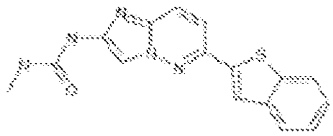 | 1.89 | 324 |
| I-59 | 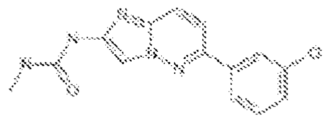 | 1.85 | 303 |
| I-60 | 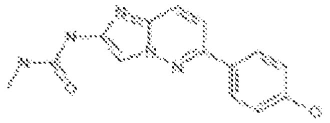 | 1.84 | 303 |

[Table 1-11]

| Compound No. | Structural formula | Retention time (min) | Mass (M+H) |
|---|---|---|---|
| I-61 | 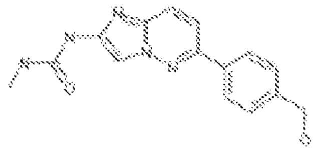 | 1.51 | 298 |
| I-62 | 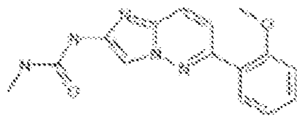 | 1.70 | 298 |
| I-63 | 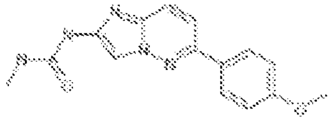 | 1.74 | 298 |
| I-64 | 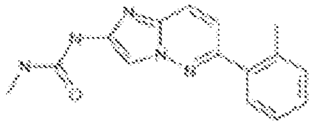 | 1.76 | 282 |
| I-65 | 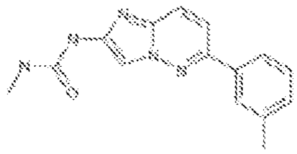 | 1.83 | 282 |
| I-66 | 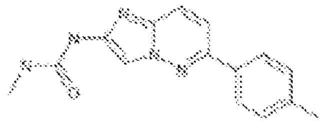 | 1.81 | 282 |

[Table 1-12]

| Compound No. | Structural formula | Retention time (min) | Mass (M+H) |
|---|---|---|---|
| I-67 | 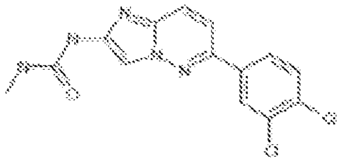 | 2.00 | 337 |
| I-68 | 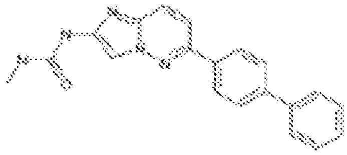 | 2.00 | 344 |

(continued)

| Compound No. | Structural formula | Retention time (min) | Mass (M+H) |
|---|---|---|---|
| I-69 | 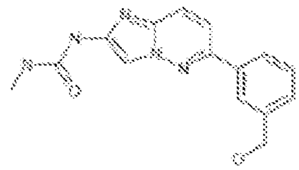 | 1.53 | 298 |
| I-70 | 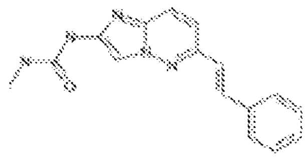 | 1.81 | 294 |
| I-71 | 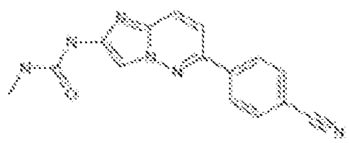 | 1.69 | 293 |

[Table 1-13]

| Compound No. | Structural formula | Retention time (min) | Mass (M+H) |
|---|---|---|---|
| I-72 | 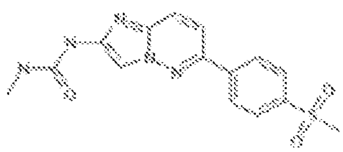 | 1.51 | 346 |
| I-73 | 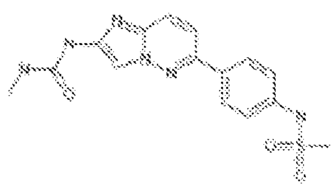 | 1.50 | 361 |
| I-74 | 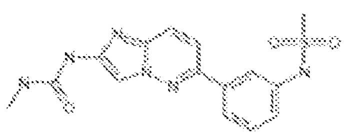 | 1.57 | 361 |
| I-75 | 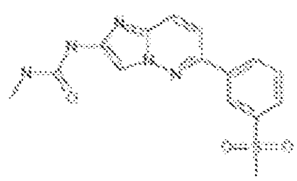 | 1.56 | 346 |
| I-76 | 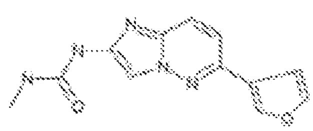 | 1.61 | 258 |

(continued)

| Compound No. | Structural formula | Retention time (min) | Mass (M+H) |
|---|---|---|---|
| I-77 | 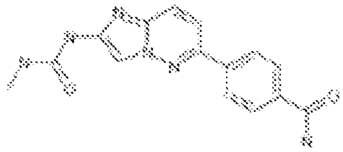 | 1.29 | 311 |

[Table 1-14]

| Compound No. | Structural formula | Retention time (min) | Mass (M+H) |
|---|---|---|---|
| I-78 | 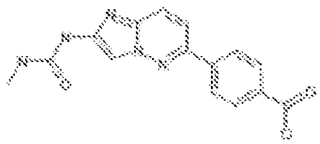 | 1.55 | 312 |
| I-79 | 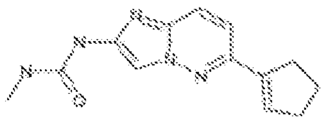 | 1.76 | 258 |
| I-80 | 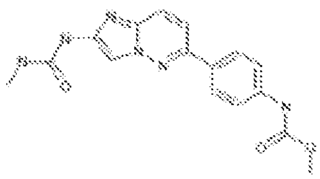 | 1.64 | 341 |
| I-81 | 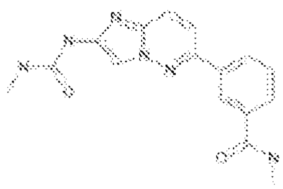 | 1.46 | 325 |
| I-82 | 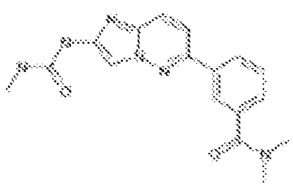 | 1.15 | 339 |

[Table 1-15]

| Compound No. | Structural formula | Retention time (min) | Mass (M+H) |
|---|---|---|---|
| I-83 | 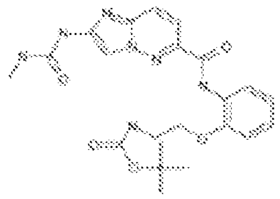 | 1.79 | 470 |
| I-84 | 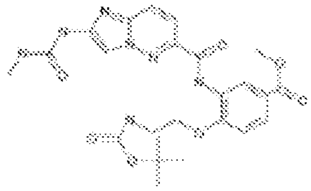 | 1.77 | 528 |
| I-85 | 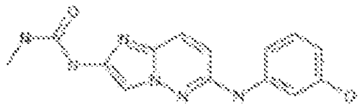 | 0.80 | 299 |
| I-86 | 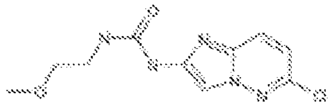 | 1.13 | 270 |
| I-87 | 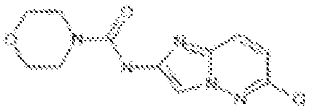 | 1.12 | 282 |
| I-88 | 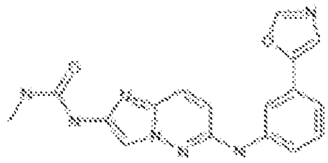 | 1.09 | 350 |

[Table 1-16]

| Compound No. | Structural formula | Retention time (min) | Mass (M+H) |
|---|---|---|---|
| I-89 | 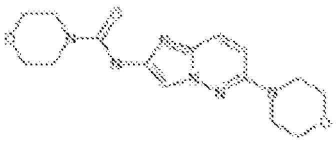 | 0.76 | 333 |
| I-90 | 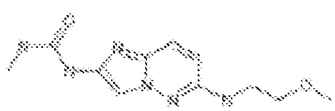 | 0.54 | 265 |

(continued)

| Compound No. | Structural formula | Retention time (min) | Mass (M+H) |
|---|---|---|---|
| I-91 | 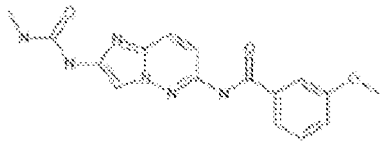 | 1.21 | 341 |
| I-98 | 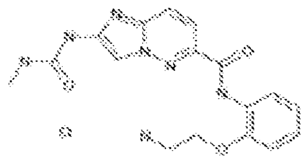 | 0.71 | 370 |
| I-99 | 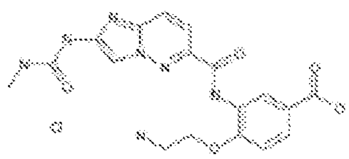 | 0.63 | 414 |
| I-100 | 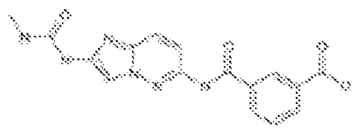 | 0.96 | 355 |

[Table 1-17]

| Compound No. | Structural formula | Retention time (min) | Mass (M+H) |
|---|---|---|---|
| I-101 | 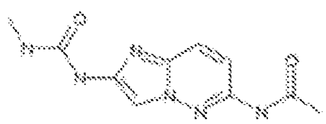 | 0.59 | 249 |
| I-102 | 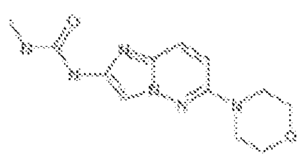 | 0.67 | 277 |
| I-103 | 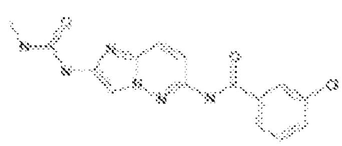 | 1.40 | 345 |
| I-104 | 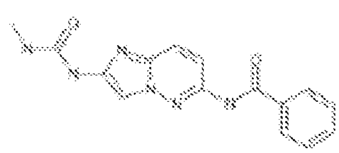 | 1.15 | 311 |

(continued)

| Compound No. | Structural formula | Retention time (min) | Mass (M+H) |
|---|---|---|---|
| I-105 | 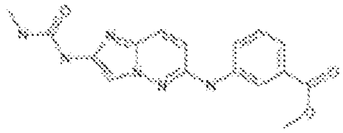 | 1.19 | 341 |
| I-106 | 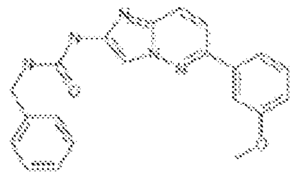 | 1.98 | 374 |

[Table 1-18]

| Compound No. | Structural formula | Retention time (min) | Mass (M+H) |
|---|---|---|---|
| I-107 | 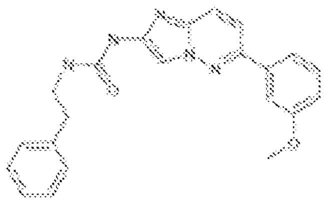 | 2.07 | 388 |
| I-108 | 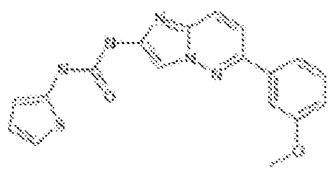 | 2.00 | 368 |
| I-109 | 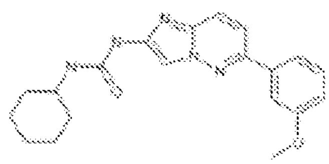 | 2.11 | 366 |
| I-110 | 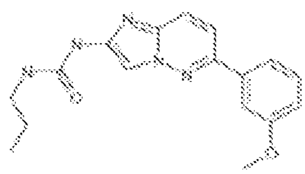 | 1.77 | 326 |
| I-111 | 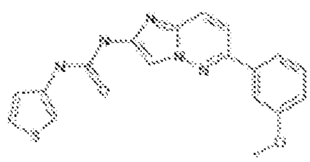 | 2.03 | 366 |

[Table 1-19]

| Compound No. | Structural formula | Retention time (min) | Mass (M+H) |
|---|---|---|---|
| I-112 | 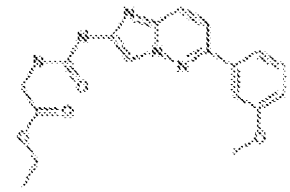 | 1.69 | 370 |
| I-113 | 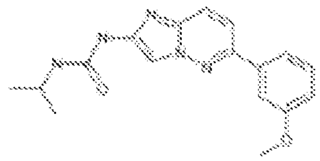 | 1.77 | 326 |
| I-114 | 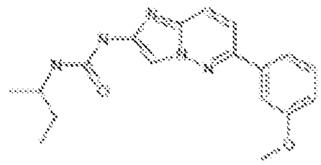 | 1.91 | 340 |
| I-115 | 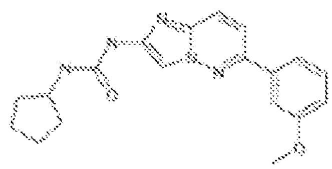 | 1.97 | 352 |
| I-116 | 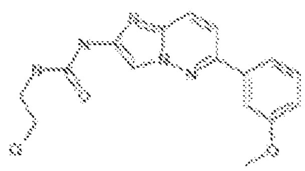 | 1.74 | 346 |

[Table 1-20]

| Compound No. | Structural formula | Retention time (min) | Mass (M+H) |
|---|---|---|---|
| I-117 | 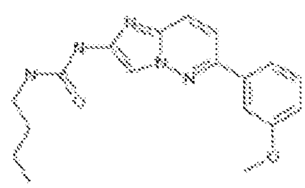 | 1.95 | 340 |
| I-118 | 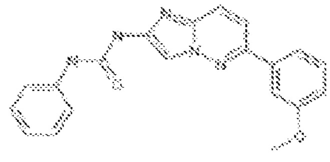 | 2.07 | 360 |

(continued)

| Compound No. | Structural formula | Retention time (min) | Mass (M+H) |
|---|---|---|---|
| I-119 | 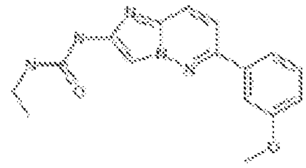 | 1.62 | 312 |
| I-120 | 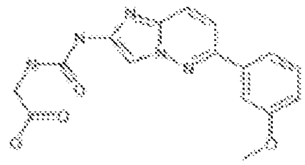 | 1.36 | 342 |
| I-121 | 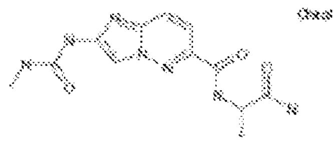 | 0.71 | 306 |

[Table 1-21]

| Compound No. | Structural formula | Retention time (min) | Mass (M+H) |
|---|---|---|---|
| I-122 | 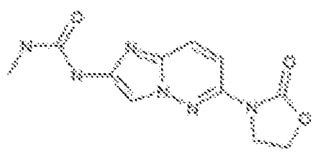 | 0.79 | 277 |
| I-123 | 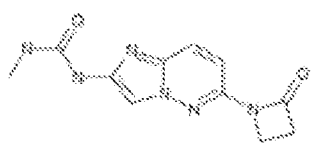 | 0.79 | 261 |
| I-124 | 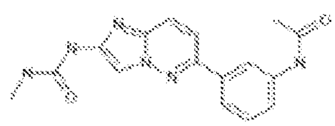 | 1.10 | 325 |
| I-125 | 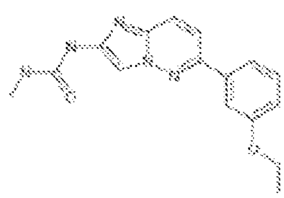 | 1.65 | 312 |
| I-126 | 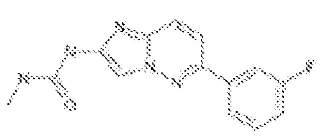 | 1.54 | 286 |

(continued)

| Compound No. | Structural formula | Retention time (min) | Mass (M+H) |
|---|---|---|---|
| I-127 | 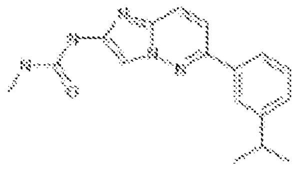 | 1.94 | 310 |

[Table 1-22]

| Compound No. | Structural formula | Retention time (min) | Mass (M+H) |
|---|---|---|---|
| I-128 | 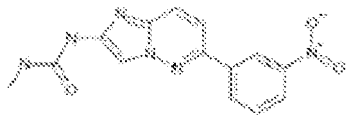 | 1.50 | 313 |
| I-129 | 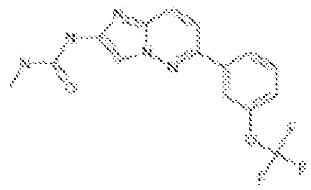 | 1.88 | 352 |
| I-130 | 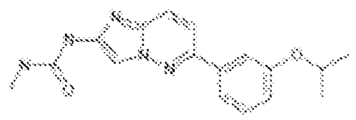 | 1.79 | 326 |
| I-131 | 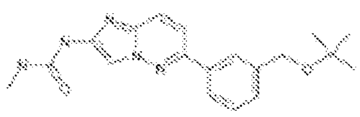 | 1.91 | 354 |
| I-132 | 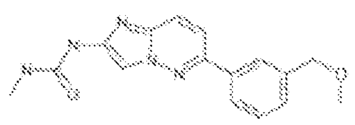 | 1.44 | 312 |
| I-133 | 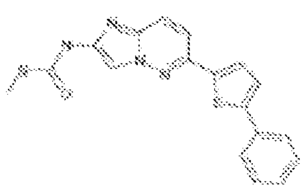 | 1.97 | 350 |

[Table 1-23]

| Compound No. | Structural formula | Retention time (min) | Mass (M+H) |
|---|---|---|---|
| I-134 | 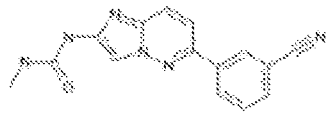 | 1.40 | 293 |
| I-135 | 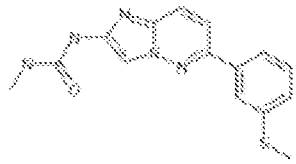 | 1.68 | 314 |
| I-136 | 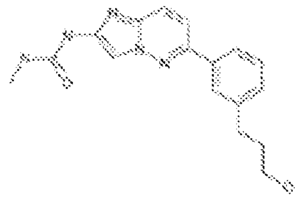 | 1.27 | 326 |
| I-137 | 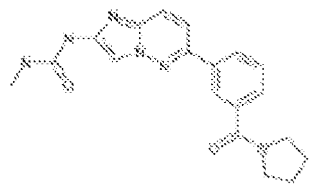 | 1.27 | 365 |
| I-138 | 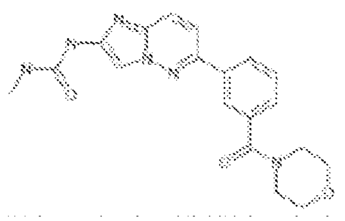 | 1.12 | 381 |

[Table 1-24]

| Compound No. | Structural formula | Retention time (min) | Mass (M+H) |
|---|---|---|---|
| I-139 | 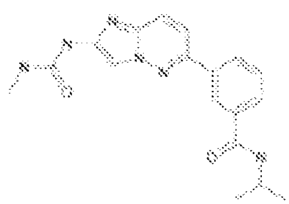 | 1.33 | 353 |
| I-140 | 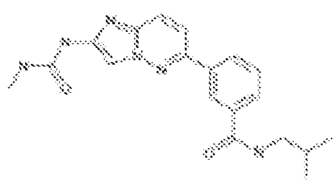 | 1.47 | 367 |

(continued)

| Compound No. | Structural formula | Retention time (min) | Mass (M+H) |
|---|---|---|---|
| I-141 | 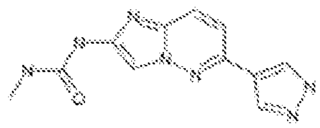 | 0.81 | 258 |
| I-142 | 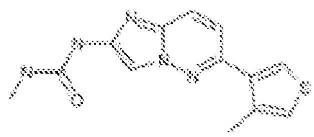 | 1.52 | 288 |
| I-143 | 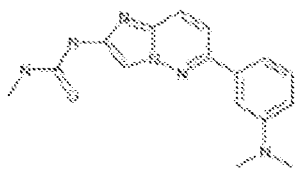 | 1.26 | 311 |

[Table 1-25]

| Compound No. | Structural formula | Retention time (min) | Mass (M+H) |
|---|---|---|---|
| I-44 | 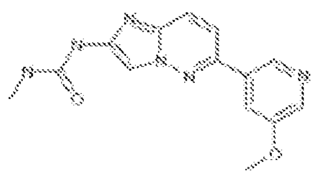 | 0.99 | 299 |
| I-145 | 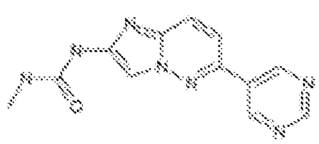 | 0.89 | 270 |
| I-146 | 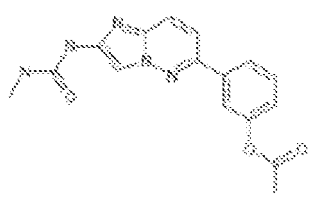 | 1.43 | 326 |
| I-147 | 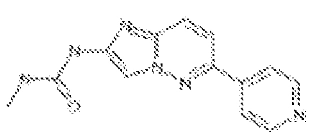 | 0.71 | 269 |
| I-148 | 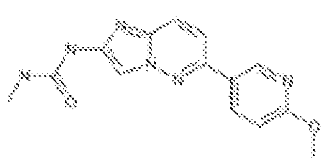 | 1.30 | 299 |

[Table 1-26]

| Compound No. | Structural formula | Retention time (min) | Mass (M+H) |
|---|---|---|---|
| I-149 | 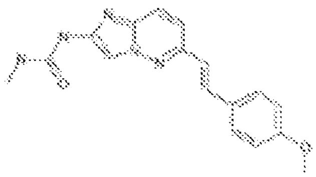 | 1.63 | 324 |
| I-150 | 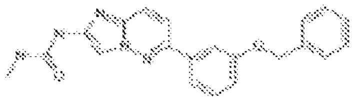 | 1.99 | 374 |
| I-151 | 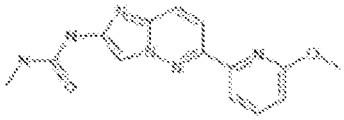 | 1.55 | 299 |
| I-152 | 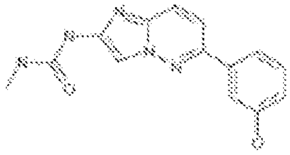 | 1.14 | 284 |
| I-153 | 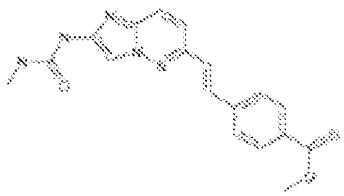 | 1.64 | 352 |

[Table 1-27]

| Compound No. | Structural formula | Retention time (min) | Mass (M+H) |
|---|---|---|---|
| I-154 | 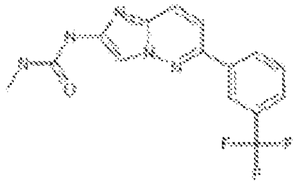 | 1.81 | 336 |
| I-155 | 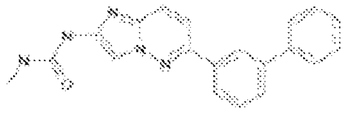 | 1.99 | 344 |
| I-156 | 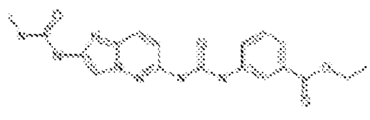 | 1.41 | 398 |

(continued)

| Compound No. | Structural formula | Retention time (min) | Mass (M+H) |
|---|---|---|---|
| I-157 | 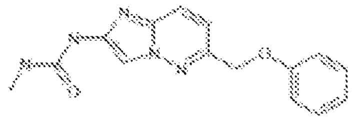 | 1.46 | 298 |
| I-158 | 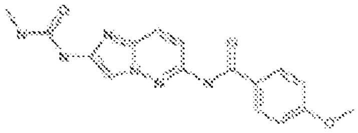 | 1.18 | 341 |
| I-159 | 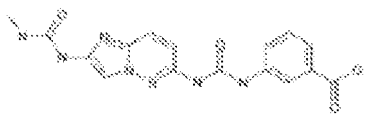 | 1.00 | 370 |

[Table 1-28]

| Compound No. | Structural formula | Detention time (min) | Mass (M+H) |
|---|---|---|---|
| I-161 | 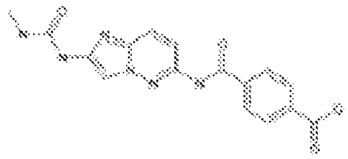 | 0.93 | 355 |
| I-161 | 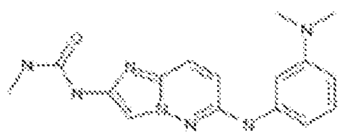 | 0.67 | 326 |
| I-162 | 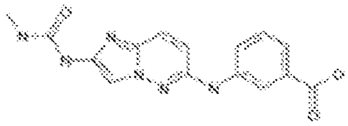 | 0.87 | 327 |
| I-163 | 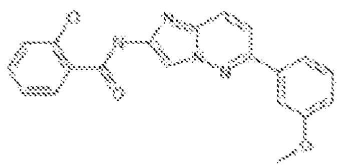 | 212 | 380 |
| I-164 | 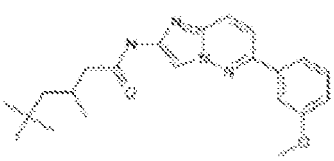 | 258 | 381 |

(continued)

| Compound No. | Structural formula | Detention time (min) | Mass (M+H) |
|---|---|---|---|
| I-165 | 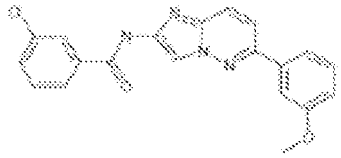 | 2.35 | 380 |

[Table 1-29]

| Compound No. | Structural formula | Retention time (min) | Mass (M+H) |
|---|---|---|---|
| I-166 | 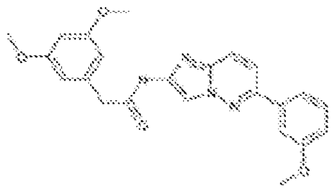 | 2.06 | 419 |
| I-167 | 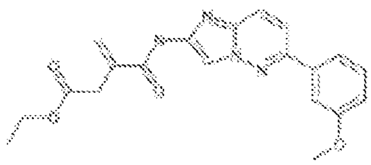 | 1.84 | 381 |
| I-168 | 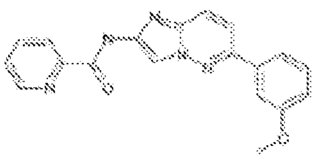 | 2.08 | 346 |
| I-169 | 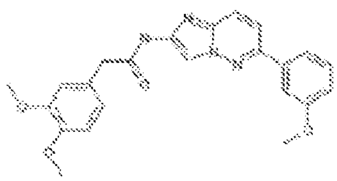 | 1.89 | 419 |
| I-170 | 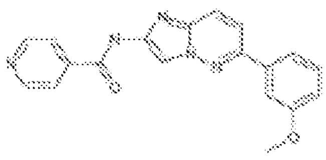 | 1.60 | 346 |

[Table 1-30]

| Compound No. | Structural formula | Retention time (min) | Mass (M+H) |
|---|---|---|---|
| I-171 | 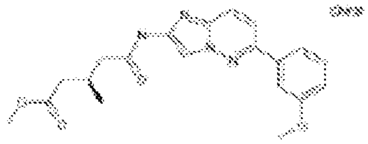 | 1.89 | 383 |
| I-172 | 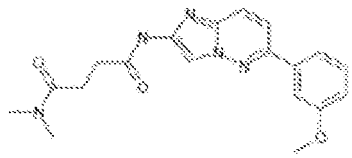 | 1.53 | 368 |
| I-173 | 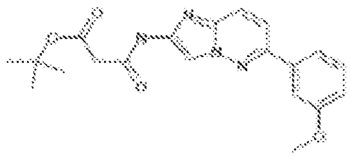 | 2.07 | 383 |
| I-174 | 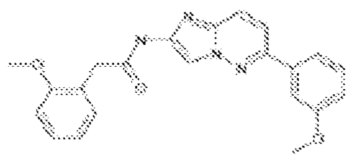 | 2.08 | 389 |
| I-175 | 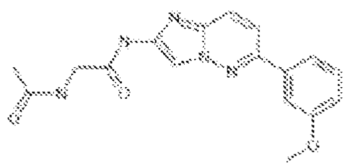 | 1.35 | 340 |

[Table 1-31]

| Compound No. | Structural formula | Retention time (min) ; | Mass (M+H) |
|---|---|---|---|
| I-176 | 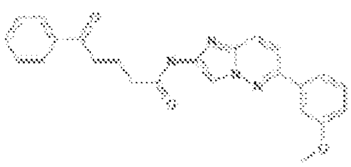 | 2.09 | 415 |
| I-177 | 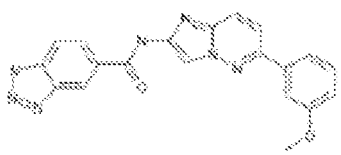 | 1.69 | 386 |
| I-178 | 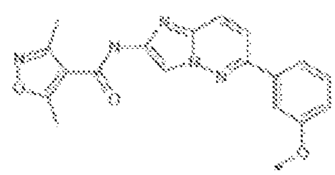 | 1.93 | 364 |

(continued)

| Compound No. | Structural formula | Retention time (min) ; | Mass (M+H) |
|---|---|---|---|
| I-179 | 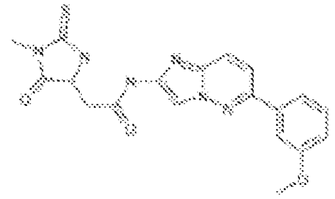 | 1.68 | 411 |
| I-180 | 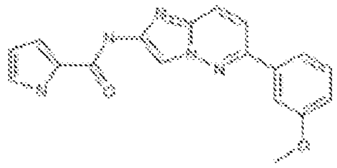 | 1.81 | 334 |

[Table 1-32]

| Compound No. | Structural formula | Retention time (main) | Mass (M+H) |
|---|---|---|---|
| I-181 | 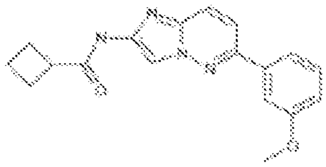 | 1.94 | 323 |
| I-182 | 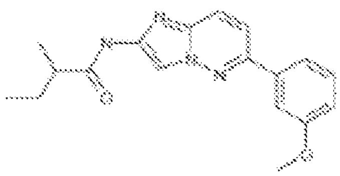 | 2.01 | 325 |
| I-183 | 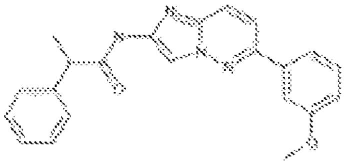 | 2.21 | 373 |
| I-184 | 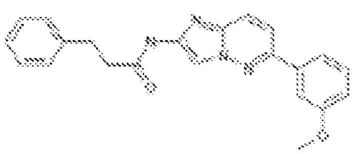 | 2.17 | 373 |
| I-185 | 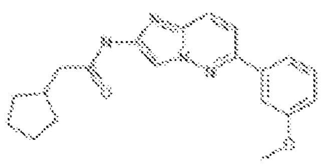 | 2.23 | 351 |

[Table 1-33]

| Compound No. | Structural formula | Retention time (min) | Mass (M+H) |
|---|---|---|---|
| I-186 | 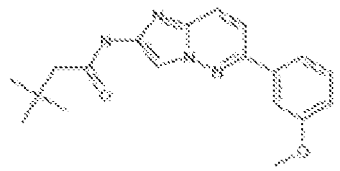 | 2.18 | 339 |
| I-187 | 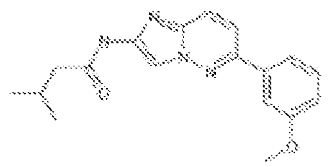 | 2.02 | 325 |
| I-188 | 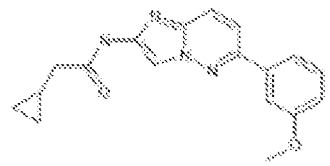 | 1.88 | 323 |
| I-189 | 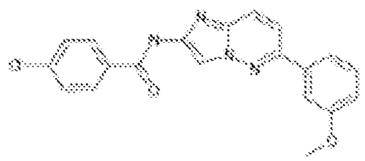 | 2.32 | 380 |
| I-190 | 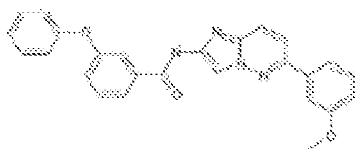 | 2.56 | 437 |

[Table 1-34]

| Compound No. | Structural formula | Retention time (min) | Mass (M+H) |
|---|---|---|---|
| I-191 | 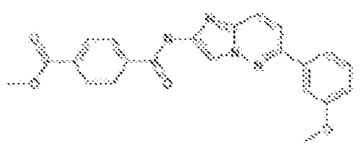 | 2.16 | 403 |
| I-192 | 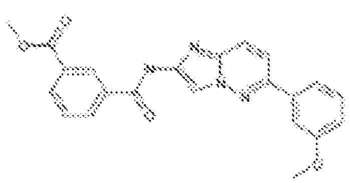 | 2.15 | 403 |

(continued)

| Compound No. | Structural formula | Retention time (min) | Mass (M+H) |
|---|---|---|---|
| I-193 | 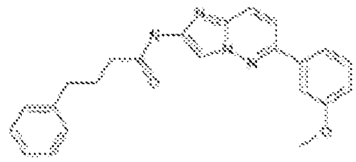 | 2.29 | 387 |
| I-194 | 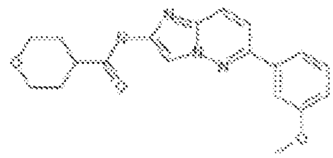 | 1.65 | 353 |
| I-195 | 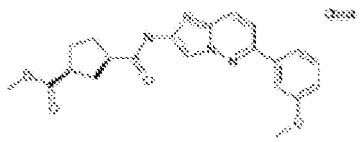 | 1.91 | 395 |
| I-196 | 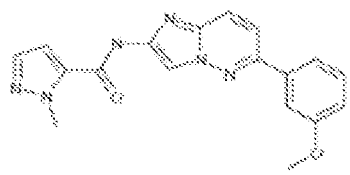 | 1.84 | 349 |

[Table 1-35]

| Compound No. | Structural formula | Retention time (min) | Mass (M+H) |
|---|---|---|---|
| I-197 | 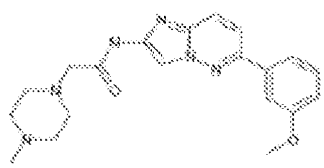 | 0.99 | 381 |
| I-198 | 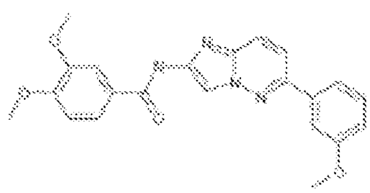 | 2.01 | 405 |
| I-199 | 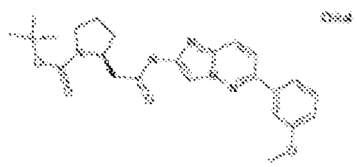 | 2.22 | 453 |

(continued)

| Compound No. | Structural formula | Retention time (min) | Mass (M+H) |
|---|---|---|---|
| I-200 | 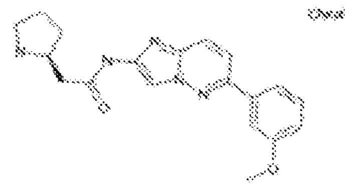 | 0.98 | 352 |
| I-201 | 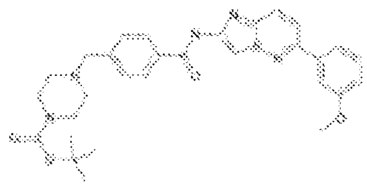 | 1.39 | 544 |

[Table 1-36]

| Compound No. | Structural formula | Retention time (min) | Mass (M+H) |
|---|---|---|---|
| I-202 | 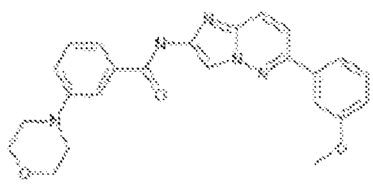 | 2.07 | 430 |
| I-203 | 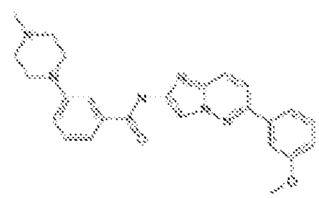 | 1.18 | 444 |
| I-204 | 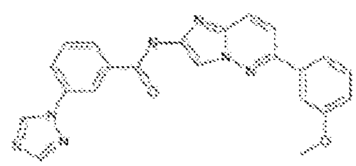 | 1.86 | 412 |
| I-205 | 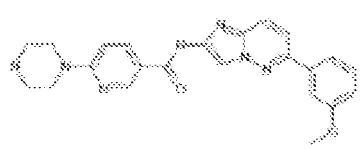 | 1.11 | 430 |
| I-206 | 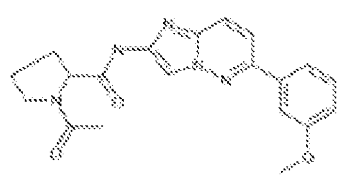 | 1.54 | 380 |

[Table 1-37]

| Compound No. | Structural formula | Retention time (min) | Mass (M+H) |
|---|---|---|---|
| I-207 | 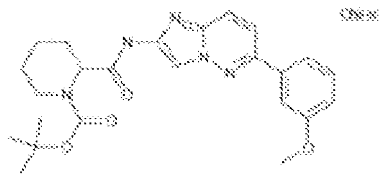 | 2.42 | 453 |
| I-208 | 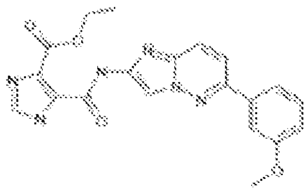 | 1.78 | 407 |
| I-209 | 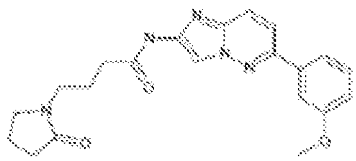 | 1.56 | 394 |
| I-210 | 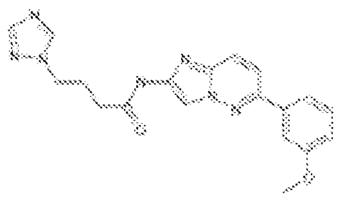 | 1.46 | 378 |
| I-211 | 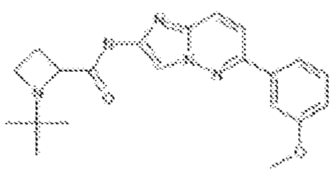 | 108 | 380 |

[Table 1-38]

| Compounds No. | Structural formula | Retention time (min) | Mass (M+H) |
|---|---|---|---|
| I-212 | 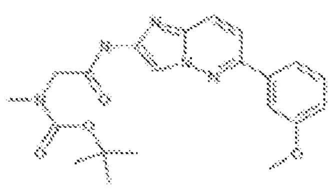 | 2.03 | 412 |
| I-213 | 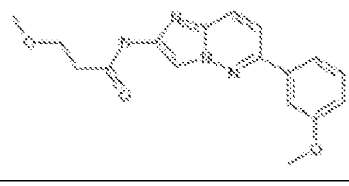 | 1.63 | 327 |

(continued)

| Compounds No. | Structural formula | Retention time (min) | Mass (M+H) |
|---|---|---|---|
| I-215 | 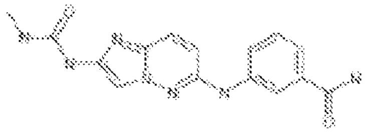 | 0.68 | 326 |
| I-216 | 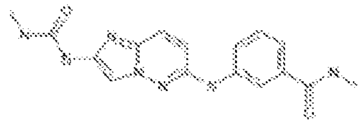 | 0.75 | 340 |
| I-217 | 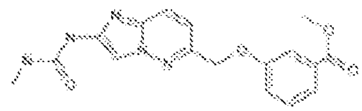 | 1.48 | 356 |

[Table 1-39]

| Compound No. | Structural formula | Retention time (min) | Mass (M+H) |
|---|---|---|---|
| I-218 | 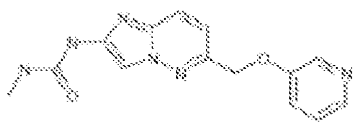 | 0.57 | 299 |
| I-220 | 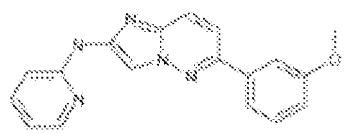 | 1.39 | 318 |
| I-221 | 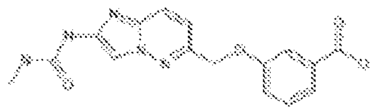 | 1.16 | 342 |
| I-222 | 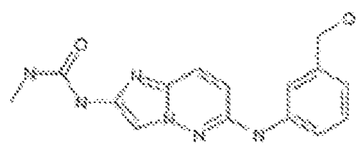 | 0.77 | 313 |
| I-223 | 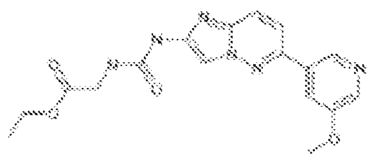 | 1.23 | 371 |
| I-224 | 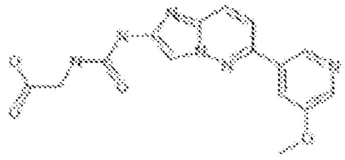 | 0.95 | 343 |

[Table 1-40]

| Compound No. | Structural formula | Retention time (min) | Mass (M+H) |
|---|---|---|---|
| I-232 | 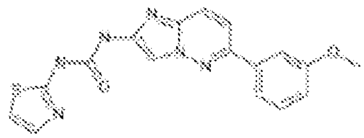 | 1.84 | 367.24 |
| I-233 | 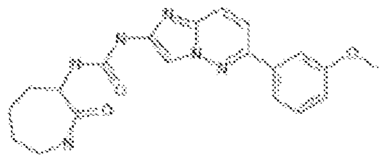 | 1.55 | 395.4 |
| I-234 | 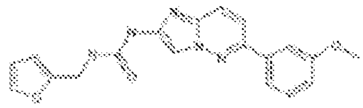 | 1.84 | 364.3 |
| I-235 | 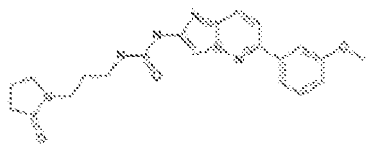 | 1.50 | 409.44 |
| I-236 | 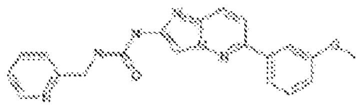 | 1.13 | 375.35 |
| I-237 | 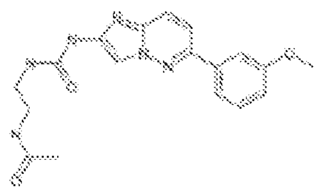 | 1.34 | 369.34 |

[Table 1-41]

| Compound No. | Structural formula | Retention time (min) | Mass (M+H) |
|---|---|---|---|
| I-239 | 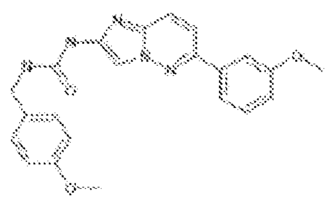 | 1.96 | 404.37 |
| I-239 | 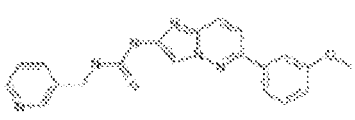 | 0.95 | 375.35 |

(continued)

| Compound No. | Structural formula | Retention time (min) | Mass (M+H) |
|---|---|---|---|
| I-240 | 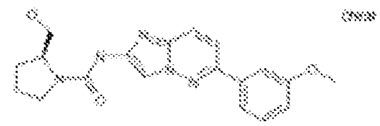 | 1.53 | 368.41 |
| I-241 | 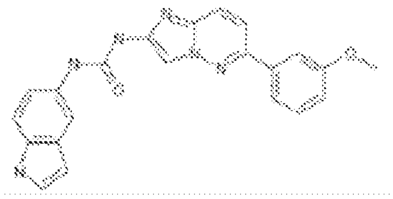 | 1.89 | 399.41 |
| I-242 | 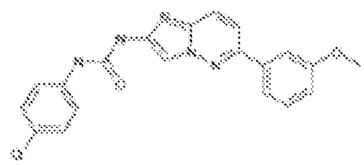 | 2.32 | 394.31 |
| I-243 | 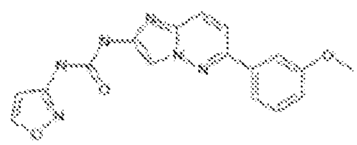 | 1.77 | 351.5 |

[Table 1-42]

| Compound No. | Structural formula | Retention time (min) | Mass (M+H) |
|---|---|---|---|
| I-244 | 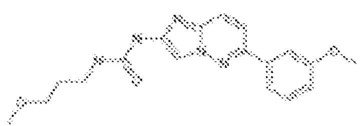 | 1.61 | 356.41 |
| I-245 | 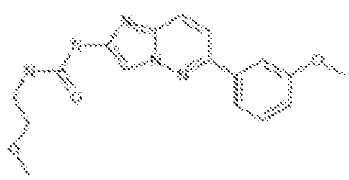 | 1.59 | 342.38 |
| I-246 | 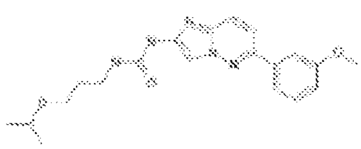 | 1.89 | 428.43 |
| I-247 | 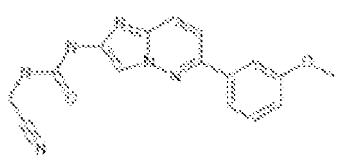 | 1.55 | 328.38 |

(continued)

| Compound No. | Structural formula | Retention time (min) | Mass (M+H) |
|---|---|---|---|
| I-248 | 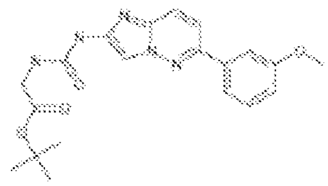 | 1.98 | 398.43 |
| I-249 | 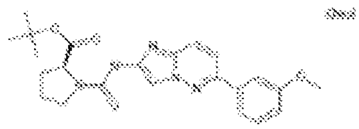 | 2.15 | 438.55 |

[Table 1-43]

| Compound No. | Structural formula | Retention time (min) | Mass (M+H) |
|---|---|---|---|
| I-250 | 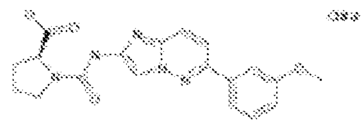 | 1.46 | 382.44 |
| I-251 | 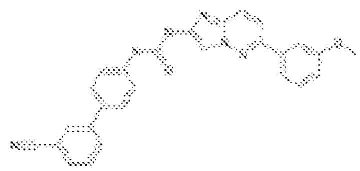 | 2.35 | 461.46 |
| I-252 | 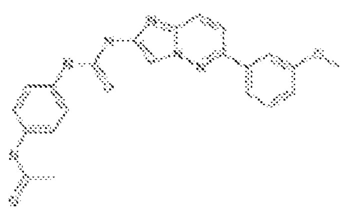 | 1.64 | 417.43 |
| I-253 | 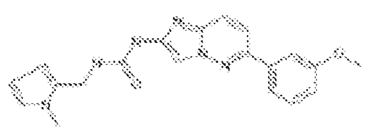 | 1.88 | 374.44 |
| I-254 | 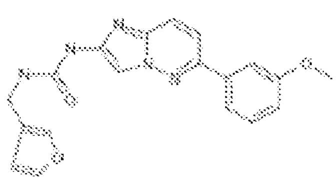 | 1.81 | 364.43 |
| I-255 | 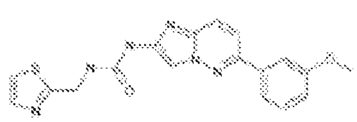 | 1.60 | 381.36 |

[Table 1-44]

| Compound No. | Structural formula | Retention time (min) | Mass (M+H) |
|---|---|---|---|
| I-256 | 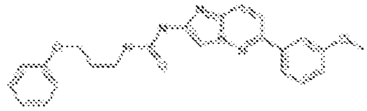 | 2.13 | 415.48 |
| I-257 | 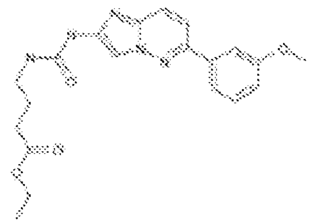 | 1.80 | 398.45 |
| I-258 | 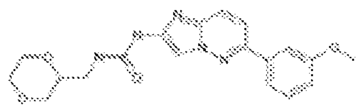 | 1.52 | 384.44 |
| I-259 | 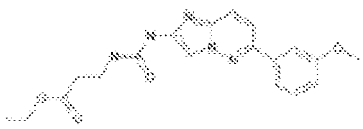 | 1.75 | 384.5 |
| I-260 | 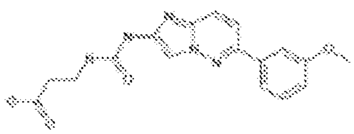 | 1.40 | 356.46 |
| I-261 | 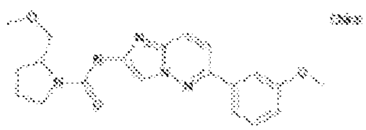 | 1.85 | 382.41 |

[Table 1-45]

| Compound No. | Structural formula | Retention time (min) | Mass (M+H) |
|---|---|---|---|
| I-262 | 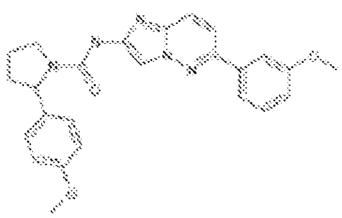 | 3.13 | 444.55 |

(continued)

| Compound No. | Structural formula | Retention time (min) | Mass (M+H) |
|---|---|---|---|
| I-263 | 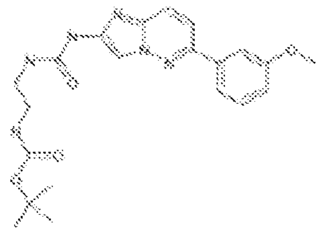 | 1.82 | 427.55 |
| I-264 | 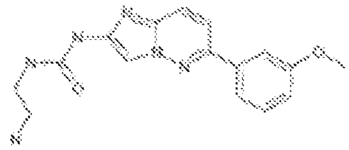 | 0.94 | 327.42 |
| I-266 | 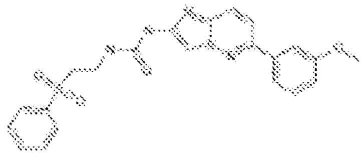 | 1.77 | 452.51 |
| I-266 | 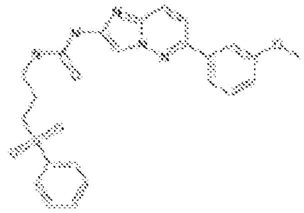 | 1.80 | 466.49 |

[Table 1-46]

| Compound No. | Structural formula | Retention time (min) | Mass (M+H) |
|---|---|---|---|
| I-267 | 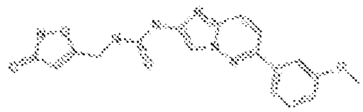 | 1.46 | 381.44 |
| I-268 | 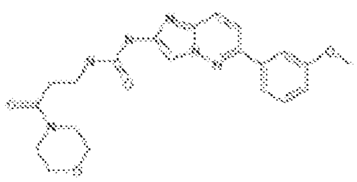 | 1.43 | 425.53 |
| I-269 | 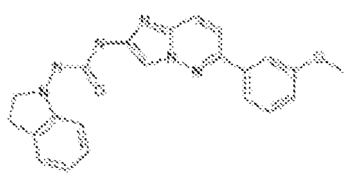 | 2.04 | 401.44 |

(continued)

| Compound No. | Structural formula | Retention time (min) | Mass (M+H) |
|---|---|---|---|
| I-270 | 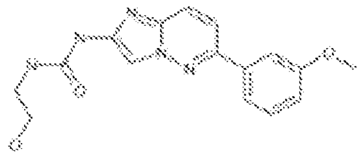 | 1.32 | 328.48 |
| I-271 | 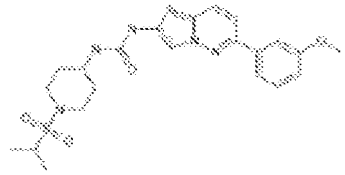 | 1.82 | 473.55 |

[Table 1-47]

| Compound No. | Structural formula | Retention time(min) | Mass (M+H) |
|---|---|---|---|
| I-272 | 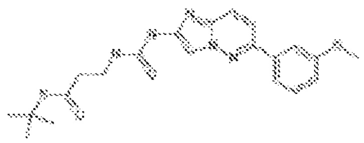 | 1.69 | 411.59 |
| I-273 | 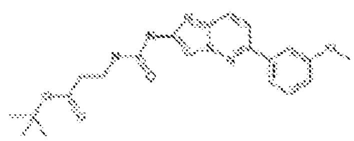 | 2.03 | 412.52 |
| I-274 | 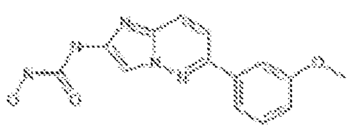 | 1.32 | 300.48 |
| I-275 | 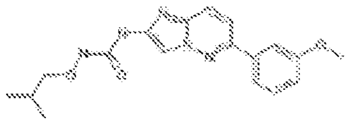 | 2.02 | 354,51 |
| I-276 | 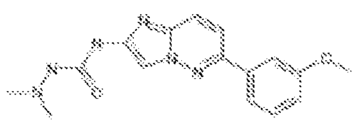 | 1.57 | 327.47 |
| I-277 | 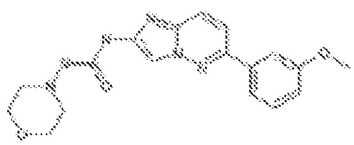 | 1.57 | 367.39 |

[Table 1-48]

| Compound No. | Structural formula | Retention time (min) | Mass (M+H) |
|---|---|---|---|
| I-278 | 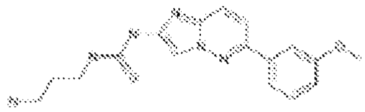 | 0.91 | 341.48 |
| I-279 | 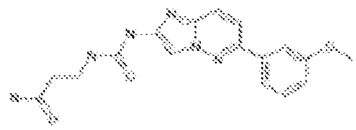 | 1.29 | 355.51. |
| I-280 | 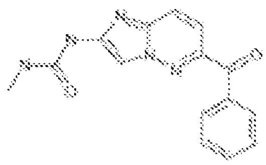 | 1.43 | 296 |
| I-281 | 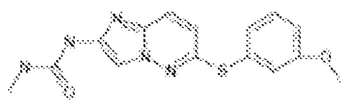 | 1.63 | 330 |
| I-282 | 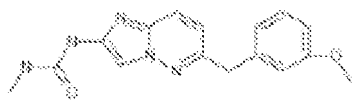 | 1.41 | 312 |
| I-283 | 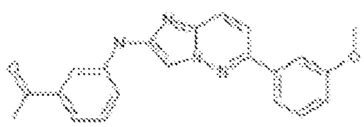 | 2.10 | 359.4 |

[Table 1-49]

| Compound No. | Structural formula | Detention time (min) | Mass (M+H) |
|---|---|---|---|
| I-284 | 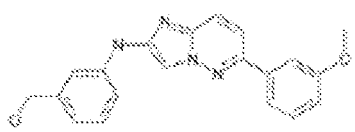 | 1.75 | 347.4 |
| I-2.85 | 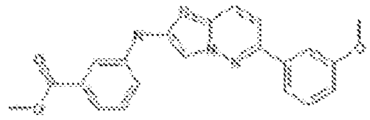 | 2.24 | 375.4 |
| I-286 | 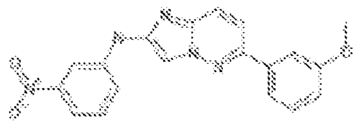 | 2.33 | 362.4 |

(continued)

| Compound No. | Structural formula | Detention time (min) | Mass (M+H) |
|---|---|---|---|
| I-287 | 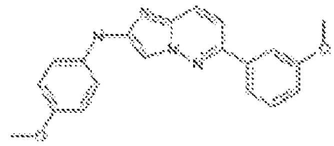 | 2.10 | 347.7 |
| I-288 | 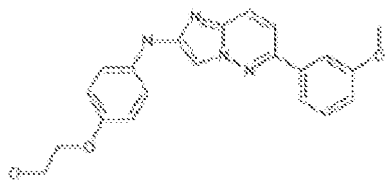 | 1.36 | 377.4 |
| I-289 | 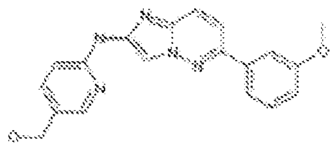 | 1.13 | 348.4 |

[Table 1-50]

| Compound No. | Structural formula | Retention time (min) | Mass (M+H) |
|---|---|---|---|
| I-290 | 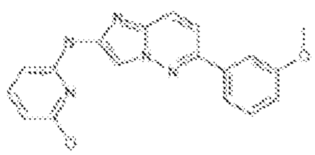 | 1.57 | 334.4 |
| I-291 | 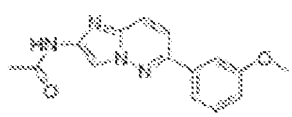 | 1.96 | 283 |
| I-292 | 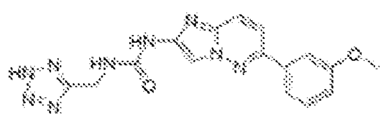 | 1,24 | 365 |
| I-293 | 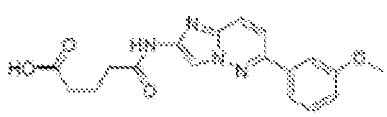 | 1.32 | 354 |
| I-294 | 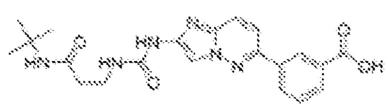 | 2.02 | 424 |

(Example 21: Measurement of PI3Kγ inhibitory activity)

Then, for each compound synthesized in the above Examples, PI3Kγ inhibitory activity was measured.

(Method)

The PI3Kγ inhibitory activity of a compound was evaluated using PI3-kinase HTRF™ assay (Millipore) according to the following procedure.

To each well of a testing plate was added 5 μL of a compound solution containing 10% DMSO (200 μM as the concentration of the compound), 5 μL of 40 μM phosphatidylinositol (4,5)-bisphosphate/20mM $MgCl_2$/10mM DTT, and 5 μL of 80 μg/mL PI-3 kinase γ/10 mM $MgCl_2$/5mM DTT, and then stood for 10 minutes.

Then, 5 μL of 40 μM ATP/10 mM $MgCl_2$/5 mM DTT was added thereto. After reacting for 30 minutes at room temperature, 5μL of a solution containing EDTA and biotinylated phosphatidylinositol (3,4,5)-triphosphate was added to quench the reaction.

5 μL of a detection reagent containing a europium-labeled anti-GST antibody, the GST-tagged PH domain, and allophycocyanin-labeled streptavidin was added thereto. After 18 hours, HTRF (excitation wavelength: 330nm, measuring wavelengths: 620nm and 665nm) was measured.

Avalue of dividing an amount of fluorescence obtained at the measuring wavelength 665nmby an amount of fluorescence obtained at 620nm was defined as an HTRF ratio. The HTRF ratio in the absence of a compoundwas defined as 100% activity, and the HTRF ratio in the absence of PI-3 kinase γ was defined as 0% activity to calculate an inhibition ratio.

(Result)

Results are shown in the tables below.

[Table 2-1]

| Compound No. | Inhibition ratio % (50μM) | Compound No. | Inhibition ratio % (50μM) | Compound No. | Inhibition ratio % (50μM) |
|---|---|---|---|---|---|
| 1-1 | 72 | 1-4 | 57 | 1-5 | 60 |
| 1-6 | 67 | 1-7 | 77 | 1-9 | 62 |
| 1-12 | 78 | 1-13 | 67 | 1-15 | 74 |
| 1-17 | 76 | 1-18 | 61 | 1-19 | 63 |
| 1-20 | 62 | 1-22 | 56 | 1-27 | 68 |
| 1-28 | 69 | 1-29 | 64 | 1-32 | 70 |
| 1-33 | 57 | 1-35 | 77 | 1-36 | 60 |
| 1-37 | 66 | 1-38 | 91 | 1-39 | 68 |
| 1-41 | 63 | 1-43 | 79 | 1-44 | 57 |
| 1-45 | 74 | 1-46 | 76 | 1-48 | 63 |
| 1-49 | 64 | 1-50 | 69 | 1-51 | 60 |
| 1-53 | 74 | 1-54 | 65 | 1-55 | 65 |
| 1-56 | 77 | 1-57 | 64 | 1-58 | 70 |
| 1-59 | 52 | 1-60 | 64 | 1-63 | 81 |
| 1-65 | 79 | 1-66 | 77 | 1-67 | 72 |
| 1-68 | 60 | 1-69 | 59 | 1-70 | 78 |
| 1-71 | 69 | 1-72 | 66 | 1-73 | 70 |
| 1-74 | 59 | 1-75 | 64 | 1-76 | 72 |
| 1-77 | 64 | 1-79 | 81 | 1-80 | 57 |
| 1-81 | 63 | 1-82 | 78 | 1-83 | 111 |
| 1-88 | 56 | 1-98 | 72 | 1-101 | 58 |
| 1-103 | 60 | 1-104 | 55 | 1-105 | 73 |
| 1-107 | 74 | 1-108 | 69 | 1-109 | 69 |
| 1-110 | 81 | 1-111 | 55 | 1-112 | 64 |
| 1-113 | 66 | 1-114 | 62 | 1-115 | 78 |
| 1-116 | 76 | 1-117 | 65 | 1-118 | 64 |

[Table 2-2]

| Compound No. | Inhibition ratio % (50μM) | Compound No. | Inhibition ratio % (50μM) | Compound No. | Inhibition ratio % (50μM) |
|---|---|---|---|---|---|
| I-119 | 96 | I-120 | 85 | I-121 | 71 |
| I-122 | 61 | I-124 | 103 | I-127 | 79 |
| I-128 | 50 | I-131 | 70 | I-132 | 78 |
| I-133 | 65 | I-134 | 59 | I-135 | 80 |
| I-136 | 79 | I-137 | 81 | I-138 | 75 |
| I-139 | 79 | I-140 | 60 | I-141 | 87 |
| I-143 | 102 | I-144 | 83 | I-145 | 70 |
| I-146 | 64 | I-147 | 77 | I-148 | 83 |
| I-149 | 53 | I-150 | 71 | I-151 | 63 |
| I-153 | 80 | I-154 | 54 | I-155 | 57 |
| I-157 | 58 | I-159 | 88 | I-160 | 77 |
| I-161 | 102 | I-162 | 59 | I-163 | 58 |
| I-164 | 80 | I-169 | 64 | I-170 | 60 |
| I-171 | 71 | I-172 | 81 | I-174 | 78 |
| I-175 | 88 | I-177 | 85 | I-180 | 98 |
| I-182 | 98 | I-186 | 78 | I-188 | 63 |
| I-192 | 52 | I-194 | 84 | I-195 | 82 |
| I-198 | 57 | I-202 | 83 | I-203 | 68 |
| I-204 | 64 | I-207 | 64 | I-209 | 81 |
| I-210 | 85 | I-211 | 61 | I-212 | 60 |
| I-213 | 73 | I-215 | 70 | I-216 | 55 |
| I-217 | 62 | I-218 | 63 | I-220 | 59 |
| I-222 | 87 | I-223 | 86 | I-224 | 89 |
| I-232 | 51 | I-233 | 79 | I-234 | 71 |
| I-235 | 78 | I-236 | 67 | I-237 | 74 |
| I-239 | 74 | I-240 | 73 | I-241 | 67 |

[Table 2-3]

| Compound No. | Inhibition ratio % (50μM) | Compound No. | Inhibition ratio % (50μM) | Compound No. | Inhibition ratio % (50μM) |
|---|---|---|---|---|---|
| 1-243 | 78 | 1-244 | 87 | 1-245 | 76 |
| 1-246 | 75 | 1-247 | 64 | 1-248 | 71 |
| 1-249 | 89 | 1-253 | 80 | 1-254 | 77 |
| 1-255 | 75 | 1-257 | 83 | 1-258 | 76 |
| 1-259 | 68 | 1-260 | 73 | 1-261 | 89 |
| 1-263 | 71 | 1-264 | 67 | 1-267 | 81 |
| 1-268 | 82 | 1-269 | 84 | 1-270 | 82 |
| 1-271 | 83 | 1-272 | 81 | 1-273 | 88 |
| 1-274 | 89 | 1-275 | 75 | 1-276 | 87 |
| 1-277 | 72 | 1-278 | 81 | 1-279 | 83 |
| 1-280 | 92 | 1-281 | 78 | 1-282 | 78 |
| 1-283 | 86 | 1-284 | 111 | 1-285 | 96 |
| 1-286 | 87 | 1-287 | 101 | 1-288 | 107 |
| 1-289 | 127 | 1-290 | 92 | 1-291 | 74 |
| 1-292 | 74 | 1-293 | 72 | 1-294 | 88 |

(Example 22: Measurement of AKT phosphorylation inhibitory activity)

Then, cells are used to measure whether or not the inhibitory activity is exhibited.

(Method)

(1) The AKT phosphorylation inhibitory activity of a compound was evaluated according to the following procedure.
(2) Human monocyte-like cell line THP-1 was washed with RPMI-1640 media, incubated in the presence of 5% $CO_2$ at 37 degrees Celsius for 3 hours, washed with Hank's balanced salt solution (HBSS), adjusted to a cell concentration of 6.6 x $10^6$ /mL, and then used in an experiment.
(3) 30 μL of the cell suspension and 60 μL of each compound solution containing 0.2% DMSO/HBSS are mixed, and then preincubated at 37 degrees Celsius for 5 minutes. 30 μL of HBSS containing 4 μg/mL of MCP-1 was added thereto, and then incubated for 30 seconds at 37 degrees Celsius.
(4) 30 μL of 20 mM Tris-HCl (pH 7.5)/150 mM NaCl/1 mM $Na_2EDTA$/1 mM EGTA/1% Triton/2.5 mM sodium pyrophosphate/1 mM β-glycerophosphate/1 mM $Na_3VO_4$/1 μg/ml leupeptin/50 nM APMSF was added thereto to dissolve cells.
(5) The amount of AKT phosphorylation in a cell solution was measured by ELISA method.
(6) To a micro well plate to which anti-phospho-Akt (Ser473) antibody (clone 193H12, derived from a rabbit) was solid-phased was added 100 μL of a prepared cell solution, incubated for 2 hours at 37 degrees Celsius, and then washed four times with Phosphate Buffered Saline/0.05% Tween-20.
(7) An anti-AKT1 antibody (clone 2H10, derived from a mouse) was added thereto, incubated for 1 hour at 37 degrees Celsius, washed similarly, and then reacted with an HRP-labeled anti-mouse IgG antibody.
(8) After incubating at 37 degrees Celsius for 30 minutes and then washing similarly, 100 μL of TMB (3,3',5,5"-tetramethylbenzidine) was added thereto, followed by reacting at room temperature for 30 minutes.
(9) 100 μL of 1 mol/L sulfuric acid was added to quench the color reaction, and then the absorbance at 450 nm was

measured.

(10) A series of diluted cell solutions of a positive control (a sample in the absence of a compound) were used as a calibration curve, the amount of AKT phosphorylation in a sample in the absence of MCP-1 was defined as 0% activity to calculate an inhibition ratio.

(Result)

Compound No. I-36: >99.9%
Compound No. I-37: 86.7%
Compound No. I-291: >99.9%
Compound No. I-112: >99.9%
Compound No. I-119: 92.9%
Compound No. I-136: >99.9%
Compound No. I-144: >99.9%
Compound No. I-146: >99.9%
Compound No. I-148: >99.9%
Compound No. I-152: >99.9%
Compound No. I-223: >99.9%
Compound No. I-266: 90.1%
Compound No. I-274: 82.6%

(Example 23: Measurement of PI3Kγ inhibitory activity (Ki value))

The PI3Kγ inhibitory activity (Ki value) of a compound was evaluated according to the following procedure.

5 μL of a compound solution containing 10% DMSO and 200 μM of a compound was changed to 5 μL of a compound solution containing 10% DMSO and 200, 64, 20, 6.4, 2, 0.64, or 0.20 μM of the compound (optionally, this is diluted to a lower concentration). By a method similar to the method for measuring PI3Kγ inhibitory activity, inhibition ratios were measured in the presence of the compound of 50, 16, 5, 1.6, 0.5, 0.16, and 0.05 μM (optionally, a lower concentration), and then an $IC_{50}$ value was calculated by a logistic approximation method, or the linear regression method using two concentrations that across 50% inhibition. Separately, 5 μL of 40 μM ATP/10 mM $MgCl_2$/5 mM DTT at the time of the start of a reaction in the absence of a compound was changed to 5 μL of 80, 40, 20, 10, 5, 2.5, 1.25, or 0.625 μM ATP/10 mM $MgCl_2$/5 mM DTT, and then ratios of HTRF was measured by a similar method. The value of subtracting an HTRF ratio at each ATP concentration from an HTRF ratio in the absence of PI-3 kinase γ was defined as a value of multiplying reaction rate v by a constant to calculate the Michaelis-Menten constant Km by the Lineweaver-Burk plot method. A Ki value of a compound was calculated by the following formula.

$$Ki = IC_{50}\ \text{value}/(1 + 10\ \mu M\ (\text{test ATP concentration})/Km(\mu M))$$

[Numerical formula 1]

$$Ki = \frac{IC_{50}\ \text{value}}{1 + \dfrac{10\ \mu M\ (\text{Test ATP concentration})}{Km\ (\mu M)}}$$

(Result)

Compound No. I-120: 0.046 μM
Compound No. I-144: 0.052 μM
Compound No. I-224: 0.074 μM

(Example 24: Measurement of PI3Kα inhibitory activity)

The PI3Kα inhibitory activity of a compound was evaluated according to the following procedure.

According to Example 21 above, after 5 μL of 80 μg/mL PI-3 kinase γ/10 mM $MgCl_2$/5 mM DTT was changed to 5 μL of 0.8 μg/mL PI-3 kinase α/10 mM $MgCl_2$/5 mM DTT, an inhibition ratio was calculated by a method similar to a method for measuring PI3Kγ inhibitory activity, and then defined as the PI3Kα inhibitory activity at 50 μM.

(Example 25: Measurement of PI3Kα inhibitory activity (Ki value))

The α inhibitory activity (Ki value) of a compound was evaluated according to the following procedure.

According to Example 23 above, 5 μL of 80 μg/mL PI-3 kinase γ/10 mM $MgCl_2$/5 mM DTT was changed to 5 μL of 0.8 μg/mL PI-3 kinase α/10 mM $MgCl_2$/5 mM DTT, and a Km value measured with PI3Kα was used to calculate a Ki value for PI3Kα by a method similar to the PI3Kγ inhibitory activity (Ki value).

(Example 26: Measurement of PI3Kβ inhibitory activity)

The PI3Kβ inhibitory activity of a compound was evaluated according to the following procedure.

According to Example 21 above, after 5 μL of 80 μg/mL PI-3 kinase γ/10 mM $MgCl_2$/5 mM DTT was changed to 5 μL of 60 μg/mL PI-3 kinase β/10 mM $MgCl_2$/5 mM DTT, a method similar to the method for measuring PI3Kγ inhibitory activity was used to calculate an inhibition ratio, which was defined as the PI3Kβ inhibitory activity at 50 μM.

(Example 27: Measurement of PI3Kβ inhibitory activity (Ki value))

The β inhibitory activity (Ki value) of a compound was evaluated according to the following procedure.

According to Example 23 above, 5 μL of 80 μg/mL PI-3 kinase γ/10 mM $MgCl_2$/5 mM DTT was changed to 5 μL of 60 μg/mL PI-3 kinase β/10 mM $MgCl_2$/5 mM DTT, and a Km value measured with PI3Kβ was used to calculate a Ki value for PI3Kβ by a method similar to the PI3Kγ inhibitory activity (Ki value).

(Example 28: Method for calculating the selectivity of PI3Kγ and PI3Kα)

The PI3Kγ/α selectivity of a compound was expressed by a value of dividing a Ki value for PI3Kα by a Ki value for PI3Kγ.

(Example 29: Method for calculating the selectivity of PI3Kγ and PI3Kβ)

The PI3Ky/β selectivity of a compound was expressed by a value of dividing a Ki value for PI3Kβ by a Ki value for PI3Kγ.

According to Examples 30 to 35 shown below, a compound of the present invention was evaluated.

(Example 30: CYP3A4 fluorescence MBI test)

The CYP3A4 fluorescence MBI test is a test to examine the enhancement of CYP3A4 inhibition of a compound by a metabolic reaction. The test was performed using as an index a reaction in which CYP3A4 expressed in *E. coli* was used as an enzyme, and 7-benzyloxytrifluoromethylcoumarin (BFC) is debenzylated by CYP3A4 enzyme to produce a metabolite 7-hydroxytrifluoromethylcoumarin (HFC) which emits fluorescence.

The reaction condition is as follows: substrate, 5.6 μmol/L 7-BFC; pre-reaction time, 0 or 30 minutes; reaction time, 15 minutes; reaction temperature, 25 degrees Celsius (room temperature); content of CYP3A4 (an enzyme expressed in *E. coli*), 62.5 pmol/mL at the time of a pre-reaction, 6.25 pmol/mL (when diluted 10 times) at the time of a reaction; concentration of a test drug, 0.625, 1.25, 2.5, 5, 10, and 20 μmol/L (6 points).

To a 96-well plate was added an enzyme and a test drug solution in K-Pi buffer solution (pH 7.4) as a pre-reaction solution in the aforementioned constitution of the pre-reaction. Then, a part thereof was transferred to another 96-well plate so as to be diluted ten times with a substrate and a K-Pi buffer solution. A coenzyme NADPH was then added to start a reaction that is an index (without a pre-reaction). After reacting for a predetermined time, 4/1 of acetonitrile/0.5 mol/L Tris (trishydroxyaminomethane) was added to quench the reaction. To the remaining pre-reaction solution was also NADPH to start a pre-reaction (with a pre-reaction). After pre-reacting for a predetermined time, a part thereof was transferred to another plate so as to be diluted ten times with a substrate and K-Pi buffer solution, and thereby the reaction that is an index started. After reacting for a predetermined time, 4/1 of acetonitrile/0.5 mol/L Tris (trishydroxyaminomethane) was added to quench the reaction. For each plate in which the index reaction was performed, the fluorescence

value of a metabolite 7-HFC was measured with a fluorescent plate reader (Ex = 420nm, Em = 535nm).

The case that only DMSO, a solvent which dissolved a drug, was added to a reaction system was defined as a control (100%). The remaining activity (%) was calculated at each concentration after a test drug solution was added, and then $IC_{50}$ was calculated with a concentration and an inhibition ratio by an inverse estimation using a logistic model. The case that the difference of $IC_{50}$ values was 5 μM or higher was determined as (+). The case that it was 3 μM or lower was determined as (-).

(Result)

Compound No. I-292: (-)
Compound No. I-224: (-)
Compound No. I-293: (-)
Compound No. I-260: (-)
Compound No. I-12: (-)
Compound No. I-239: (-)
Compound No. I-267: (-)

(Example 31: CYP inhibition test)

Using a pooled human liver microsome commercially available, and selecting as indexes O-de-ethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), which are typical substrate metabolic reactions of human main CYP5 molecular species (CYP1A2, 2C9, 2C19, 2D6, and 3A4), it was evaluated in what degree the amount of each metabolite produced was inhibited by a test compound.

The reaction condition is as follows: substrate, 0.5 μmol/L of ethoxyresorufin (CYP1A2), 100 μmol/L of tolbutamide (CYP2C9), 50 μmol/L of S-mephenytoin (CYP2C19), 5 μmol/L of dextromethorphan (CYP2D6), and 1 μmol/L of terfenadine (CYP3A4); reaction time, 15 minutes; reaction temperature, 37 degrees Celsius; enzyme, pooled human liver microsome 0.2 mg protein/mL; test drug concentration, 1, 5, 10, and 20 μmol/L (4 points).

To a 96-well plate was added five kinds of substrates, a human liver microsome, and a test drug in 50 mM Hepes buffer solution as a reaction solution in the aforementioned constitution. A coenzyme NADPH was added to start a metabolic reaction, which is an index. After reacting at 37 degrees Celsius for 15 minutes, a solution of methanol/acetonitrile (1/1 (v/v)) was added to quench the reaction. After centrifugation at 3000 rpm for 15 minutes, resorufin (CYP1A2 metabolite) in the supernatant was quantitated with a fluorescence multilabel counter, and hydroxylated tolbutamide (CYP2C9 metabolite), 4'-hydroxylated mephenytoin (CYP2C19 metabolite), dextromethorphan (CYP2D6 metabolite), terfenadine in alcohol form (CYP3A4 metabolite) were quantitated with LC/MS/MS.

The case that only DMSO, a solvent which dissolved a drug, was added to a reaction system was defined as a control (100%). The remaining activity (%) at each concentration in cases that a test drug solution was added was calculated, and then $IC_{50}$ was calculated with a concentration and an inhibition ratio by an inverse estimation using a logistic model.

(Result)

Compound No. I-292: 5 kinds >20 μM
Compound No. I-224: 5 kinds >20 μM
Compound No. I-293: 5 kinds >20 μM
Compound No. I-260: 5 kinds >20 μM
Compound No. I-267: 5 kinds >20 μM

(Example 32: FAT test)

20 μL of *Salmonella enterica* subsp. *typhimurium* (*Salmonella typhimurium* TA98 line, TA100 line) cryopreserved was inoculated to 10 mL of liquid nutrient medium (2.5% Oxoid nutrient broth No. 2), and then precultured at 37 degrees Celsius for 10 hours with shaking. Regarding TA98 line, after 9mL of a bacterial suspension was centrifuged (2000 x g, 10 minutes) to remove the culture solution, the bacteria was suspended in 9 mL of Micro F buffer solution

($K_2HPO_4$: 3.5 g/L, $KH_2PO_4$: 1 g/L, $(NH_4)_2SO_4$: 1g/L, tri-sodium citric acid dihydrate : 0.25 g/L, $MgSO_4 \cdot 7H_20$ : 0.1g/L), and then added to 110 mL of Exposure media (Micro Fbuffer solution containing biotin: 8 μg/mL, histidine: 0.2 μg/mL, glucose: 8 mg/mL). Regarding TA 100 line, to 3.16 mL of the bacterial suspension was added 120 mL of Exposure media to prepare a test bacterial suspension. 12 μL of each of a solution of a test substance in DMSO (diluted eight times in a common ratio of 2 from the maximum dose of 50mg/mL); DMSO as a negative control; as a positive control, in the case of a non-metabolism-activation condition, 50 μg/mL 4-nitroquinoline-1-oxide solution in DMSO for TA98 line, 0.25 μg/mL 2-(2-furyl)-3-(5-nitro-2-furyl)acrylamide solution in DMSO for TA 100 line; and in the case of a metabolism-activation condition, 40 μg/mL 2-aminoanthracene solution in DMSO for TA98 line, 20 μg/mL of 2-aminoanthracene solution in DMSO for TA100 line, and 588 μL of the test bacterial suspension (in the case of a metabolism activation condition, a mixed solution of 498 μL of the test bacterial suspension and 90 μL S9 mix) were mixed, and then cultured at 37 degrees Celsius at 90 minutes with shaking. 460 μL of the bacterial suspension to which a test substance was exposed was mixed with 2300 μL of Indicator media (a Micro F buffer solution containing biotin: 8 μg/mL, histidine: 0.2 μg/mL, glucose: 8 mg/mL, bromocresol purple: 37.5 μg/mL). 50 μL thereof was dispensed to microplate 48 wells/dose, and then statically cultured at 37 degrees Celsius for 3 days. In a well containing bacteria that obtained proliferation potency by the mutation of amino acid (histidine) synthetase gene, the change of pH caused the color change from purple to yellow. Thus, in 48 wells per dose, the number of the bacteria-proliferation well in which the color changed to yellow was counted, compared with a group of negative controls, and then evaluated.

(Result)

Compound No. I-28: (-)
Compound No. I-29: (-)
Compound No. I-120: (-)

(Example 33: Solubility test)

The solubility of a compound was determined under a condition in which 1% DMSO was added. 10 mM compound solution was prepared using DMSO, and then 6 μL of the compound solution was added to 594 μL of artificial intestinal juice in pH 6.8 (to 250 mL of 0.2 mol/L potassium dihydrogen phosphate reagent solution was added 118 mL of 0.2 mol/L NaOH reagent solution and water to provide a final volume of 1000 mL). After standing at 25 degrees Celsius for 16 hours, the mixed solution was filtrated with suction. The filtrate was diluted twice with methanol/water (1/1), and then a concentration in the filtration was measured with HPLC or LC/MS/MS by the absolute calibration method.

(Result)

Compound No. I-28: >50 μM
Compound No. I-37: >50 μM
Compound No. I-75: >50 μM
Compound No. I-81: >50 μM
Compound No. I-120: >50 μM
Compound No. I-136: >50 μM
Compound No. I-144: >50 μM
Compound No. I-292: >50 μM
Compound No. I-224: >50 μM
Compound No. I-270: >50 μM

(Example 34: Metabolic stability test)

After a subject compound was reacted for a certain time using a pooled human liver microsome commercially available, a reacted sample and an unreacted sample are compared to calculate a survival ratio, and then the degree of metabolism in the liver was evaluated.

0.2 mL of a buffer solution (50 mmol/L of Tris-HCl in pH 7.4, 150 mmol/L of potassium chloride, and 10 mmol/L of magnesium chloride) containing a human liver microsome of 0.5mg protein/mL was reacted in the presence of 1 mmol/L NADPH at 37 degrees Celsius for 0 or 30 minutes (oxidative reaction). After reacting, 50 μL of the reaction

solution was added to 100 μL of a solution of methanol/acetonitrile (1/1 (v/v)), mixed, and then centrifuged at 3000 rpm for 15 minutes. A test compound in the supernatant was quantitated with LC/MS/MS. An amount of the compound remained at a reaction time of 0 minute was defined as 100%, and, based on that, an amount of the test compound remained after the reaction was calculated.

(Result)

Compound No. I-28: 98.6%
Compound No. I-29: 98.8%
Compound No. I-37: 96.8%
Compound No. I-118: 95.9%
Compound No. I-144: >99.9%
Compound No. I-152: >99.9%
Compound No. I-163: 98.1%
Compound No. I-210: 97%
Compound No. I-221: >99.9%

(Example 35: hERG test)

For the purpose of a risk evaluation of QT interval extension of electrocardiogram, using the HEK293 cell that was made express human ether-a-go-go related gene (hERG) channel, the activity on delayed rectification $K^+$ current ($I_{Kr}$) playing an important role in a cardiac ventricle repolarization process was examined.
Using an automatic patch-clamp system (PatchXpress 7000A, Axon Instruments Inc.), by a whole-cell patch-clamp method, after the cell was maintained at a membrane potential of -80 mV, $I_{Kr}$ induced on giving depolarizing stimulation of +50 mV for 2 seconds and further repolarizing stimulation of -50 mV for 2 seconds was recorded. After generated current became stable, extracellular fluid (NaCl: 137 mmol/L, KCl: 4 mmol/L, $CaCl_2 \cdot 2H_2O$: 1.8 mmol/L, $MgCl_2 \cdot 6H_2O$: 1 mmol/L, glucose: 10 mmol/L, HEPES (4-(2-hydroxyethyl)-1-piperazineethanesulfonic acid): 10 mmol/L, pH = 7.4) dissolving a test substance at an intended concentration was applied to the cell for 10 minutes under room temperature condition. From the $I_{Kr}$ obtained, using an analysis soft (DataXpress ver. 1, Molecular Devices Corporation), a current value in a membrane potential maintained was used as a standard, and the absolute value of the maximum tail current was measured. Moreover, an inhibition ratio for the maximum tail current prior to the application of the test substance was calculated, and then compared with a group of media-application (0.1 % dimethylsulfoxide solution) to evaluate influence of the test substance on $I_{Kr}$.

(Result)

Compound No. I-81: 6%
Compound No. I-294: 6.4%
Compound No. I-137: 8.4%
Compound No. I-143: 7.8%
Compound No. I-182: 9.3%
Compound No. I-208: 0.5%
Compound No. I-248: 6.7%
Compound No. I-280: 5.8%

(Discussion)

As described above, the compound of the present invention exhibited excellent PI3-kinase γ inhibitory activity *in vitro* and *in vivo.* Accordingly, the pharmaceutical composition of the present invention may be used for the prevention and/or as a therapeutic agent for diseases such as encephalitis, myelitis and encephalomyelitis, meningitis, inflammatorypolyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, andthelike),pleurisy,pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, sarcoidosis, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

(Example 36: Formulation example 1 Tablet)

A tablet consisting of the following constitution is produced by a conventional method.

| | |
|---|---|
| The compound of the present invention | 100mg |
| Lactose | 60mg |
| Potato starch | 30mg |
| Polyvinyl alcohol | 2mg |
| Magnesium stearate | 1mg |
| Tar dye | minute amount |

(Example 37: Formulation example 2 Powder)

A powder consisting of the following constitution is produced by a conventional method.

| | |
|---|---|
| The compound of the present invention | 150mg |
| Lactose | 280mg |

(Example 38: Formulation example 3 Syrup)

Syrup consisting of the following constitution is produced by a conventional method.

| | |
|---|---|
| The compound of the present invention | 100mg |
| Refined white sugar | 40 g |
| Ethyl *p*-hydroxybenzoate | 40mg |
| Propyl *p*-hydroxybenzoate | 10mg |
| Chocolate flavor | 0.1 cc |

Water was added to this to provide a total amount of 100 cc.

The present invention has been exemplified so far by reference to preferable embodiments of the present invention, but it should not be construed that the present invention is restricted by the embodiments of the present invention. It should be understood that the scope of the present invention should be construed only by the claims. It would be understood that those skilled in the art can perform an invention practically equivalent to the present invention,

based on the description of the present invention and technical common sense from the specific description of preferable embodiments of the present invention. It would be understood that the patents, patent applications and literature cited herein should be incorporated herein by reference to the present specification in their entire contents, similarly to the case where the description is described specifically herein.

INDUSTRIAL APPLICABILITY

The present invention provides medicaments for the treatment of phosphatidylinositol-3-kinase dependent diseases, a compound used therefor, a pharmaceutically acceptable salt thereof, or a prodrug thereof including a solvate thereof and the like. The compound of present invention exhibits excellent inhibitory activity on PI3-kinase γ as described in the above Examples.

## Claims

**1.** A pharmaceutical composition comprising a compound represented by the formula (I):

[Chem. 50]

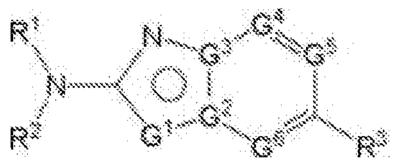

(I)

wherein $R^1$ is a hydrogen atom or alkyl;
$R^2$ is substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, or substituted or unsubstituted aminocarbonylamino;
$R^3$ is a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^8$ (wherein $R^8$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl); or a group represented by the formula: $-S(O)_mR^9$ (wherein $R^9$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted acyl; and m is 0, 1, or 2);
a cyclic group represented by the formula:

[Chem. 51]

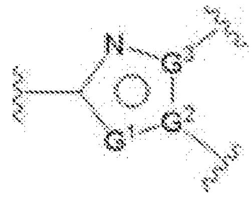

is a cyclic group represented by any of the following formulas (A) - (D) :

[Chem. 52]

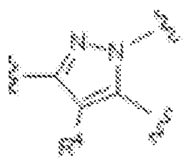 , 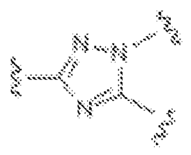 , 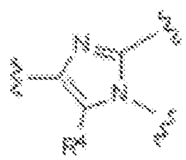 , and 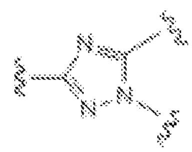

(A) (B) (C) (D) ;

$G^4$ is $C(R^5)$ or a nitrogen atom;
$G^5$ is $C(R^6)$ or a nitrogen atom;
$G^6$ is $C(R^7)$ or a nitrogen atom; and
$R^4$, $R^5$, $R^6$, and $R^7$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{10}$ (wherein $R^{10}$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{11}$ (wherein $R^{11}$ is a hydrogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, substituted or unsubstituted amino, or substituted or unsubstituted acyl; and n is 0, 1, or 2);
with the proviso that: when the cyclic group is a cyclic group represented by (C) or (D) and $R^2$ is substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl, $R^3$ is not 6-membered ring heteroaryl substituted with substituted or unsubstituted amino;
when the cyclic group is a cyclic group represented by (C) or (D), $G^1$ is CH, $G^4$ is CH, and $G^5$ is CH, $R^2$ is not trifluoromethylcarbonyl; and

when the cyclic group is a cyclic group represented by (C), $G^4$ is CH, $G^5$ is CH, $G^6$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the following formula:

[Chem. 53]

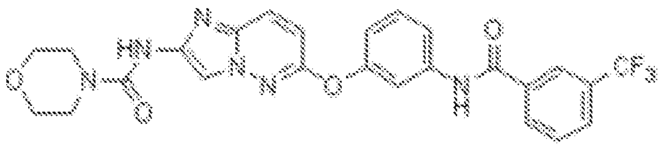

or

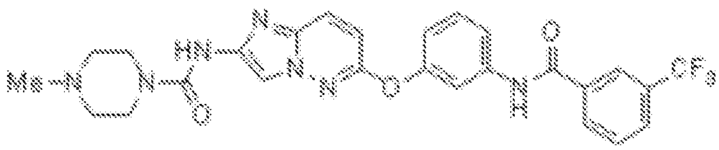

,

a pharmaceutically acceptable salt thereof, or a solvate thereof.

**2.** The pharmaceutical composition according to claim 1, comprising a compound represented by the formula (II):

[Chem. 54]

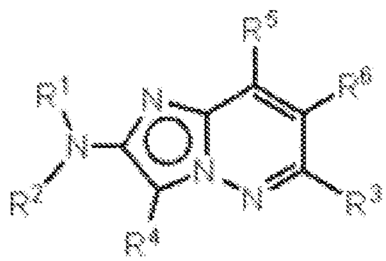

(II)

wherein the definitions of $R^1$, $R^2$, $R^3$, $R^4$, $R^5$, and $R^6$ are the same as claim 1,
a pharmaceutically acceptable salt thereof, or a solvate thereof.

**3.** A compound represented by the formula (IV):

[Chem. 55]

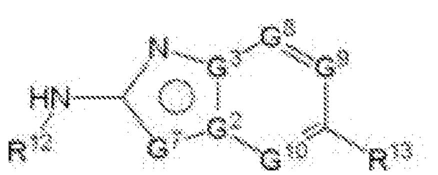

(IV)

wherein $R^{12}$ is substituted or unsubstituted aryl, substituted or unsubstituted heteroaryl, a substituted or unsubstituted non-aromatic heterocyclic group, substituted or unsubstituted alkylcarbonyl, substituted or unsubstituted alkyloxycarbonyl, or substituted or unsubstituted aminocarbonyl;
$R^{13}$ is a halogen atom, substituted or unsubstituted alkyl, substituted or unsubstituted amino, substituted or unsubstituted alkenyl, substituted or unsubstituted aryl, substituted or unsubstituted heteroaryl, a substituted or unsubstituted non-aromatic heterocyclic group, substituted or unsubstituted arylamino, substituted or unsubstituted heteroarylamino, substituted or unsubstituted alkyloxycarbonyl, substituted or unsubstituted aminocarbonyl, substituted or unsubstituted aminocarbonylamino, substituted or unsubstituted alkylamino, substituted or unsubstituted acyl, substituted or unsubstituted acylamino, a group represented by the formula: $-OR^{14}$ (where-

in $R^{14}$ is substituted or unsubstituted aryl, substituted or unsubstituted heteroaryl, or a substituted or unsubstituted non-aromatic heterocyclic group); or a group represented by the formula: $-S(O)_mR^{15}$ (wherein $R^{15}$ is substituted or unsubstituted aryl or substituted or unsubstituted heteroaryl; and m is 0, 1, or 2);
a cyclic group represented by the formula:

[Chem. 56]

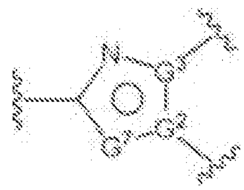

is a cyclic group represented by any of the following formulas (E)-(H) :

[Chem. 57]

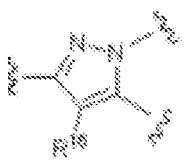 , 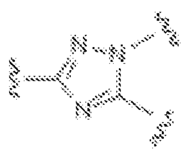 , 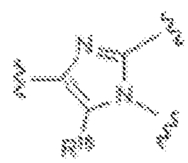 , and 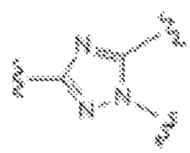

(E) (F) (G) (H) ;

$G^8$ is C ($R^{17}$) or a nitrogen atom;
$G^9$ is C($R^{18}$) or a nitrogen atom;
$G^{10}$ is C($R^{19}$) or a nitrogen atom; and
$R^{16}$, $R^{17}$, $R^{18}$, and $R^{19}$ are each independently a hydrogen atom, a halogen atom, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl, a substituted or unsubstituted non-aromatic heterocyclic group, nitro, substituted or unsubstituted amino, cyano, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, substituted or unsubstituted aminocarbonyl, substituted or unsubstituted aminocarbonylamino, a group represented by the formula: $-OR^{20}$ (wherein $R^{20}$ is substituted or unsubstituted alkyl, substituted or unsubstituted alkenyl, substituted or unsubstituted alkynyl, substituted or unsubstituted aryl, substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl), or a group represented by the formula: $-S(O)_nR^{21}$ (wherein $R^{21}$ is substituted or unsubstituted alkyl or substituted or unsubstituted aryl; and n is 0, 1, or 2);
with the proviso that: when the cyclic group is a cyclic group represented by (G) or (H) and $R^{12}$ is substituted or unsubstituted acyl, substituted or unsubstituted alkyloxycarbonyl, substituted or unsubstituted alkenyloxycarbonyl, substituted or unsubstituted alkynyloxycarbonyl, substituted or unsubstituted cycloalkyloxycarbonyl, substituted or unsubstituted cycloalkenyloxycarbonyl, substituted or unsubstituted aryloxycarbonyl, substituted or unsubstituted heteroaryloxycarbonyl, substituted or unsubstituted non-aromatic heterocyclic group-oxycarbonyl, or substituted or unsubstituted aminocarbonyl, $R^{13}$ is not 6-membered ring heteroaryl substituted with substituted or unsubstituted amino;
when $R^{12}$ is substituted or unsubstituted phenyl, $G^2$ is a carbon atom, $G^3$ is a nitrogen atom, and $G^{10}$ is a nitrogen atom, $R^{16}$ is not cyano or carbamoyl;
when the cyclic group is a cyclic group represented by (G) or (H), $R^{12}$ is substituted or unsubstituted phenyl, $G^7$ is a nitrogen atom, and $G^8$ is a nitrogen atom, $R^{13}$ is not substituted or unsubstituted phenyl;

when the cyclic group is a cyclic group represented by (G) or (H) , $G^7$ is CH, $G^8$ is CH, and $G^9$ is CH, $R^{12}$ is notmethoxycarbonyl, methylcarbonyl, or trifluoromethylcarbonyl;
when the cyclic group is a cyclic group represented by (E) or (F), $R^{12}$ is methyloxycarbonyl, $G^7$ is CH, $G^8$ is CH, and $G^9$ is CH, $R^{13}$ is not phenylthio or phenylsulfinyl; and
when the cyclic group is a cyclic group represented by (G), $G^8$ is CH, $G^9$ is CH, $G^{10}$ is a nitrogen atom, $R^1$ is a hydrogen atom, and $R^3$ is substituted or unsubstituted 3-pyridyl, $R^2$ is not aminocarbonyl substituted with tetrazolylalkyl, and the compound is not a compound represented by the following formula:

[Chem. 58]

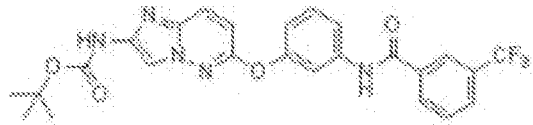
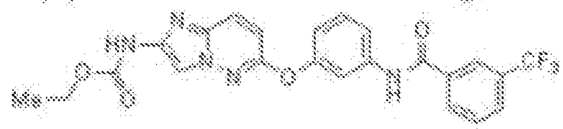
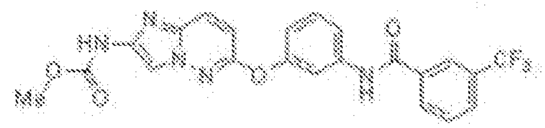
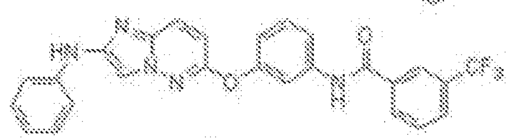
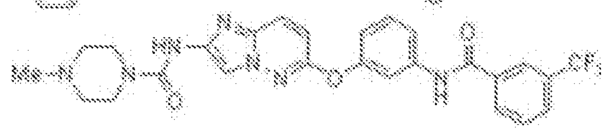
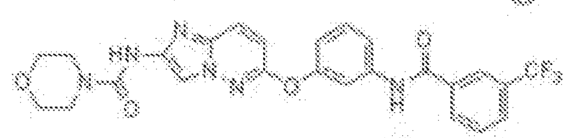
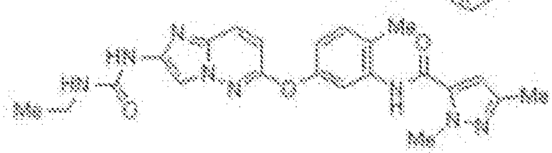
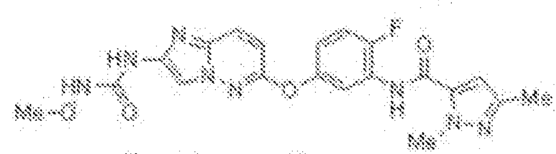
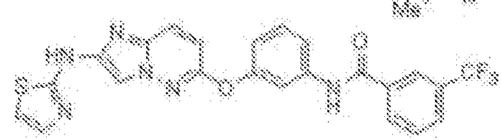
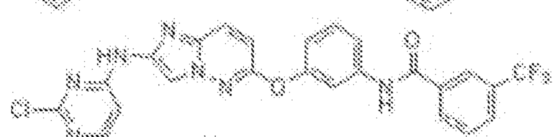
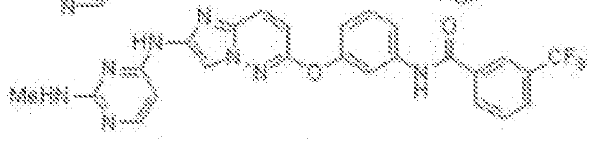

or

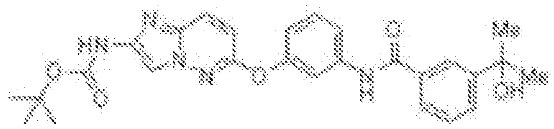,

a pharmaceutically acceptable salt thereof, or a solvate thereof.

**4.** The compound according to claim 3 represented by the formula (V):

[Chem. 59]

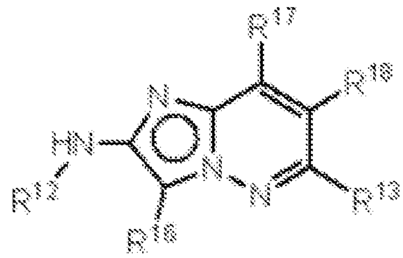

(V)

wherein the definitions of $R^{12}$, $R^{13}$, $R^{16}$, $R^{17}$, and $R^{18}$ are the same as claim 3,

a pharmaceutically acceptable salt thereof, or a solvate thereof.

**5.** The compound according to claim 4 wherein $R^{16}$, $R^{17}$, and $R^{18}$ are hydrogen atoms, a pharmaceutically acceptable salt thereof, or a solvate thereof.

**6.** The compound according to any of claims 3-5 wherein $R^{12}$ is substituted or unsubstituted aryl or substituted or unsubstituted heteroaryl, a pharmaceutically acceptable salt thereof, or a solvate thereof.

**7.** The compound according to any of claims 3-5 wherein $R^{12}$ is substituted or unsubstituted aminocarbonyl, a pharmaceutically acceptable salt thereof, or a solvate thereof.

**8.** The compound according to any of claims 3-5 wherein $R^{13}$ is substituted or unsubstituted aryl, or substituted or unsubstituted heteroaryl, a pharmaceutically acceptable salt thereof, or a solvate thereof.

**9.** The compound according to any of claims 3-5 wherein $R^{13}$ is substituted or unsubstituted aryloxy, substituted or unsubstituted arylthio, or substituted or unsubstituted arylamino, a pharmaceutically acceptable salt thereof, or a solvate thereof.

**10.** The compound according to any of claims 3-5 wherein $R^{13}$ is substituted or unsubstituted alkyloxycarbonyl or substituted or unsubstituted aminocarbonyl, a pharmaceutically acceptable salt thereof, or a solvate thereof.

**11.** The compound according to any of claims 3-5 wherein $R^{12}$ is substituted or unsubstituted aryl or substituted or unsubstituted heteroaryl, and $R^{13}$ is substituted or unsubstituted aryl or substituted or unsubstituted heteroaryl, a pharmaceutically acceptable salt thereof, or a solvate thereof.

**12.** The compound according to any of claims 3-5 wherein $R^{12}$ is substituted or unsubstituted aminocarbonyl, and $R^{13}$ is substituted or unsubstituted acylamino, substituted or unsubstituted arylamino, substituted or unsubstituted heteroarylamino, substituted or unsubstituted aryl, substituted or unsubstituted heteroaryl, substituted or unsubstituted acyl, the formula: $-OR^{14}$ (wherein the definition of $R^{14}$ is the same as claim 3), or the formula: $-S(O)_mR^{15}$ (wherein the definitions of $R^{15}$ and m are the same as claim 3), a pharmaceutically acceptable salt thereof, or a solvate thereof.

**13.** A pharmaceutical composition containing the compound, a pharmaceutically acceptable salt thereof, or a solvate thereof according to any of claims 3-12as an active ingredient according to any of claims 3-12.

**14.** A pharmaceutical composition for treating phosphatidylinositol-3-kinase dependent diseases containing the compound, a pharmaceutically acceptable salt thereof, or a solvate thereof according to any of claims 3-12as an active ingredient according to any of claims 3-12.

**15.** The pharmaceutical composition according to any of claims 1, 2, 13, and 14, wherein the pharmaceutical composition is for treating and/or preventing inflammation.

**16.** A method for preventing or treating inflammation, wherein the method is **characterized by** administering the compound, a pharmaceutically acceptable salt thereof, or a solvate thereof according to any of claims 3-12.

**17.** Use of the compound, a pharmaceutically acceptable salt thereof, or a solvate thereof according to any of claims 3-12 for producing a therapeutic agent and/or a prophylactic agent for inflammation.

**18.** The compound according to any of claims 3-12, a pharmaceutically acceptable salt thereof, or a solvate thereof, wherein the compound is for treating and/or preventing inflammation.

## INTERNATIONAL SEARCH REPORT

International application No.
PCT/JP2009/057706

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D487/04, A61K31/5025, A61K31/506, A61K31/5377, A61K31/55, A61P9/00, A61P9/10, A61P11/02, A61P17/04, A61P19/02, A61P29/00, A61P31/00, A61P35/00, A61P37/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | US 4237300 A (MERCK & CO., INC.), 02 December, 1980 (02.12.80), Examples 6, 25, 26, 34 (Family: none) | 1 |
| X | JP 2005-154335 A (Nippon Kayaku Co., Ltd.), 16 June, 2005 (16.06.05), Claims; table 1 (Family: none) | 1 |
| X | WO 2006/038116 A2 (WARNER-LAMBERT CO., L.L.C.), 13 April, 2006 (13.04.06), Example 6 & EP 1799680 A2 & US 2008/0021059 A1 & JP 2008-515874 A | 1 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 May, 2009 (18.05.09) | 26 May, 2009 (26.05.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/057706

C (Continuation). DOCUMENTS CONSIDERED TO BE RELEVANT

| Category* | Citation of document, with indication, where appropriate, of the relevant passages | Relevant to claim No. |
|---|---|---|
| X | WO 2005/089763 A1 (WARNER-LAMBERT CO., L.L.C.), 29 September, 2005 (29.09.05), Scheme 3 & EP 1737459 A1 & US 2007/0191394 A1 & JP 2007-529496 A | 1 |
| X | JP 52-073896 A (MARCK & CO., INC.), 21 June, 1977 (21.06.77), Claims & DE 2655681 A1 & GB 1524589 A & FR 2334357 A1 | 1 |
| X | WO 2007/046548 A1 (TANABE SEIYAKU CO., LTD.), 26 April, 2007 (26.04.07), Table 9 & EP 1945220 A1 & JP 2008-007493 A | 1 |
| X | WO 2002/044156 A2 (GLAXO GROUP LTD.), 06 June, 2002 (06.06.02), Example 68 & EP 1341771 A2 & US 2004/0082583 A1 & JP 2004-517080 A | 1,2 |
| X | WO 2008/016131 A1 (Takeda Chemical Industries, Ltd.), 07 February, 2008 (07.02.08), Claims; examples (Family: none) | 1,2 |
| X | D. R. NEWELL et al., Evaluation of rodent-only toxicology for early clinical trials with novel cancer therapeutics, British Journal of Cancer, 1999, Vol.81, No.5, p.760-768 | 1,2 |
| X | US 3701780 A (MERCK & CO., INC.), 31 October, 1972 (31.10.72), Example 23; column 9, line 1 to column 10, line 29 & GB 1353370 A | 3,18 |
| X | WO 2008/030579 A2 (BIOGEN IDEC MA INC.), 13 March, 2008 (13.03.08), Page 38 (Family: none) | 3,4,18 |
| X | JP 9-176165 A (Nihon Nohyaku Co., Ltd.), 08 July, 1997 (08.07.97), Table 1; examples (Family: none) | 1,3,7,13,14, 18 |
| X | JP 64-068375 A (The Wellcome Foundation Ltd.), 14 March, 1989 (14.03.89), Claims; examples & EP 0305093 A1 & US 5091531 A | 1-5,13,18 |

Form PCT/ISA/210 (continuation of second sheet) (April 2007)

**INTERNATIONAL SEARCH REPORT**

International application No.
PCT/JP2009/057706

C (Continuation). DOCUMENTS CONSIDERED TO BE RELEVANT

| Category* | Citation of document, with indication, where appropriate, of the relevant passages | Relevant to claim No. |
|---|---|---|
| X | WO 2008/016192 A2 (TAKEDA PHARMACEUTICAL CO., LTD.), 07 February, 2008 (07.02.08), Claims; examples (Family: none) | 1-7,9,12,13, 18 |
| X | WO 2008/025821 A1 (CELLZOME(UK) LTD.), 26 March, 2008 (26.03.08), Claims; examples (Family: none) | 1,3,6,8, 10-15,17,18 |
| X | WO 2005/037837 A1 (Nihon Nohyaku Co., Ltd.), 28 April, 2005 (28.04.05), Claims; examples & EP 1674454 A1 & US 2007/0129383 A1 & JP 2005-514793 A | 1,3,6-15,17, 18 |
| X | WO 2004/108729 A1 (Nihon Nohyaku Co., Ltd.), 16 December, 2004 (16.12.04), Claims; examples & EP 1630165 A1 & US 2007/0010515 A1 & JP 2005-506762 A | 1,3,6-15,17, 18 |
| A | WO 2005/113556 A1 (ICOS CORP.), 01 December, 2005 (01.12.05), Full text & EP 1761540 A1 & US 2008/0275067 A1 & JP 2007-537291 A | 1-15,17,18 |
| A | WO 2004/029055 A1 (BAYER HEALTHCARE AG.), 08 April, 2004 (08.04.04), Full text & EP 1549652 A1 & US 2006/0128732 A1 & JP 2006-508063 A | 1-15,17,18 |
| P,X | WO 2008/150015 A1 (TAKEDA PHARMACEUTICAL CO., LTD.), 11 December, 2008 (11.12.08), Claims; examples (Family: none) | 1,3,9,13,14, 18 |

Form PCT/ISA/210 (continuation of second sheet) (April 2007)

**INTERNATIONAL SEARCH REPORT**

International application No.
PCT/JP2009/057706

Continuation of A. CLASSIFICATION OF SUBJECT MATTER
(International Patent Classification (IPC))

*C07D487/04*(2006.01)i, *A61K31/5025*(2006.01)i, *A61K31/506*(2006.01)i, *A61K31/5377*(2006.01)i, *A61K31/55*(2006.01)i, *A61P9/00*(2006.01)i, *A61P9/10*(2006.01)i, *A61P11/02*(2006.01)i, *A61P17/04*(2006.01)i, *A61P19/02*(2006.01)i, *A61P29/00*(2006.01)i, *A61P31/00*(2006.01)i, *A61P35/00*(2006.01)i, *A61P37/00*(2006.01)i, *A61P43/00*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/057706

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 16
because they relate to subject matter not required to be searched by this Authority, namely:
Claim 16 involves methods for treatment of the human body or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7145051 A
- WO 9529673 A
- WO 2007095588 A
- JP 2005008581 A
- WO 2005054246 A
- JP 4092321 B
- JP 4096264 B
- JP 4105767 B
- EP 0018837 A
- JP 52073896 A
- WO 2002044156 A
- WO 2008016131 A
- WO 2008016192 A
- WO 2009010530 A


### Non-patent literature cited in the description


- **M.P. Wymann et al.** *Biochemical Society Transactions,* 2003, vol. 31, 275-280
- **Rueckle T. et al.** *NATURE REVIEWS DRUG DISCOVERY,* 2006, vol. 5, 903-918
- **Laffargue M. et al.** *Immunity,* 2002, vol. 16, 441-451
- **Hirsch E. et al.** *Science,* 2000, vol. 287, 1049-1053
- **Li Z. et al.** *Science,* 2000, vol. 287, 982-983
- **Sasaki T. et al.** *Science,* 2000, vol. 287, 1040-1046
- **Lupia E. et al.** *Am J Pathol.,* 2004, vol. 165, 2003-2011
- **Barber D F et al.** *Nat Med,* 2005, vol. 11, 933-935
- **Camps.** *Nat Med,* 2005, vol. 11, 936-943
- **Campbell et al.** *Circ Res.,* 2005, vol. 96, 197-206
- **Yadav M. et al.** *J Immunol.,* 2006, vol. 176, 5494-503
- *Japanese Journal of Clinical Immunology,* 2007, vol. 30 (5), 369-374
- **Ui M T et al.** *Trends Biochem. Sci.,* 1995, vol. 20, 303-307
- **Bochis R J et al.** *J. Med. Chem.,* 1981, vol. 24, 1518-1521
- **Peterson L H et al.** *J. Heterocyclic Chem.,* 1981, vol. 18, 659-662
- **Bochis R J et al.** *J. Med. Chem.,* 1978, vol. 21 (2), 235-236
- **Hasegawa M. et al.** *J. Med. Chem.,* 2007, vol. 50, 4453-4470
- **Jaramillo C. et al.** *Tetrahedron Letters,* 2002, vol. 43, 9051-9054
- **Mourad A E et al.** *J. Heterocyclic Chem.,* 1993, vol. 30, 1365-1372
- *Synthesis,* 1998, 867
- **T. W. Green.** Protective Groups in Organic Synthesis. John Wiley & Sons Inc, 1981
- **T. W. Greene.** Protective Groups in Organic Synthesis. John Wiley & Sons Inc, 1981
- **R. C. Larock.** *Comprehensive Organic Transformations,* 1989